# EUROPEAN PATENT APPLICATION

(11) **EP 4 480 964 A1**
(43) Date of publication of application: **25.12.2024**
(21) Application number: 23306000.3
(22) Date of filing: 22.06.2023
(51) Int. Cl.: C07K 16/18, C12N 15/11, A61K 39/395, A61P 21/00

(54) **TRANSFERRIN RECEPTOR-BINDING MOLECULES, CONJUGATES THEREOF AND THEIR USES TO PREVENT OR TREAT MUSCULAR DISEASES**

(71) Applicant: Vect-Horus, 13005 Marseille (FR); Centre National de la Recherche Scientifique, 75016 Paris (FR); Université d'Aix Marseille, 13007 Marseille (FR)
(72) Inventor: JACQUOT, Guillaume, 13100 AIX EN PROVENCE (FR); DAVID, Marion, 13010 MARSEILLE (FR); COHEN, Romy, 13010 MARSEILLE (FR); TEMSAMANI, Jamal, 30900 NIMES (FR); LECORCHE, Pascaline, 13004 MARSEILLE (FR); KHRESTCHATISKY, Michel, 13007 MARSEILLE (FR)
(74) Representative: Cabinet Becker et Associés

(57) **Abstract**

The invention relates to Variable Domain of Camelid Heavy Chain-only (VHH) molecules which bind TfR and the uses thereof e.g., to transport molecules of therapeutic interest into muscle cells, typically in pathological conditions.

## Description

### FIELD OF INVENTION

The invention relates to a Transferrin receptor (TfR)-binding molecule, conjugate compounds involving such a TfR-binding molecule, and uses thereof. The invention more particularly relates to Variable Domain of Camelid Heavy Chain-only (VHH) molecules binding TfR at the surface of muscle cells as well as their uses in a prophylactic or therapeutic context, e.g., to transport nucleic acid molecules, in particular oligonucleotide therapeutics, to muscles.

### BACKGROUND OF THE INVENTION

In the muscular system, muscle tissue is categorized into three distinct types: skeletal, cardiac and smooth muscle tissues. Each type of muscle tissue in the human body has a unique structure and a specific role. Muscular diseases are usually associated with muscle weakness or muscle dysfunction that can lead to lifethreatening complications. Many examples of such diseases have been characterized, including various forms of muscular dystrophy and neuromuscular disorders. Muscular dystrophy is a group of inherited diseases that gradually cause the muscles to weaken, leading to an increasing level of disability. There are various types of muscular dystrophy, with each type involving an eventual loss of strength and possible deformity. These include Duchenne muscular dystrophy (DMD), Becker muscular dystrophy (BMD), Facioscapulohumeral Muscular Dystrophy (FSHD), Pompe disease, familial hypertrophic cardiomyopathy, etc. Neuromuscular disorders affect the nerves that control voluntary muscles and the nerves that communicate sensory information back to the brain. They represent multifaceted abnormal conditions, with little or no cure, leading to patient deaths from complete muscle wasting and atrophy. These disorders include Spinal Muscular Atrophy (SMA), Amyotrophic Lateral Sclerosis (ALS), Charcot-Marie Tooth (CMT), Multiple Sclerosis (MS), etc. Many muscular diseases are single gene disorders associated with gain-of-function or loss-of-function mutations, which may have dominant or recessive phenotypes. For example, activating mutations have been identified in genes encoding ion channels, structural proteins, metabolic proteins, and signaling proteins that contribute to muscle disease. For example, DMD is caused by mutations of the DMD gene located on the short arm (p) of the X chromosome (Xp21.2). SMA is caused by deletion or mutation in the SMN1 gene, which encodes a protein known as survival motor neuron (SMN). This protein plays an important role in the functioning and maintenance of motor neurons. Despite advances in understanding the genetic etiology of muscular diseases, effective treatment options remain very limited.

RNA therapeutics are a new drug class designed to prevent and/or treat a specific disease using RNA-based molecules. They comprise a diverse group of oligonucleotide-based drugs such as antisense oligonucleotides (ASOs), small interfering RNAs (siRNAs), short hairpin RNAs (shRNAs), double-stranded RNA (dsRNA), micro-RNA (miRNA), etc. that can be designed to selectively interact with biological targets in the context of diseases which currently cannot be addressed or properly addressed with small molecule-based drugs or monoclonal antibodies. They are promising therapeutic solutions to modulate gene expression and to address mutations in several pathological conditions such as muscular disorders. Furthermore, RNA-based therapeutics have the potential to modulate entire disease pathways, and thereby may provide better treatment options to target the pathophysiological mechanisms of various disorders, which may lead to better outcomes for patients. Additionally, many RNA-based therapeutics have already been approved by the United States Food and Drug Administration (FDA) and the European Medical Agency (EMA), with increasing number of therapies in various phases of clinical trials demonstrating the validity of such RNA therapies for various diseases. However, its anionic charge and its susceptibility to RNases present in both the bloodstream and tissues make it difficult for a therapeutic RNA to enter cells efficiently and function on its own. Delivery remains the central challenge to the therapeutic application of RNA therapeutics. RNA therapeutics are ferried into their target cells using available delivery systems. Various strategies have been developed. These include lipid particles (LNPs) and direct conjugation to delivery agents (e.g., cholesterol). Significant progress has been made in recent years using N-acetylgalactosamine (GalNAc) as a targeting ligand for liver specific asialoglycoprotein receptor (Nair JK et al., Tai, W, Debacker et al.). Indeed, it was demonstrated that GalNAc-siRNA conjugates can successfully silence liver-expressed genes. However, there is a need for efficient strategies to prevent or treat muscle diseases.

### SUMMARY OF THE INVENTION

The present invention relates to particular VHH molecules that advantageously target transferrin receptor (TfR) in muscle tissues, and uses thereof to transport various therapeutic agents to muscles. More particularly, the invention provides conjugate compounds comprising such VHH molecules, which are optimized for addressing muscles and mediating effective functional delivery of oligonucleotide therapeutics to muscle tissues. The invention demonstrates that the conjugate compounds of the invention can effectively accumulate in the muscles and deliver conjugated therapeutics in the muscle tissue, and thus are suitable for efficient prevention and treatment of muscular diseases.

An object of the invention thus relates to a VHH molecule which binds TfR in muscle tissue.

A further object of the invention relates to a conjugate compound comprising one or more VHH molecule(s) which bind TfR in muscle tissue.

A particular object of the invention is a conjugate compound comprising: (i) one or more VHH molecule(s) of formula FR1-CDR1-FR2-CDR2-FR3-CDR3-FR4, and (ii) one or more oligonucleotide(s), wherein said VHH molecule binds TfR at the surface of a muscle cell.

Preferably, the muscle cell is a skeletal cell, a cardiac muscle cell or a muscle cancer cell.
Preferred VHH molecules according to the invention bind to TfR with an affinity (Kd) of 1 pM to 10 µM, even more preferably with a Kd of 0.01 nM to 4 µM or 0.01 nM to 500 nM or 0.01 nM to 100 nM or 0.1 nM to 4 µM or 0.1 nM to 500 nM or 0.1 nM to 100 nM.

In another aspect, preferred VHH molecules according to the invention bind human, non-human primate and/or rodent TfR1 at the surface of muscle cells.

In a particular aspect, the VHH molecule according to the invention comprises:
- a CDR1 comprising a sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710, 711, and/or
- a CDR2 comprising a sequence selected from SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608 and 611, 712, and/or
- a CDR3 comprising a sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 and 713-715.

In another particular aspect, the VHH molecule according to the invention comprises SEQ ID NOs: 1, 2 and 3; or SEQ ID NOs: 5, 6 and 7; or SEQ ID NOs: 9, 10 and 11; or SEQ ID NOs: 13, 14 and 15; SEQ ID NOs: 17, 2 and 3; or SEQ ID NOs: 19, 2 and 3; or SEQ ID NOs: 1, 21 and 3; or SEQ ID NOs: 1, 23 and 3; or SEQ ID NOs: 1, 2 and 25; or SEQ ID NOs: 1, 2 and 27; or SEQ ID NOs: 1, 2 and 29; or SEQ ID NOs: 1, 2 and 31; or SEQ ID NOs: 1, 2 and 33; or SEQ ID NOs: 67, 2 and 3; or SEQ ID NOs: 69, 2 and 3; or SEQ ID NOs: 1, 71 and 3; or SEQ ID NOs: 1, 73 and 3; or SEQ ID NOs: 1, 75 and 3; or SEQ ID NOs: 1, 2 and 77; or SEQ ID NOs: 1, 2 and 79; or SEQ ID NOs: 1, 2 and 81; or SEQ ID NOs: 1, 2 and 83; or SEQ ID NOs: 1, 2 and 85; or SEQ ID NOs: 392, 2 and 3; or SEQ ID NOs: 1, 113 and 3; or SEQ ID NOs: 1, 115 and 3; or SEQ ID NOs: 1, 2 and 117; or SEQ ID NOs: 1, 2 and 119; or SEQ ID NOs: 1, 2 and 121; or SEQ ID NOs: 1, 2 and 123; or SEQ ID NOs: 125, 2 and 3; or SEQ ID NOs: 17, 73 and 3; or SEQ ID NOs: 17, 128 and 3; or SEQ ID NOs: 5, 160 and 7; or SEQ ID NOs: 5, 162 and 7; or SEQ ID NOs: 5, 164 and 7; or SEQ ID NOs: 5, 166 and 7; or SEQ ID NOs: 9, 169 and 11; or SEQ ID NOs: 9, 171 and 11; or SEQ ID NOs: 175, 176 and 177; or SEQ ID NOs: 179, 176 and 180; or SEQ ID NOs: 182, 176 and 177; or SEQ ID NOs: 184, 176 and 177; or SEQ ID NOs: 186, 187 and 188; or SEQ ID NOs: 190, 191 and 192; or SEQ ID NOs: 194, 195 and 196; or SEQ ID NOs: 198, 199 and 200; or SEQ ID NOs: 201, 202 and 203; or SEQ ID NOs: 205, 206 and 207; or SEQ ID NOs: 410, 6 and 7; or SEQ ID NOs: 413, 6 and 7; or SEQ ID NOs: 5, 416 and 7; or SEQ ID NOs: 5, 419 and 7; or SEQ ID NOs: 426, 6 and 7; or SEQ ID NOs: 5, 431 and 7; or SEQ ID NOs: 434, 6 and 7; or SEQ ID NOs: 437, 6 and 7; or SEQ ID NOs: 5, 6 and 452; or SEQ ID NOs: 5, 6 and 455; or SEQ ID NOs: 607, 608 and 609; or SEQ ID NOs: 610, 611 and 612; or SEQ ID NOs: 671, 2 and 3; or SEQ ID NOs: 672, 2 and 3; or SEQ ID NOs: 673, 6 and 7; or SEQ ID NOs: 674, 6 and 7; or SEQ ID NOs: 1, 2 and 713; or SEQ ID NOs: 5, 6 and 714; or SEQ ID NOs: 674, 164 and 7; or SEQ ID NOs: 710, 6 and 7; or SEQ ID NOs: 5, 6 and 715; or SEQ ID NOs: 674, 712 and 7; or SEQ ID NOs: 711, 6 and 7.

In a further particular aspect, the VHH molecule comprises an amino acid sequence selected from any one of SEQ ID NOs: 213-271, 273-299, 412, 415, 418, 421, 423, 425, 428, 430, 433, 436, 439, 441, 443, 445, 447, 449, 451, 454, 457, 613-615, 675-678 and 701-709, wherein said amino acid sequence optionally comprises a tag and/or a linker.

In a further particular aspect, the VHH molecule comprises an amino acid sequence selected from any one of SEQ ID NOs: 214, 273, 276-284, 412, 415, 418, 421, 423, 425, 428, 430, 433, 436, 439, 441, 443, 445, 447, 449, 451, 454, 457, 677, 678 and 702-709. In a further particular aspect, the VHH molecule comprises an amino acid sequence selected from any one of SEQ ID NOs: 213, 216-271, 274, 275, 675, 676 and 701. In a further particular aspect, the VHH molecule comprises an amino acid sequence selected from any one of SEQ ID NOs: 215 and 285-299. In a further particular aspect, the VHH molecule comprises an amino acid sequence selected from any one of SEQ ID NOs: 613-615. In a further particular aspect, the VHH molecule is humanized, and preferably selected from SEQ ID NO: 87-92, 130-149, 152-154, 236-241, 252-271 and 273-275.

In another particular aspect, the oligonucleotide according to the invention is selected from any singleor double-stranded oligonucleotide such as small interfering RNA (siRNA), small activating RNAs (saRNA), gapmer, antisense oligonucleotide (ASO), shRNA, miRNA, aptamer RNA and bridged nucleic acid (BNA).

Another object of the invention relates to a conjugate compound, wherein the VHH molecule is conjugated to the oligonucleotide by covalent or non-covalent binding, directly or via at least one conjugation linker. The conjugate compound may further comprise at least one additional compound.

In a particular aspect, the additional compound is a half-life extending moiety or stabilizing group or scaffold such as an antibody or a fragment thereof (such as a Fc fragment), a VHH molecule, PEG, a serum albumin protein or a serum albumin binding moiety, preferably an Fc fragment. Preferably, the Fc fragment is a Fc heterodimer comprising a modified Fc having a sequence of SEQ ID NO: 664 on the knob arm, and a modified Fc having a sequence of SEQ ID NO: 665 on the hole arm.

Another object of the invention relates to a pharmaceutical composition comprising a conjugate compound as described herein, and a pharmaceutically acceptable support, carrier or excipient.

The VHH molecules, conjugate compounds and pharmaceutical compositions of the invention may be administered via any conventional route, preferably parenterally, more preferably systemically, intravenously, intramuscularly or subcutaneously. The invention may be used in any mammal, particularly in human subjects. It is suitable to treat any muscular or neuromuscular disease, such as a myopathy, a cardiomyopathy, a muscular dystrophy (such as DMD, BMD, FSHD, Pompe disease or familial hypertrophic cardiomyopathy), a peripheral neuropathy, ALS, SMA, MS, CMT, or a cancer of the muscle (such as rhabdomyosarcoma or leiomyosarcoma).

### LEGEND TO THE FIGURES

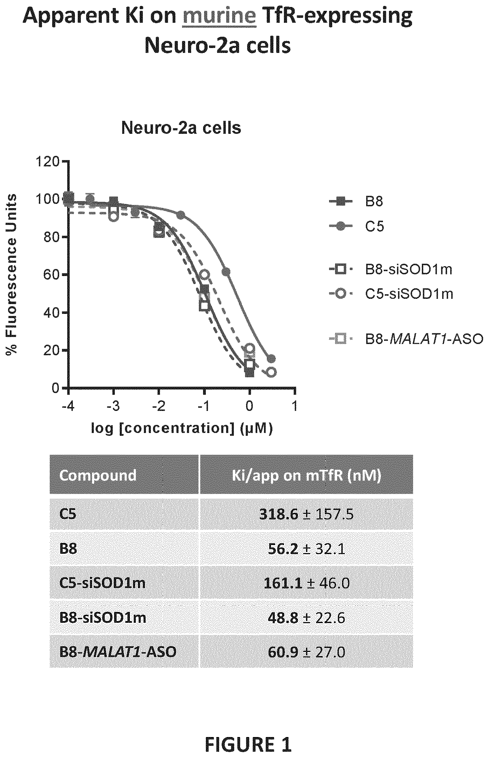

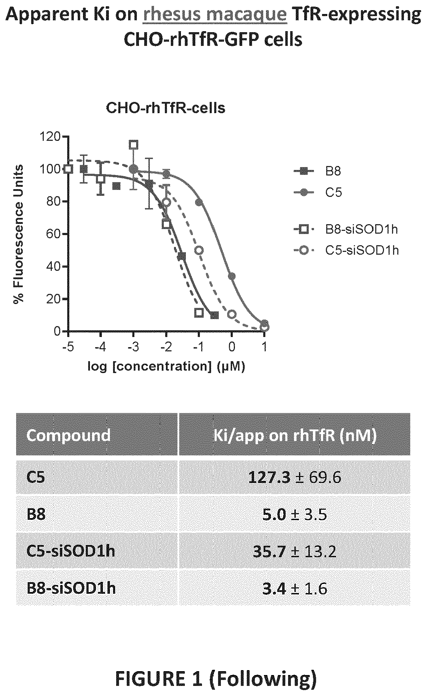

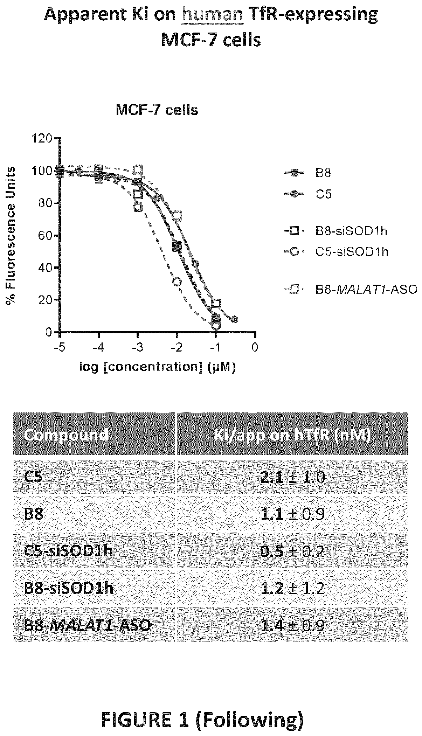

**Figure 1** shows concentration-dependent binding and apparent binding affinity values (Ki/app) of VHH-Oligo conjugates (such as B8-siSOD1m, C5-siSOD1m, B8-*MALAT1*-ASO, B8-siSOD1h or C5-siSOD1h) and free VHHs (B8 or C5), in competition with a fluorescent reference TfR-binding VHH, to murine, macaque rhesus or human TfR expressed by murine Neuro-2A cells, CHO cells stably expressing rhesus TfR or human MCF-7 cells, respectively.

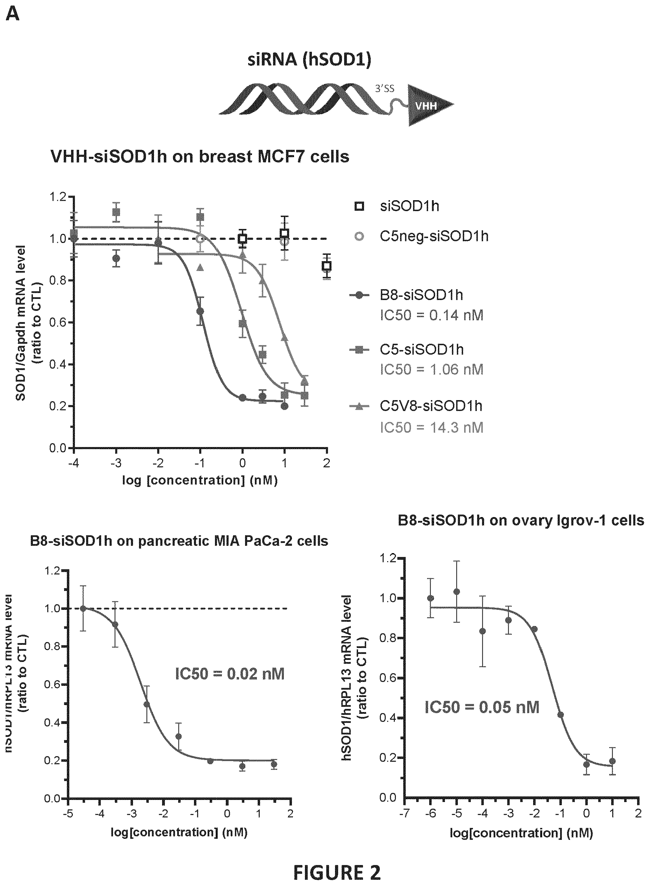

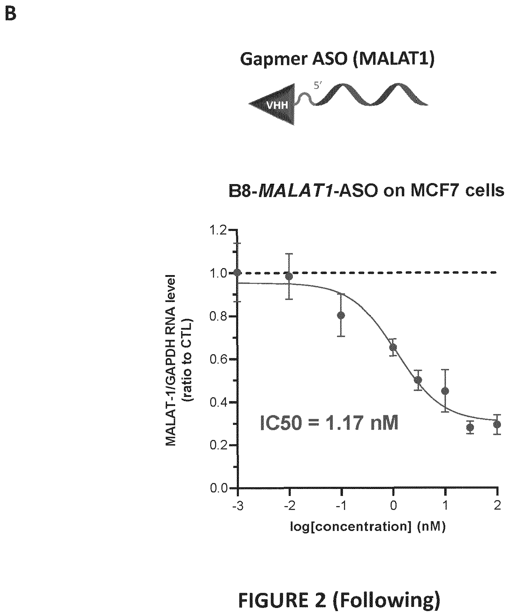

**Figure 2** shows concentration-dependent downregulation of mRNA or lncRNA levels after free uptake on human MCF-7 cells or MIA PaCa-2 cells of either (A) TfR-binding VHH-siRNA conjugates targeting the human *SOD1* mRNA (VHH-siSOD1h wherein VHH is B8, C5 or C5V8) or a non-binding conjugate (C5neg-siSOD1h), or (B) a TfR-binding VHH-ASO conjugate, targeting the human and mouse *MALAT-1* long non-coding RNA (VHH-*MALAT1*-ASO wherein VHH is B8).

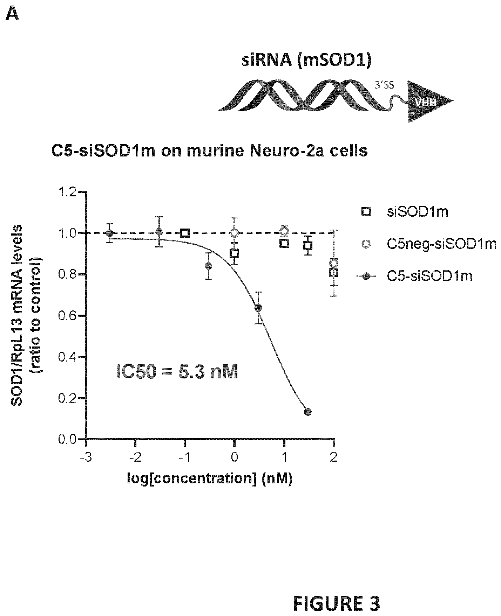

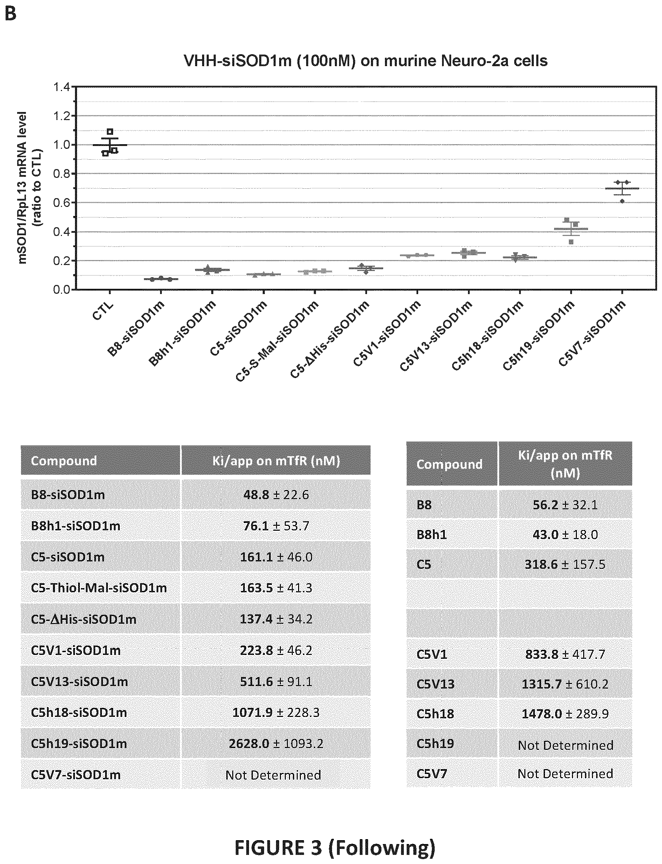

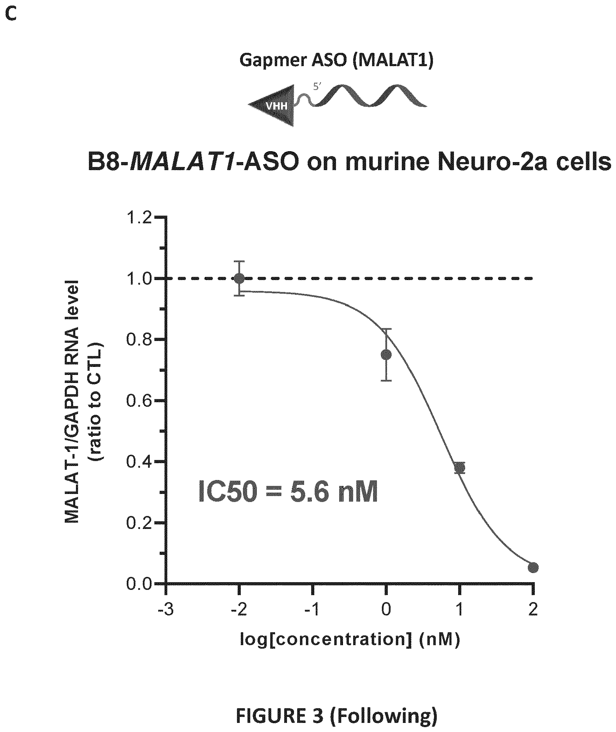

**Figure 3** shows mRNA or lncRNA downregulation potential of TfR-binding VHH-Oligo conjugates after free uptake on the murine Neuro-2A cell line. (A) Figure 3A shows concentration-dependent downregulation of *SOD1* mRNA levels obtained with a TfR-binding VHH-siRNA conjugate (VHH-siSOD1m wherein VHH is C5) or a non-binding conjugate (C5neg-siSOD1m). (B) Figure 3B shows downregulation of *SOD1* mRNA levels obtained with various examples of TfR-binding VHH-siRNA conjugates targeting the murine *SOD1* mRNA (i.e., VHH-siSOD 1m or VHH-thiol-Mal-siSOD 1m or VHH-ΔHis-siSOD1m, wherein VHH is or comprises C5, B8, B8h1, C5V1, C5V13, C5h18, C5h19 or C5V7) and apparent binding affinity values (Ki/app) of tested conjugates for TfR evaluated on murine Neuro-2A cells as shown in Figure 1. (C) Figure 3C shows downregulation of *MALAT-1* lncRNA levels obtained with a VHH-MALATI-ASO conjugate targeting the human and mouse *MALAT-1* long non-coding RNA (VHH-*MALAT1*-ASO wherein VHH is B8).

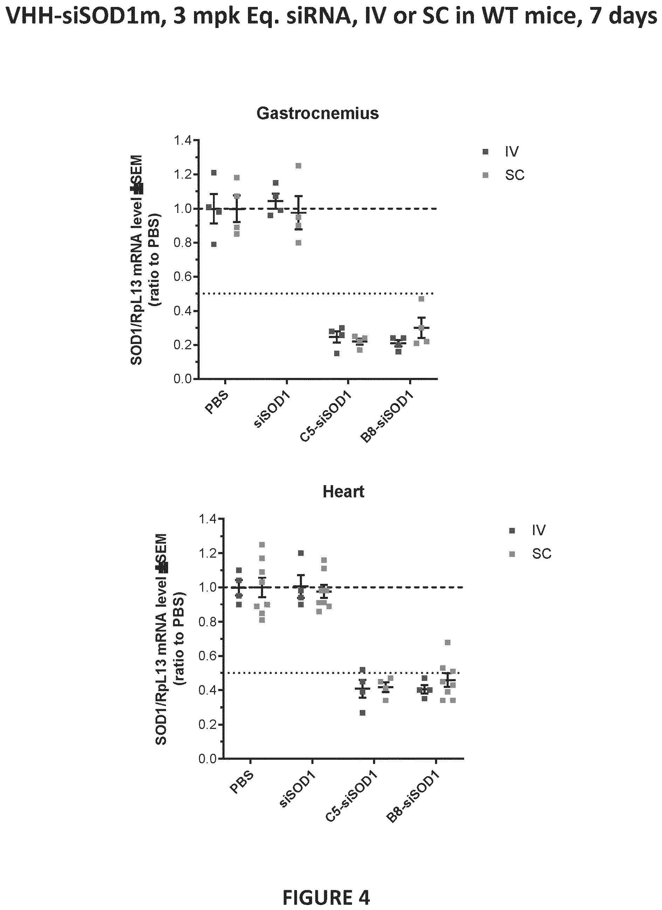

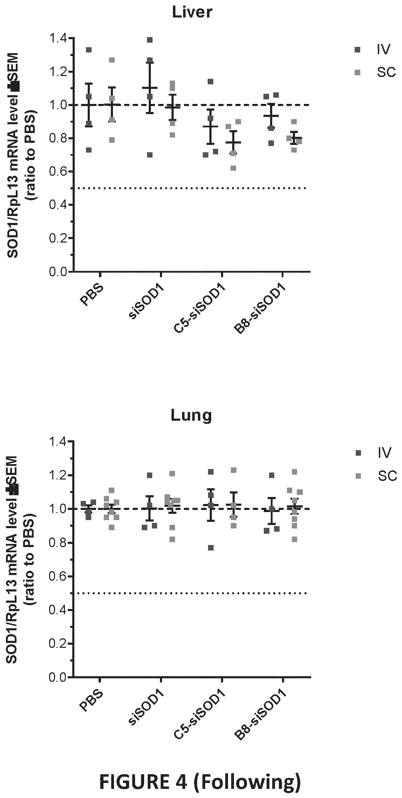

**Figure 4** shows muscle-specific downregulation of murine *SOD1* mRNA levels following single systemic (intravenous IV or subcutaneous SC) administration of VHH-siRNA conjugates (wherein VHH is C5 or B8) in wild-type C57Bl/6 mice.

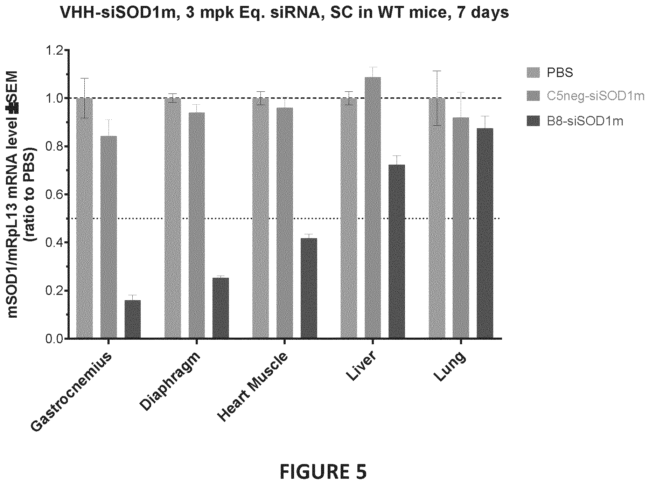

**Figure 5** shows TfR-specific and muscle-specific downregulation of murine *SOD1* mRNA levels following single subcutaneous administration of a TfR-binding VHH-siSOD1 conjugate (wherein VHH is B8) in wild-type C57Bl/6 mice.

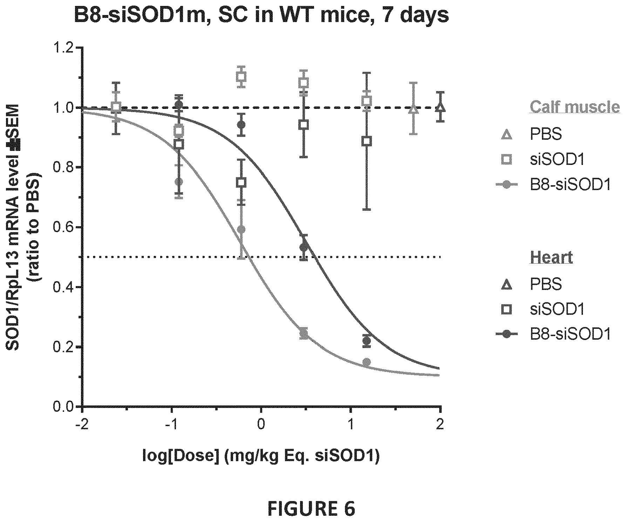

**Figure 6** shows dose-dependent downregulation of murine *SOD1* mRNA levels in skeletal muscle (gastrocnemius) or cardiac muscle following single subcutaneous administration of a TfR-binding VHH-siSOD1m conjugate (wherein VHH is B8) in wild-type C57Bl/6 mice.

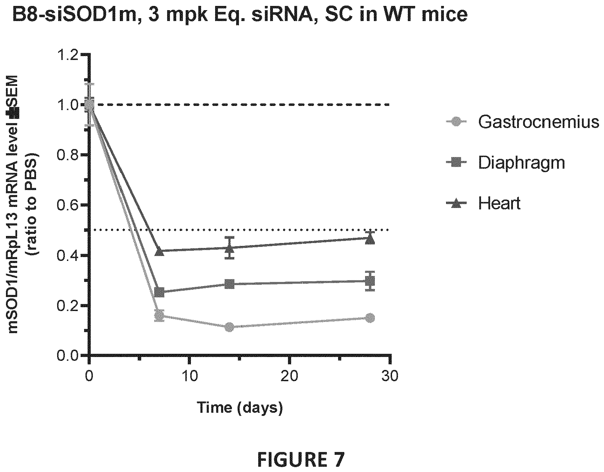

**Figure 7** shows the time-course of the murine *SOD1* mRNA downregulation in muscle tissues (e.g., gastrocnemius, diaphragm, heart tissue) following single subcutaneous (SC) administration of a TfR-binding VHH-siSOD1m conjugate (wherein VHH is B8) in wild-type C57Bl/6 mice.

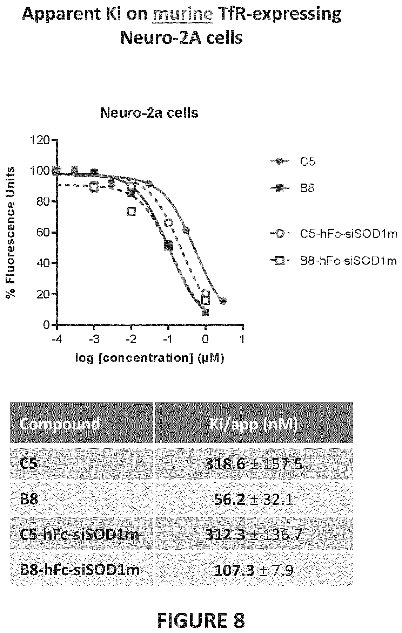

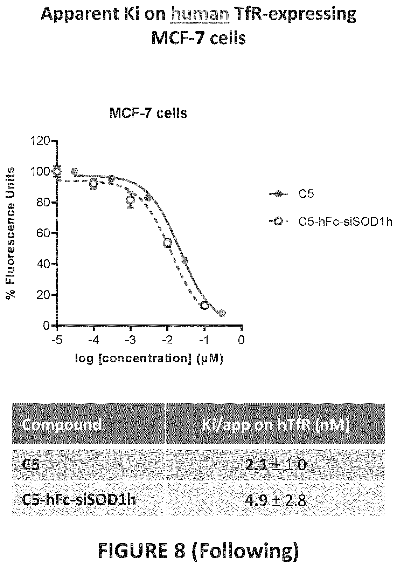

**Figure 8** shows concentration-dependent binding and apparent binding affinity values (Ki/app) of a TfR-binding VHH-hFc-siSOD1 conjugate (wherein VHH is C5 or B8), in competition with a fluorescent reference TfR-binding VHH, to murine and human TfR expressed by murine Neuro-2A and human MCF-7 cells, respectively.

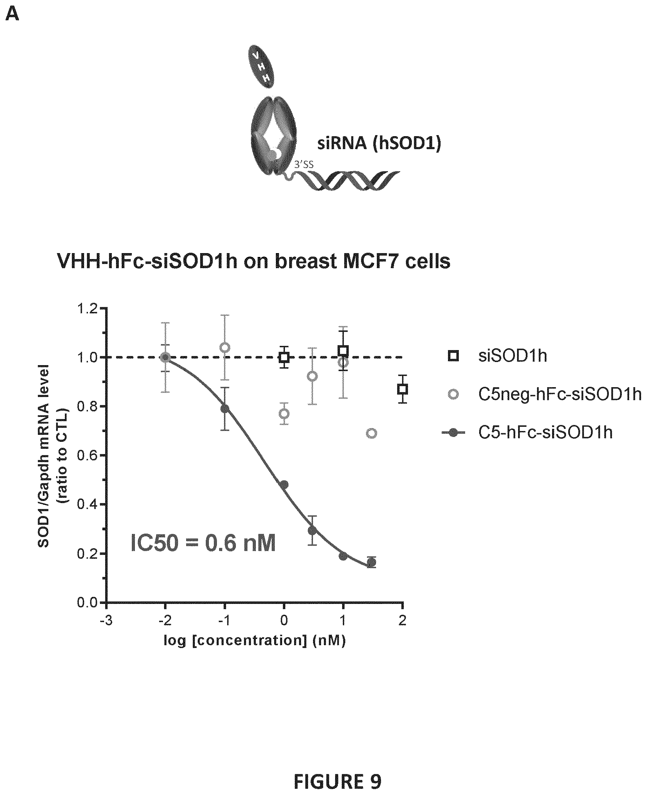

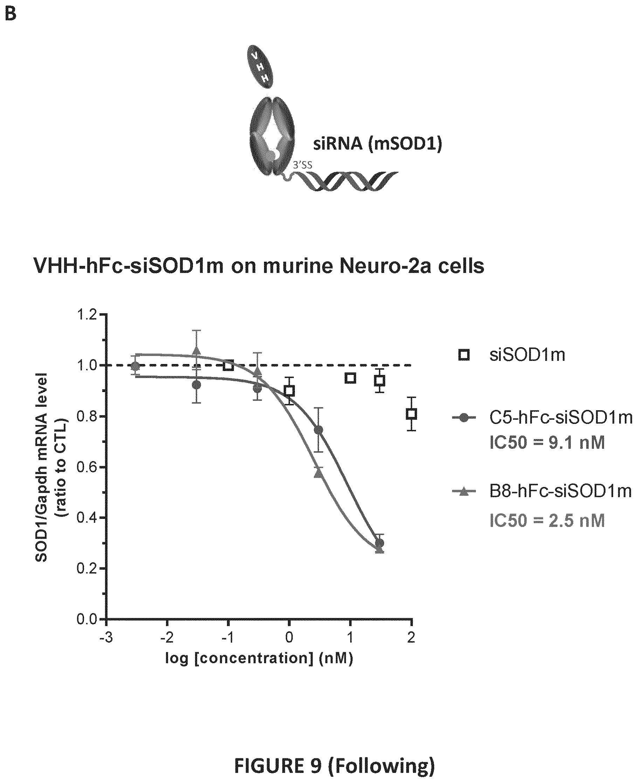

**Figure 9** shows concentration-dependent downregulation of human or murine *SOD1* mRNA levels after free uptake on (A) human breast MCF-7 cells and (B) murine Neuro-2A cells, respectively, of TfR-binding VHH-hFc-siSOD1 conjugates (wherein VHH is C5 or B8) targeting the human or murine *SOD1* mRNA.

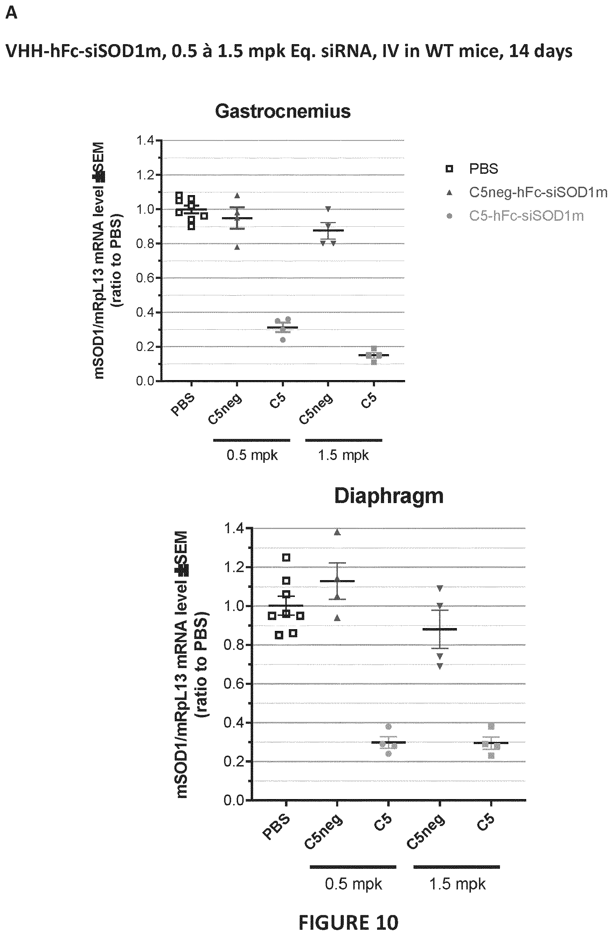

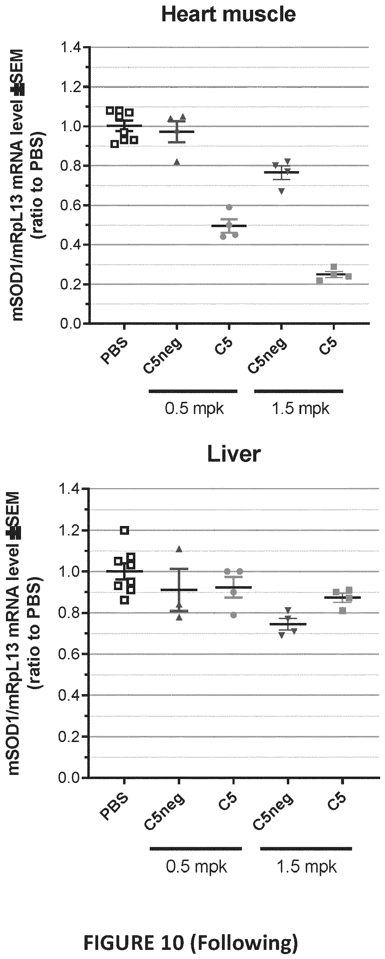

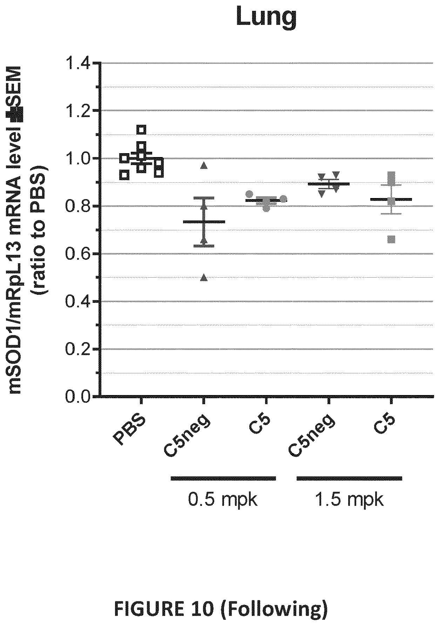

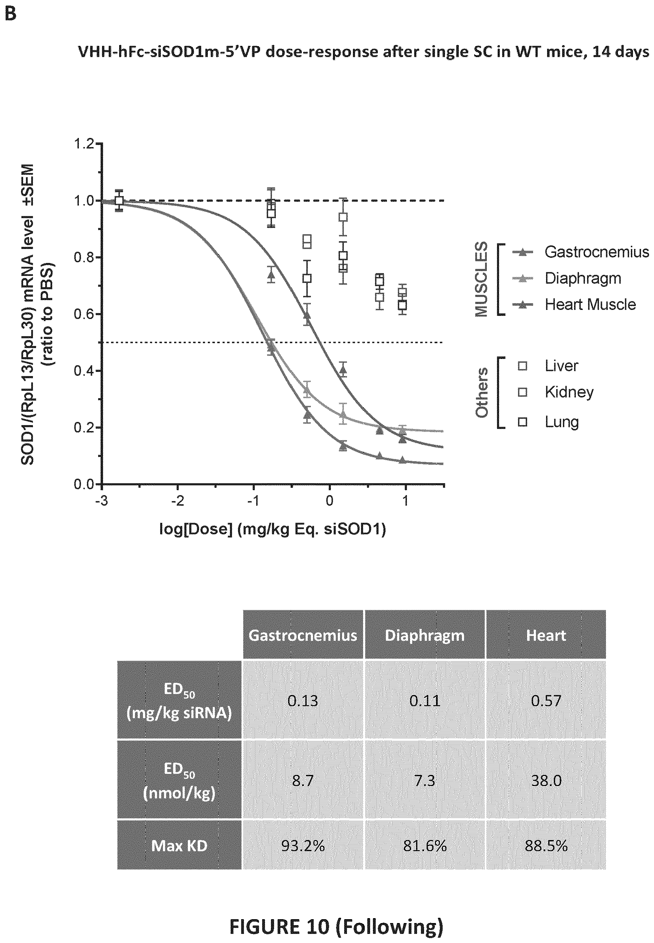

**Figure 10** shows muscle-specific and dose-dependent downregulation of murine SOD1 mRNA levels in wild-type C57B1/6 mice following (A) single intravenous administration of a TfR-binding VHH-hFc-siSOD1m conjugate (wherein VHH is C5) in wild-type C57Bl/6 mice, or (B) single subcutaneous administration of a TfR-binding VHH-hFc-siSOD1m-5'VP conjugate (wherein VHH is C5) with estimated ED50 values and maximal downregulation effect (Max KD) in muscle tissues.

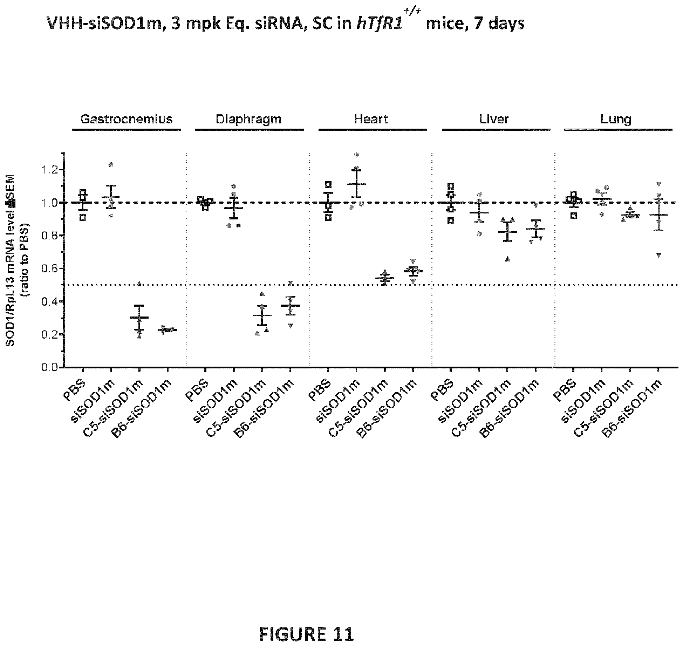

**Figure 11** shows muscle-specific downregulation of murine *SOD1* mRNA levels following single subcutaneous (SC) administration of a TfR-binding VHH-siSOD1m conjugate (wherein VHH is C5 or B6) in *hTfR1*^{*+*/*+*} -KI mice. No downregulation of murine SOD1 mRNA levels was observed in any of the tested samples analyzed after treatment with the unconjugated siSOD1m; both hTfR-binding conjugates induced similar potent and muscle tissue-selective effect, with about 70-80% knock-down in gastrocnemius and diaphragm, and about 40-50% knock-down in cardiac muscle, while no effect in the liver or lungs.

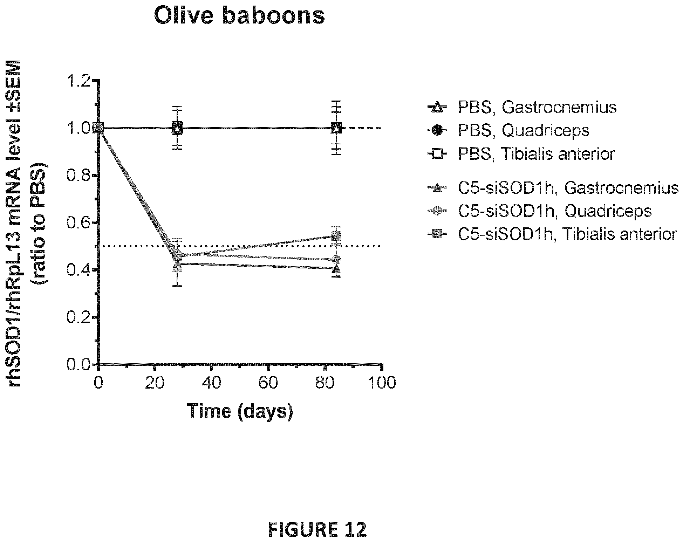

**Figure 12** shows the time-course of the *SOD1* mRNA downregulation in muscle tissues following single intravenous administration of a TfR-binding VHH-siSOD1h conjugate (wherein VHH is C5) in various muscular tissues (such as gastrocnemius, quadriceps or tibialis anterior tissue) of olive baboons.

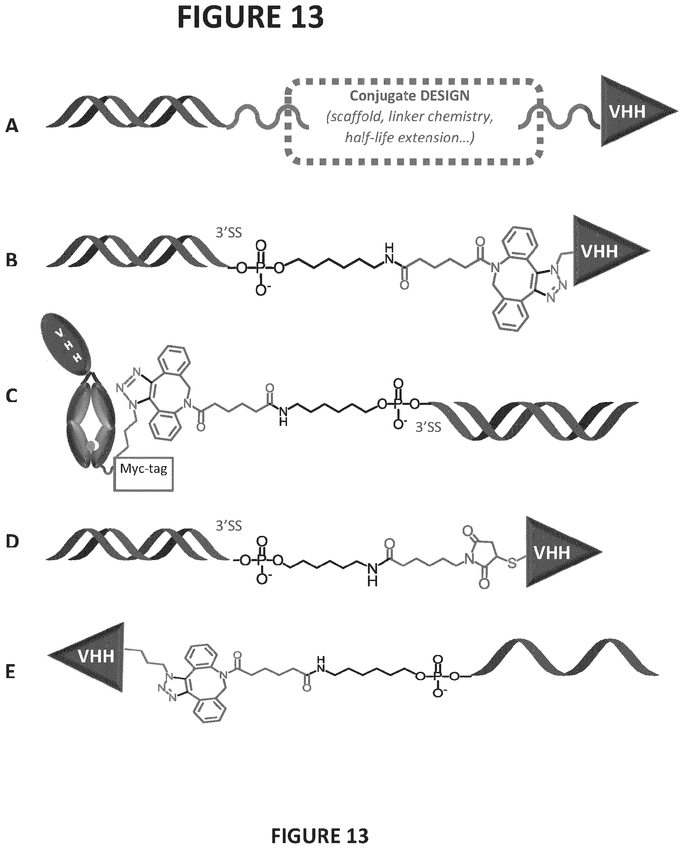

**Figure 13** shows (A) the general structure of VHH-Oligo conjugates of the present invention comprising i) the oligo moiety, which can be any oligonucleotide-based drug such as a single-stranded ASO or a duplex siRNA, ii) the VHH and iii) a linking moiety, which can consist of a half-life extending entity onto which the oligo and the VHH are linked at two distinct sites, and (B-E) a detailed structure of the linker used for some of the conjugates evaluated in the experimental part such as a VHH-siRNA conjugate (B), a VHH-hFc-siRNA conjugate (C), a VHH-thiol-Mal-siRNA (D) or a VHH-ASO conjugate (E).

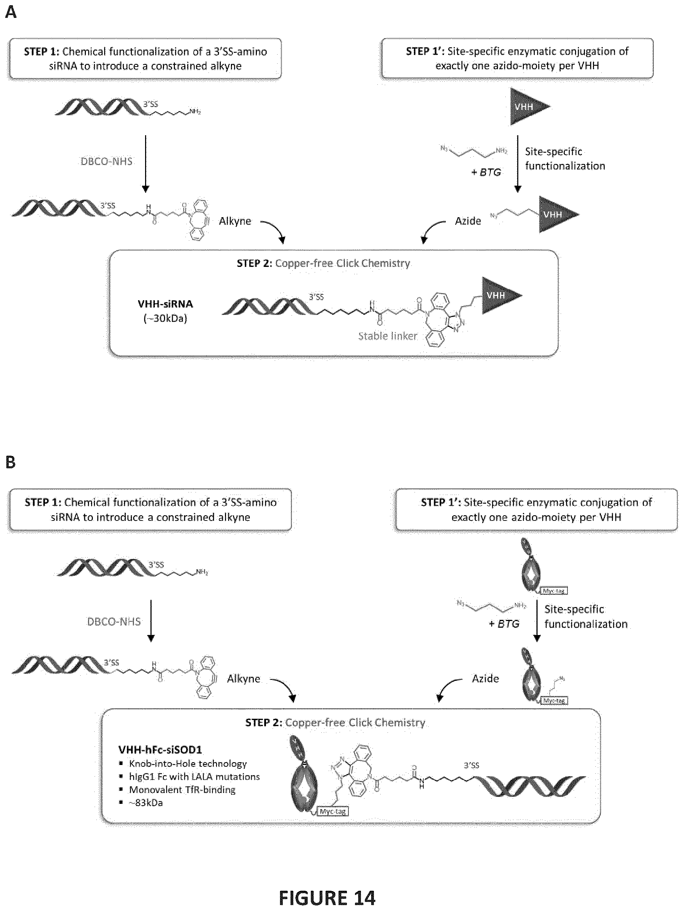

**Figure 14** shows examples of a conjugation strategy yielding VHH-Oligo (A) or heterodimeric VHH-hFc-Oligo (B) conjugates with a stable linker. Such a general conjugation strategy involves a convergent synthesis with the parallel modification of: (i) the VHH (A) or the heterodimeric VHH-hFc (B) to site-specifically introduce for example an azido-linker; and (ii) the oligonucleotide to introduce for example a constrained alkyne group complementary to the azido functional group. In a final step, both functionalized azido-VHH (A) or VHH-hFc-azido (B) and alkyne-Oligo precursors are linked to each other using preferably a copper-free click reaction, yielding VHH-Oligo or VHH-hFc-Oligo conjugates with a stable linker.

### DETAILED DESCRIPTION OF THE INVENTION

The present invention provides novel transferrin receptor (TfR)-binding agents which can be used to transport molecules, such as therapeutic, imaging or diagnostic agents, to muscles. More particularly, the invention discloses improved VHH molecules which bind TfR in muscle tissues and cells, and uses thereof. Accordingly, targeting drugs to TfR is particularly suitable for treating any muscular or neuromuscular disease, such as a myopathy, a cardiomyopathy, a muscular dystrophy (such as DMD, BMD, FSHD, Pompe disease or familial hypertrophic cardiomyopathy), a peripheral neuropathy, ALS, SMA, MS or CMT.

TfR is involved in iron transport into cells and organs by its ligand transferrin (Tf). This receptor was shown to be highly expressed in brain endothelium (Jefferies et al., Pardridge et al.), albeit it is also abundant in blood cells and lung (Chan and Gerhardt). This receptor has been used to deliver pharmacological agents across the blood brain barrier (Johnsen et al., WO2012075037, WO2016208695, WO2020144233, etc.). Furthermore, the TfR has been described as playing an important role in muscle development (Ying Li et al.). In addition to its role in muscle development, the TfR plays a crucial functional role in iron homeostasis in muscle tissues and systemic metabolism (Ying Li et al., Barrientos T. et al., Xu W., et al.). The TfR is involved in the incorporation of iron, transported by its transferrin ligand, and in the regulation of cell growth (Neckers and Trepel 1986, Ponka and Lok 1999).

There are two types of transferrin receptors: TfR1 and a homologous receptor, TfR2, expressed primarily in the liver. In the context of the invention, the term TfR is used to designate the TfR1 homologue. TfR is a type II homodimeric transmembrane glycoprotein consisting of two identical 90 kDa subunits linked by two disulfide bridges (Jing and Trowbridge 1987, McClelland et al., 1984). Each monomer has a short cytoplasmic N-terminal domain of 61 amino acids containing a YTRF (Tyrosine-Threonine-Arginine-Phenylalanine) internalization motif, a single hydrophobic transmembrane segment of 27 amino acids, and a broad C-terminal extracellular domain of 670 amino acids, containing a trypsin cleavage site and a transferrin binding site (Aisen, 2004). Each subunit is capable of binding a transferrin molecule. The extracellular domain has one O-glycosylation site and three N-glycosylation sites, the latter being particularly important for the proper folding and transport of the receptor to the cell surface (Hayes et al., 1997). There are also palmitoylation sites in the intramembranous domain, that presumably anchor the receptor and allow its endocytosis (Alvarez et al., 1990, Omary and Trowbridge, 1981). In addition, an intracellular phosphorylation site is present, whose functions are uncertain, and which plays no role in endocytosis (Rothenberger et al., 1987).

The TfR receptor is expressed at high level by highly proliferating cells, whether healthy or neoplastic (Gatter et al., 1983). Many studies have shown high levels of TfR expression in cancer cells compared to healthy cells. Thus, pathologies such as breast cancer (Yang et al., 2001), gliomas (Prior et al., 1990), pulmonary adenocarcinoma (Kondo et al., 1990), chronic lymphocytic leukemia (Das Gupta and Shah, 1990) or non-Hodgkin's lymphoma (Habeshaw et al., 1983) show increased TfR expression, correlated with tumor grade and stage of disease or prognosis. Targeting drugs to TfR may thus be suitable for cancer treatment, especially for the treatment of a cancer of the muscle such as rhabdomyosarcoma, leiomyosarcoma, etc.

Using purified membrane preparations from cells expressing high levels of hTfR and mTfR, inventors generated and selected VHH molecules, in particular VHH molecules that bind both the human and non-human TfR. They also showed that when fused to an oligonucleotide such as siRNA or ASO, these VHH molecules retain TfR binding capacity and efficient delivery *in vivo,* in muscle cells and other cell types.

The VHH molecules exhibit appropriate levels of affinity and specificity to undergo proper endocytosis following TfR binding. The invention thus provides novel TfR-binding molecules which represent valuable agents for drug targeting to muscles. More particularly, inventors showed that conjugates of the invention comprising a VHH molecule and an oligonucleotide such as a siRNA, bind TfR in various muscle tissues and mediate target mRNA downregulation *in vivo* in WT mice expressing the murine TfR1 or in humanized in *hTfR1*^{*+*/*+*} mice expressing the human Tfr1 and in non-human primates. For example, TfR-binding conjugates according to the invention induced potent and muscle tissue-selective effect reaching more than 70 % mRNA knock-down in gastrocnemius, diaphragm, and cardiac muscle. Among the strategies evaluated to deliver RNA therapeutics to their target organs/tissues is receptor-mediated transcytosis (RMT), a physiological process involving binding of a ligand to its receptor expressed at the organ/tissue. Interestingly, a single systemic administration of TfR-binding VHH-siRNA conjugate induced a potent and durable downregulation of the target mRNA levels in all muscle tissues tested in mice as well as non-human primates (such as gastrocnemius, quadriceps and tibialis anterior muscle), with 60% knock-down observed for more than three months in non-human primates. Overall, inventors demonstrated cross-species rodent/NHP/human ability of the TfR-binding VHH-siRNA conjugates to mediate TfR-dependent binding, functional uptake and durable target mRNA downregulation *in vivo* at low therapeutic dose in skeletal and cardiac muscle tissues. Furthermore, inventors also showed that when conjugated to RNA therapeutics (such as a siRNA or ASO) via an antibody or antibody fragment scaffold, such as a human IgG1 Fc region, the VHH molecules according to the invention retain TfR binding capacity *in vitro,* and demonstrate potent and muscle-selective targeting and functional uptake properties *in vivo* at low dose.

An object of the invention thus relates to VHH molecules, wherein said VHH molecules bind both a human and a non-human (e.g., non-human primate (NHP) or rodent, such as rat or murine) TfR. Preferably, the VHH bind TfR-expressing muscle tissues. The invention also relates to conjugates comprising such VHH, their manufacture, compositions comprising the same and the use thereof.

### Definitions

Unless otherwise defined herein, all scientific and technical terms used in connection with the present invention have the same meaning as commonly understood by one of ordinary skill in the art.

As used herein, the term "treatment", "treat" or "treating" refers to any act intended to ameliorate the health status of patients such as therapy, prevention, prophylaxis and retardation of a disease such as a muscular disease, a neuromuscular disease or a cancer of the muscle, or of at least one symptom of the muscular disease, the neuromuscular disease or the cancer. It designates both a curative treatment and/or a prophylactic treatment of a muscular disease, of a neuromuscular disease or of a cancer. In the context of cancer, this includes, inter alia, the alleviation of symptoms, the reduction of inflammation, the inhibition of cancer cell growth, and/or the reduction of tumor size. For example, in the case of cancer, a response to treatment includes a reduction in cachexia, increase in survival time, elongation in time to tumor progression, reduction in tumor mass, reduction in tumor burden and/or a prolongation in time to tumor metastasis, time to tumor recurrence, tumor response, complete response, partial response, stable disease, progressive disease, progression free survival, overall survival, each as measured for example by standards set by the National Cancer Institute and the U.S. Food and Drug Administration for the approval of new drugs (Johnson et al., J. Clin. Oncol., 2009; 21(7): 1404-1411).

A "therapeutically effective dose" as described herein refers to the dose that gives a therapeutic effect for a given condition and administration schedule. It is typically the average dose of an active substance to administer to appreciably improve some of the symptoms associated with a disease or a pathological state. For example, in treating a cancer of the muscle (such as rhabdomyosarcoma or leiomyosarcoma) or of other tissues, a pathology, a lesion or a disorder affecting muscles (such as a muscular or neuromuscular disease), the dose of an active substance that decreases, prevents, delays, eliminates or stops one of the causes or symptoms of the disease or disorder would be therapeutically effective. A "therapeutically effective dose" of an active substance does not necessarily cure a muscle disease or disorder but will provide a treatment for this disease or disorder so that its appearance is delayed, impeded or prevented, or its symptoms are attenuated, or its term is modified or, for example, is less severe, or the recovery of the patient is accelerated.

"VHH molecules" as used herein correspond to the variable region of heavy chain only camelid antibodies that are naturally devoid of light chains. VHH have a very small molecular weight of around 12-15 kDa. They contain a single chain molecule that can bind its cognate antigen using a single domain. The antigen-binding surfaces of VHHs are usually more convex (or protruding) than those of conventional antibodies, which are usually flat or concave. More specifically, VHHs are composed of 4 Framework Regions (or FRs) whose sequences and structures are defined as conserved, and three Complementarity Determining Regions (or CDRs) showing high variability both in sequence content and structure conformation, which are involved in antigen binding and provide antigen specificity. Compared to conventional human antibody VH, a few amino acids are substituted in the FR2 region and complementarity-determining regions (CDRs) of VHH. For instance, highly conserved hydrophobic amino acids (such as Val42, Gly49, Leu50, and/or Trp52) in FR2 region are often replaced by hydrophilic amino acids (Phe42, Glu49, Arg50, Gly52), rendering the overall structure more hydrophilic and contributing to high stability, solubility and resistance to aggregation. VHH molecules according to the present invention are polypeptides comprising (or consisting of, or consisting essentially of) an antigen-binding domain of a heavy chain only antibody (HcAb). The term "and/or" as used herein is to be taken as specific disclosure of each of the two specified features or components with or without the other. For example, "A and/or B" is to be taken as specific disclosure of each of (i) A, (ii) B and (iii) A and B, just as if each is set out individually.

The term "a" or "an" can refer to one of or a plurality of the elements it modifies (e.g., "a VHH molecule" can mean one or more VHH molecules) unless it is contextually clear either one of the elements or more than one of the elements is described.

### VHH molecules

In order to generate VHH molecules having suitable properties, the inventors tested over 2000 TfR-binding VHH from a library of VHH produced by lama immunization with a TfR immunogen. Following analysis of said clones for binding and specificity, the inventors further selected about 450 clones which were all sequenced and compared. Further VHH with controlled/improved binding properties were produced by mutagenesis or by humanization. The sequences of the relevant domains and preferred VHH are provided in the experimental section and sequence listing. The properties of the VHH and conjugates thereof are also illustrated in the experimental section.

VHH molecules of the invention typically comprise or consist of the formula:
FR1-CDR1-FR2-CDR2-FR3 -CDR3 -FR4,
wherein FRn designates framework regions and CDRn designates complementarity determining regions.

In a particular embodiment, VHH molecules of the invention comprise a CDR1 domain comprising or consisting of an amino acid sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710 or 711, or variants thereof having at least 60%, in particular at least 65%, 70% or 75%, for example at least 80% or 85% amino acid identity to any one of said sequences over the entire length thereof, preferably at least 85%, 86%, 87%, 88%, 89%, 90%, 91%, 92%, 93%, 94%, 95%, 96%, 97%, 98% or 99% (the preferred percentages of identity for a particular sequence being preferably percentages corresponding to an integer number of amino acids), said variants retaining a TfR binding capacity. Preferred VHH molecules of the invention contain a CDR1 domain having an amino acid sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710, 711, or variants thereof having several amino acid modifications, for example at least 3 amino acid modifications, preferably at most 3 or 2 amino acid modifications, in a particular aspect at most 1 amino acid modification. The "% identity" between amino acid (or nucleic acid) sequences may be determined by techniques known per se in the art. Typically, the % identity between two nucleic acid or amino acid sequences is determined by means of computer programs such as GAP provided in the GCG program package (Program Manual for the Wisconsin Package, Version 8, August 1996, Genetics Computer Group, 575 Science Drive, Madison, Wisconsin, USA 53711) (Needleman, S.B. and Wunsch, C.D., (1970), Journal of Molecular Biology, 48, 443-453). The % identity between two sequences designates the identity over the entire length of said sequences. As indicated above, the preferred percentages of identity for a particular sequence are preferably percentages corresponding to an integer number of amino acids both in the reference sequence (which is for example SEQ ID NOs: 1, 5, 9, 13, 17, 19, 67, 69, 125, 175, 179, 182, 184, 186, 190, 194, 198, 201, 205, 392, 410, 413, 426, 434 or 437, 607, 610, 671-674, 710, 711, or any other reference sequences herein identified such as SEQ ID NOs: 2, 6, 10, 14, 21, 23, 71, 73, 75, 113, 115, 128, 160, 162, 164, 166, 169, 171, 176, 187, 191, 195, 199, 202, 206, 608, 611, 712, 3, 7, 11, 15, 25, 27, 29, 31, 33, 77, 79, 81, 83, 85, 117, 119, 121, 123, 177, 180, 188, 192, 196, 200, 203, 207, 609, 612 or 713-715 and in the variant thereof.

Specific examples of VHH molecules of the invention comprise a CDR1 sequence comprising, or consisting essentially of SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671 to 674, 710 or 711.

In a further particular embodiment, VHH molecules of the invention comprise a CDR2 domain comprising or consisting of an amino acid sequence selected from SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611 or 712, or variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, said variants retaining a TfR binding capacity. Preferred VHH molecules of the invention contain a CDR2 domain having an amino acid sequence selected from SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611 or 712, or variants thereof having several amino acid modifications, for example at least 3 amino acid modifications, preferably at most 3 or 2 amino acid modifications, in a particular aspect at most 1 amino acid modification.

Specific examples of VHH molecules of the invention comprise a CDR2 sequence comprising, or consisting essentially of SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611 or 712.

In a further particular embodiment, VHH molecules of the invention comprise a CDR3 domain comprising or consisting of an amino acid sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609 612 or 713-715, or variants thereof having at least 60%, in particular at least 65%, 70% or 75%, for example at least 80% or 85%, amino acid identity to any one of said sequences over the entire length thereof, preferably at least 80%, more preferably at least 85%, 86%, 87%, 88%, 89%, 90%, 91%, 92%, 93%, 94%, 95%, 96%, 97%, 98% or 99%, said variants retaining a TfR binding capacity. Preferred VHH molecules of the invention contain a CDR3 domain having an amino acid sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 or 713-715, or variants thereof having several amino acid modifications, for example at least 3 amino acid modifications, preferably at most 3 or 2 amino acid modifications, in a particular aspect at most 1 amino acid modification.

Specific examples of VHH molecules of the invention comprise a CDR3 sequence comprising, or consisting essentially of SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 or 713-715.

In a further particular embodiment, VHH molecules of the invention comprise:
- a CDR1 domain comprising or consisting of an amino acid sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710 or 711, or variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, more preferably at least 95%; and
- a CDR2 domain comprising or consisting of an amino acid sequence selected from SEQ ID NOs: 2, 6, 10, 14, 21, 23, 71, 73, 75, 113, 115, 128, 160, 162, 164, 166, 169, 171, 176, 187, 191, 195, 199, 202, 416, 419, 431, 206, 608, 611 or 712, or variants thereof having at least 60%, in particular at least 65%, 70% or 75%, for example at least 80% or 85%, amino acid identity to any one of said sequences over the entire length thereof, preferably at least 85%, 86%, 87%, 88%, 89%, 90%, 91%, 92%, 93%, 94%, 95%, 96%, 97%, 98% or 99%, more preferably at least 95%; and
- a CDR3 domain comprising or consisting of an amino acid sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 or 713-715, or variants thereof having at least 60%, in particular at least 65%, 70% or 75%, for example at least 80% or 85%, amino acid identity to any one of said sequences over the entire length thereof, preferably at least 80%, 85%, 86%, 87%, 88%, 89%, 90%, 91%, 92%, 93%, 94%, 95%, 96%, 97%, 98% or 99%, more preferably at least 95%,
said VHH having a TfR-binding capacity.

In a preferred embodiment, the VHH molecules of the invention comprise:
- a CDR1 domain having an amino acid sequence selected from the group consisting of SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710, 711 and variants thereof having at most 3, 2 or 1 amino acid modifications; and
- a CDR2 domain having an amino acid sequence selected from the group consisting of SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611, 712 and variants thereof having at most 3, 2 or 1 amino acid modifications; and
- a CDR3 domain having an amino acid sequence selected from the group consisting of SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612, 713-715 and variants thereof having at most 3, 2 or 1 amino acid modifications.

In a more preferred embodiment, the VHH molecules of the invention comprise a CDR1, a CDR2 and a CDR3, wherein said CDR1, CDR2 and CDR3 domains comprise or consist of, respectively:
. SEQ ID NOs: 1, 2 and 3; or
. SEQ ID NOs: 17, 2 and 3; or
. SEQ ID NOs: 19, 2 and 3; or
. SEQ ID NOs: 67, 2 and 3; or
. SEQ ID NOs: 69, 2 and 3; or
. SEQ ID NOs: 1, 21 and 3; or
. SEQ ID NOs: 1, 23 and 3; or
. SEQ ID NOs: 1, 71 and 3; or
. SEQ ID NOs: 1, 73 and 3; or
. SEQ ID NOs: 1, 75 and 3; or
. SEQ ID NOs: 1, 2 and 25; or
. SEQ ID NOs: 1, 2 and 27; or
. SEQ ID NOs: 1, 2 and 29; or
. SEQ ID NOs: 1, 2 and 31; or
. SEQ ID NOs: 1, 2 and 33; or
. SEQ ID NOs: 1, 2 and 77; or
. SEQ ID NOs: 1, 2 and 79; or
. SEQ ID NOs: 1, 2 and 81; or
. SEQ ID NOs: 1, 2 and 83; or
. SEQ ID NOs: 1, 2 and 85; or
. SEQ ID NOs: 5, 6 and 7; or
. SEQ ID NOs: 9, 10 and 11; or
. SEQ ID NOs: 13, 14 and 15, or
. SEQ ID NOs: 392, 2 and 3; or
. SEQ ID NOs: 1, 113 and 3; or
. SEQ ID NOs: 1, 115 and 3; or
. SEQ ID NOs: 1, 2 and 117; or
. SEQ ID NOs: 1, 2 and 119; or
. SEQ ID NOs: 1, 2 and 121; or
. SEQ ID NOs: 1, 2 and 123; or
. SEQ ID Nos: 125, 2 and 3; or
. SEQ ID NOs: 17, 73 and 3; or
. SEQ ID NOs: 17, 128 and 3; or
. SEQ ID NOs: 5, 160 and 7; or
. SEQ ID NOs: 5, 162 and 7; or
. SEQ ID NOs: 5, 164 and 7; or
. SEQ ID NOs: 5, 166 and 7; or
. SEQ ID NOs: 9, 169 and 11; or
. SEQ ID NOs: 9, 171 and 11; or
. SEQ ID NOs: 175, 176 and 177; or
. SEQ ID NOs: 179, 176 and 180; or
. SEQ ID NOs: 182, 176 and 177; or
. SEQ ID NOs: 184, 176 and 177; or
. SEQ ID NOs: 186, 187 and 188; or
. SEQ ID NOs: 190, 191 and 192; or
. SEQ ID NOs: 194, 195 and 196; or
. SEQ ID NOs: 198, 199 and 200; or
. SEQ ID NOs: 201, 202 and 203; or
. SEQ ID NOs: 205, 206 and 207, or
. SEQ ID NOs: 410, 6 and 7; or
. SEQ ID NOs: 413, 6 and 7; or
. SEQ ID NOs: 5, 416 and 7; or
. SEQ ID NOs: 5, 419 and 7; or
. SEQ ID NOs: 426, 6 and 7; or
. SEQ ID NOs: 5, 431 and 7; or
. SEQ ID NOs: 434, 6 and 7; or
. SEQ ID NOs: 437, 6 and 7; or
. SEQ ID NOs: 5, 6 and 452; or
. SEQ ID NOs: 5, 6 and 455; or
. SEQ ID NOs: 607, 608 and 609; or
. SEQ ID NOs: 610, 611 and 612, or
. SEQ ID NOs: 671, 2 and 3; or
. SEQ ID NOs: 672, 2 and 3; or
. SEQ ID NOs: 673, 6 and 7; or
. SEQ ID NOs: 674, 6 and 7; or
. SEQ ID NOs: 1, 2 and 713; or
. SEQ ID NOs: 5, 6 and 714; or
. SEQ ID NOs: 674, 164 and 7; or
. SEQ ID NOs: 710, 6 and 7; or
. SEQ ID NOs: 5, 6 and 715; or
. SEQ ID NOs: 674, 712 and 7; or
. SEQ ID NOs: 711, 6 and 7; or
variants thereof as defined above, preferably variants having at most 3, 2 or 1 amino acid modifications.

In another preferred embodiment, the VHH molecules of the invention comprise a CDR1, a CDR2 and a CDR3, wherein said CDR1, CDR2 and CDR3 domains are the domains of C5 or of variants thereof (as listed in Table 1), comprising or consisting of, respectively:
. SEQ ID NOs: 1, 2 and 3; or
. SEQ ID NOs: 13, 14 and 15; or
. SEQ ID NOs: 17, 2 and 3; or
. SEQ ID NOs: 19, 2 and 3; or
. SEQ ID NOs: 67, 2 and 3; or
. SEQ ID NOs: 69, 2 and 3; or
. SEQ ID NOs: 1, 21 and 3; or
. SEQ ID NOs: 1, 23 and 3; or
. SEQ ID NOs: 1, 71 and 3; or
. SEQ ID NOs: 1, 73 and 3; or
. SEQ ID NOs: 1, 75 and 3; or
. SEQ ID NOs: 1, 2 and 25; or
. SEQ ID NOs: 1, 2 and 27; or
. SEQ ID NOs: 1, 2 and 29; or
. SEQ ID NOs: 1, 2 and 31; or
. SEQ ID NOs: 1, 2 and 33; or
. SEQ ID NOs: 1, 2 and 77; or
. SEQ ID NOs: 1, 2 and 79; or
. SEQ ID NOs: 1, 2 and 81; or
. SEQ ID NOs: 1, 2 and 83; or
. SEQ ID NOs: 1, 2 and 85; or
. SEQ ID NOs: 392, 2 and 3; or
. SEQ ID NOs: 1, 113 and 3; or
. SEQ ID NOs: 1, 115 and 3; or
. SEQ ID NOs: 1, 2 and 117; or
. SEQ ID NOs: 1, 2 and 119; or
. SEQ ID NOs: 1, 2 and 121; or
. SEQ ID NOs: 1, 2 and 123; or
. SEQ ID NOs: 125, 2 and 3; or
. SEQ ID NOs: 17, 73 and 3; or
. SEQ ID NOs: 17, 128 and 3; or
. SEQ ID NOs: 671, 2 and 3; or
. SEQ ID NOs: 672, 2 and 3; or
. SEQ ID NOs: 1, 2 and 713; or
variants thereof as defined above, preferably variants having at most 3, 2 or 1 amino acid modifications.

In another preferred embodiment, the VHH molecules of the invention comprise a CDR1, a CDR2 and a CDR3, wherein said CDR1, CDR2 and CDR3 domains are the domains of B6 (as listed in Table 1), comprising or consisting of, respectively:
- SEQ ID NOs: 9, 10 and 11; or
- SEQ ID NOs: 9, 169 and 11; or
- SEQ ID NOs: 9, 171 and 11, or
variants thereof as defined above, preferably variants having at most 3, 2 or 1 amino acid modifications.

In a particular embodiment, the VHH molecules of the invention are B6 or variants thereof, that target the apical domain of the hTfR and comprise a CDR1, a CDR2 and a CDR3, comprising or consisting of, respectively:
- SEQ ID NOs: 9, 10 and 11; or
- SEQ ID NOs: 9, 169 and 11; or
- SEQ ID NOs: 9, 171 and 11; or
- SEQ ID NOs: 175, 176 and 177; or
- SEQ ID NOs: 179, 176 and 180; or
- SEQ ID NOs: 182, 176 and 177; or
- SEQ ID NOs: 184, 176 and 177; or
variants thereof as defined above, preferably variants having at most 3, 2 or 1 amino acid modifications.

In another particular embodiment, the VHH molecules of the invention are VHH molecules targeting the apical domain of the hTfR and comprising a CDR1, a CDR2 and a CDR3 (as listed in Table 1), comprising or consisting of, respectively:
. SEQ ID NOs: 9, 10 and 11; or
. SEQ ID NOs: 9, 169 and 11; or
. SEQ ID NOs: 9, 171 and 11; or
. SEQ ID NOs: 175, 176 and 177; or
. SEQ ID NOs: 179, 176 and 180; or
. SEQ ID NOs: 182, 176 and 177; or
. SEQ ID NOs: 184, 176 and 177; or
. SEQ ID NOs: 186, 187 and 188; or
. SEQ ID NOs: 190, 191 and 192; or
. SEQ ID NOs: 194, 195 and 196; or
. SEQ ID NOs: 198, 199 and 200; or
. SEQ ID NOs: 201, 202 and 203; or
. SEQ ID NOs: 205, 206 and 207, or
variants thereof as defined above, preferably variants having at most 3, 2 or 1 amino acid modifications.

In another preferred embodiment, the VHH molecules of the invention comprise a CDR1, a CDR2 and a CDR3, wherein said CDR1, CDR2 and CDR3 domains are the domains of B8 or of variants thereof (as listed in Table 1), comprising or consisting of, respectively:
. SEQ ID NOs: 5, 6 and 7; or
. SEQ ID NOs: 5, 160 and 7; or
. SEQ ID NOs: 5, 162 and 7; or
. SEQ ID NOs: 5, 164 and 7; or
. SEQ ID NOs: 5, 166 and 7; or
. SEQ ID NOs: 410, 6 and 7; or
. SEQ ID NOs: 413, 6 and 7; or
. SEQ ID NOs: 5, 416 and 7; or
. SEQ ID NOs: 5, 419 and 7; or
. SEQ ID NOs: 426, 6 and 7; or
. SEQ ID NOs: 5, 431 and 7; or
. SEQ ID NOs: 434, 6 and 7; or
. SEQ ID NOs: 437, 6 and 7; or
. SEQ ID NOs: 5, 6 and 452; or
. SEQ ID NOs: 5, 6 and 455, or
. SEQ ID NOs: 673, 6 and 7; or
. SEQ ID NOs: 674, 6 and 7; or
. SEQ ID NOs: 5, 6 and 714; or
. SEQ ID NOs: 674, 164 and 7; or
. SEQ ID NOs: 710, 6 and 7; or
. SEQ ID NOs: 5, 6 and 715; or
. SEQ ID NOs: 674, 712 and 7; or
. SEQ ID NOs: 711, 6 and 7; or
variants thereof as defined above, preferably variants having at most 3, 2 or 1 amino acid modifications.

In another particular embodiment, the VHH molecules of the invention are cross-reactive with human, mouse and/or non-human primate species, as detailed in Tables 5 and 6 below. Preferred VHH molecules of the invention comprise FRs domains as defined below.

In a particular embodiment, the FR1 domain comprises or consists of SEQ ID NO: 35 as represented below, or variants thereof having at least 58%, for example at least 60%, 62%, 64%, 66%, 68%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%, amino acid identity to this sequence over the entire length thereof, preferably at least 80%: EVQLVESGGGLVQPGGSLKLSCAAS (SEQ ID NO: 35). More preferably, the bold amino acid residues are present and the variability occurs only on the other positions.

In a specific embodiment, the E in position 1 may be replaced with Q.

In a specific embodiment, the V in position 5 may be replaced with Q.

In a specific embodiment, the E in position 6 may be replaced with Q.

In a specific embodiment, the G in position 10 may be replaced with K or A.

In a specific embodiment, the L in position 11 may be replaced with V or E.

In a specific embodiment, the P in position 14 may be replaced with A.

In a specific embodiment, the G in position 16 may be replaced with D.

In another specific embodiment, the K in position 19 may be replaced with R.

In another specific embodiment, the A in position 23 may be replaced with V or T.

In another specific embodiment, the S in position 25 may be replaced with D.

More preferably, the FR1 contains at most 4 amino acid modifications by reference to this sequence, even more preferably at most 3, even more preferably at most 2 amino acid modifications in non-bold amino acid residues. In a preferred embodiment, the amino acid modification is R in position 19.

In a further specific embodiment, the FR1 has an amino acid sequence selected from any one of the amino acid sequences listed below:
EVQLVESGGGVVQPGGSLKLSCVAS (SEQ ID NO: 36);
EVQLVESGGGVVQPGGSLRLSCAAS (SEQ ID NO: 37);
EVQLVESGGGLVQPGGSLRLSCTAS (SEQ ID NO: 38); or
EVQLVESGGGEVQPGGSLKLSCVAS (SEQ ID NO: 39); or variants thereof.

Further specific examples of the FR1 of VHH molecules according to the invention are provided below (see also Table 3):
EVQLVESGGGVVQPGGSLKLSCAAS (SEQ ID NO: 331),
EVQLVESGGGLVQPGGSLRLSCAAS (SEQ ID NO: 332),
EVQLVESGGGVVQPGGSLRLSCAAD (SEQ ID NO: 333),
EVQLVESGGGVVQPGGSLRLSCVAS (SEQ ID NO: 400),
QVQLVQSGGGLVQAGGSLTLSCTAS (SEQ ID NO: 334),
EVQLVESGGGLVQAGGSLRLSCTAS (SEQ ID NO: 335),
QVQLVQSGGGLVQPGGSLRLSCAAS (SEQ ID NO: 336),
EVQLVESGGGLVQAGDSLRLSCTAS (SEQ ID NO: 337),
QVQLVQSGGGLVQAGGSLRLSCAAS (SEQ ID NO: 338),
EVQLVQSGGGLVQAGGSLRLSCAAS (SEQ ID NO: 339),
EVQLVESGGGLVQPGESLRLSCTAS (SEQ ID NO: 340),
EVQLVESGGGLVQPGGSLRLSCVSS (SEQ ID NO: 341),
EVQLVESGGGLVQAGDSLRLSCAAS (SEQ ID NO: 619), or
VQLVESGGRLVQAGGSLRLSCTAS (SEQ ID NO: 620).
In a particular embodiment, VHH molecules of the invention comprise a FR2 domain comprising or consisting of SEQ ID NO: 40 as represented below, or variants thereof having at least 58%, for example at least 60%, 62%, 64%, 66%, 68%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%, amino acid identity to this sequence over the entire length thereof: MR**W**YRQA**P**G**K**QRELVAT (SEQ ID NO: 40). More preferably, the bold amino acid residues are present and the variability occurs only on the other positions.

In a specific embodiment, the M in position 1 may be replaced with I or V.

In a specific embodiment, the R in position 2 may be replaced with G or H or S.

In a specific embodiment, the Y in position 4 may be replaced with F or V.

In a specific embodiment, the R in position 5 may be replaced with G.

In a specific embodiment, the Q in position 6 may be replaced with R or E.

In a specific embodiment, the A in position 7 may be replaced with R.

In a specific embodiment, the G in position 9 may be replaced with I or E.

In a specific embodiment, the Q in position 11 may be replaced with E or G or I or D.

In a specific embodiment, the R in position 12 may be replaced with L.

In a specific embodiment, the E in position 13 may be replaced with N or H.

In a specific embodiment, the L in position 14 may be replaced with F or W or S or Q.

In a specific embodiment, the V in position 15 may be replaced with Q or I.

In a specific embodiment, the A in position 16 may be replaced with M.

In a specific embodiment, the T in position 17 may be replaced with G or S.

More preferably, the FR2 contains at most 6 amino acid modifications by reference to this sequence, even more preferably at most 5, at most 3, even more preferably at most 2 amino acid modifications in non-bold amino acid residues. In preferred embodiments, the amino acid modifications are V in position 4 and/or G in position 11 and L in position 12 and/or W in position 14.

In a particular embodiment, VHH molecules of the invention comprise at least one of the following amino acids in the FR2 domain: Phe42, Glu49, Arg50 or Gly52 (according to IMGT numbering).

In a further specific embodiment, the FR2 has an amino acid sequence selected from any one of the amino acid sequences listed below:
IRWYRQAPGKQREFVAG (SEQ ID NO: 41);
MRWYRQAPGKQREWVAG (SEQ ID NO: 42);
MGWFRRAPGKERELVAS (SEQ ID NO: 43);
VRWYRQRPGKQREWVAG (SEQ ID NO: 44); or variants thereof.

Further specific examples of the FR2 of VHH molecules according to the invention are provided below (see also Table 3):
IRWVRQAPGKGLEWVAG (SEQ ID NO: 342),
IRWYRQAPGKGLEFVAG (SEQ ID NO: 343),
IRWVRQAPGKGLEFVAG (SEQ ID NO: 344),
IRWYRQAPGKGREFVAG (SEQ ID NO: 345),
IRWVRQAPGKQREFVAG (SEQ ID NO: 346),
IRWYRQAPGKGLEWVAG (SEQ ID NO: 347),
MRWYRQAPGKGLEWVAG (SEQ ID NO: 348),
MRWYGQAPGKQREWVAG (SEQ ID NO: 349),
MRWYREAPGKQREWVAG (SEQ ID NO: 350),
MRWYRQAPIKQREWVAG (SEQ ID NO: 351),
MRWYRQAPGKIREWVAG (SEQ ID NO: 352),
MGWFRRAPGKERNLVAS (SEQ ID NO: 353),
MGWFRRAPGKERESVAS (SEQ ID NO: 354),
MGWFRRAPGKERELQAS (SEQ ID NO: 355),
MGWFRRAPEKERELVAS (SEQ ID NO: 356),
MGWFRRAPGKDRELVAS (SEQ ID NO: 357),
MSWVRQAPGKGRELVAS (SEQ ID NO: 358),
MGWFRRAPGKERELIAS (SEQ ID NO: 359),
LAWHRQIPGKEREWVAG (SEQ ID NO: 360),
MAWHRQAPGKERLWVAG (SEQ ID NO: 361),
VGWYRQAPGEQRVLVAH (SEQ ID NO: 362),
MGWFRQAPGKEREFVAA (SEQ ID NO: 363),
MGWYRQAPGKQRELVAV (SEQ ID NO: 364),
MGWFRQTPGKEREFVAA (SEQ ID NO: 365),
MRWYRQAPGKQREQVAG (SEQ ID NO: 458),
MRWYRQAPGKQREFVAG (SEQ ID NO: 459),
MRWYRQAPGKQRHWVAG (SEQ ID NO: 460),
MIWYRQAPGKQREWVAG (SEQ ID NO: 461),
MEWYRQAPGKQREWVAG (SEQ ID NO: 462),
MRWYRQAPGKQREWVAA (SEQ ID NO: 463),
MRWYRQAPGKQREWVAK (SEQ ID NO: 464),
IGWFRQAPGKEREKVSC (SEQ ID NO: 621), or
MHWFRQAPGKEREFVGA (SEQ ID NO: 622), or
IRWYSQAPGKQREFVAG (SEQ ID NO: 716), or
MRWYRQAPGKQREWVSG (SEQ ID NO: 720).

In a particular embodiment, VHH molecules of the invention comprise a FR3 domain comprising or consisting of SEQ ID NO: 45 as represented below, or variants thereof having at least 58%, for example at least 60%, 62%, 64%, 66%, 68%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%, amino acid identity to this sequence over the entire length thereof, preferably at least 70%: YYADSVKG**RFTISRDN**AKNTVYLQMNSLKPE**DTAVYYC** (SEQ ID NO: 45). More preferably, the bold amino acid residues are present and the variability occurs only on the other positions.

In a specific embodiment, the Y in position 1 may be replaced with N.

In a specific embodiment, the Y in position 2 may be replaced with A.

In a specific embodiment, the A in position 3 may be replaced with P or I.

In a specific embodiment, the D in position 4 may be replaced with S or N.

More preferably, the FR3 contains at most 7 amino acid modifications by reference to this sequence, even more preferably at most 6, at most 3, even more preferably at most 2 amino acid modifications in non-bold amino acid residues.

In a further specific embodiment, the FR3 has an amino acid sequence selected from any one of the amino acid sequences listed below:
NYADSMKGRFTISRDNTKNAVYLQIDSLKPEDTAVYYC (SEQ ID NO: 46);
NYPDSAKGRFTISRDNAKNTVYLQIDSLKPEDTAVYYC (SEQ ID NO: 47);
YAISSVKGRFTISRDNAENTVFLQMNSLKPDDTAVYYC (SEQ ID NO: 48); or
NYPDSMKGRFTISRDNAKNTVYLQINSLKSEDTAVYYC (SEQ ID NO: 49); or variants thereof.

Further specific examples of the FR3 of VHH molecules according to the invention are provided below (see also Table 3):
NYADSMKGRFTISRDNTKNALYLQIDSLRPEDTAVYYC (SEQ ID NO: 366)
NYADSVKGRFTISRDNTKNTLYLQIDSLRPEDTAVYYC (SEQ ID NO: 367),
NYADSVKGRFTISRDNAKNTLYLQMNSLRPEDTAVYYC (SEQ ID NO: 368),
NYADSVKGRFTISRDNTKNTLYLQINSLRPEDTAVYYC (SEQ ID NO: 369),
NYADSMKGRFTISRDNTKNTLYLQMNSLRPEDTAVYYC (SEQ ID NO: 370),
NYADSVKGRFTISRDNAKNTLYLQIDSLRPEDTAVYYC (SEQ ID NO: 371),
NYADSVKGRFTISRDNTKNTLYLQMNSLRPEDTAVYYC (SEQ ID NO: 372),
NYADSVKGRFTISRDNTKNALYLQMNSLRPEDTAVYYC (SEQ ID NO: 373),
NYADSVKGRFTISRDNTKNTLYLQMDSLRPEDTAVYYC (SEQ ID NO: 374),
NYADSVKGRFTISRDNSKNTLYLQMNSLRAEDTAVYYC (SEQ ID NO: 375),
NYADSVKGRFTISRDNAKNAVYLQMNSLRPEDTAVYYC (SEQ ID NO: 376),
NYADSVKGRFTISRDNAKNTLYLQMNSLKPEDTAVYYC (SEQ ID NO: 377),
NYADSMKGRFTISRDNAKNTLYLQMNSLRPEDTAVYYC (SEQ ID NO: 378),
NYPDSVKGRFTISRDNAKNTVYLQMNSLRPEDTAVYYC (SEQ ID NO: 379),
YYADGMRGRFTISRDNSENTVSLQMNNLKPEDTAVYYC (SEQ ID NO: 380),
YYANSMKERFTISRDNAQNTVSLQISSLKPEDTAVYYC (SEQ ID NO: 381),
YYADGMKGRFTISRDNAENTVSLQINSLKPEDTAIYYC (SEQ ID NO: 382),
YYADSSVKGRFTISRDNAENTVSLQMNSLKPEDTAVYYC (SEQ ID NO: 383),
SYRDSVKGRFTISRDNAKNTVFLQMNSLEPEDTGVYYC (SEQ ID NO: 384),
SYADSVKGRFTISRDDAKNTVYLQMDNLTPEDTAVYFC (SEQ ID NO: 385),
EYKDSVKGRFTISRDNARNTIYLEMKNLKPEDTAIYYC (SEQ ID NO: 386),
DYADGVMGRFTISRNSALNTVYLQMDSLKSTDTGVYVC (SEQ ID NO: 387),
KYGDSVKGRFTISRDDAKNTVYLQMNSLKPEDTAVYYC (SEQ ID NO: 388),
TYADSVKGRFTISRDNAKNTVYLQMNSLEPTDTAVYYC (SEQ ID NO: 389),
YYADSVKGRFTISRDTVKDMVYLQMNSLKPEDTAVYYC (SEQ ID NO: 623),
EYADSVKGRFTISRDNAKSTVYLQMNNLKPEDTAVYYC (SEQ ID NO: 624),
VYPDSAKGRFTISRDNAKNTVYLQIDSLKPEDTAVYYC (SEQ ID NO: 625), or
FYPDSAKGRFTISRDNAKNTVYLQIDSLKPEDTAVYYC (SEQ ID NO: 626), or
NYADSMKGRLTISRDNTKNAVYLQIDSLKPEDTAVYYC (SEQ ID NO: 717), or
NYPDIAKGRFTISRDNAKNTVYLQIDSLKPEDTAVYYC (SEQ ID NO: 718), or
NYPDSAKGRFTISEDNAKNTVYLQIDSLKPEDTAVYYC (SEQ ID NO: 719).

In a particular embodiment, VHH molecules of the invention comprise a FR4 domain comprising or consisting of SEQ ID NO: 50 as represented below, or variants thereof having at least 58%, for example at least 60%, 62%, 64%, 66%, 68%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%, amino acid identity to this sequence over the entire length thereof, preferably at least 90%: W**GQGTQVT**VSS (SEQ ID NO: 50). More preferably, the bold amino acid residues are present and the variability occurs only on the other positions.

More preferably, the FR4 contains at most 4 amino acid modifications by reference to this sequence, even more preferably at most 3, even more preferably at most 2 amino acid modifications in non-bold amino acid residues. A specific illustrative example of a FR4 sequence is SEQ ID NO: 50.

Other specific examples of the FR4 according to the invention are the following:
WGQGTLVTVSS (SEQ ID NO: 390),
WGKGTQVTVSS (SEQ ID NO: 391) or
WGRGTQVTVSS (SEQ ID NO: 670).

Specific examples of TfR-binding VHH molecules of the invention are molecules comprising or consisting of an amino acid sequence selected from any one of SEQ ID NOs: 213-271, 273-299, 412, 415, 418, 421, 423, 425, 428, 430, 433, 436, 439, 441, 443, 445, 447, 449, 451, 454, 457, 613-615, 675-678 and 701-709 (see Table 1). In the examples corresponding to SEQ ID NOs: 213-271, 273-299, 412, 415, 418, 421, 423, 425, 428, 430, 433, 436, 439, 441, 443, 445, 447, 449, 451, 454, 457, 613-615, 675-678 and 701-709, the VHH molecule does not comprise any tag sequence (when x is 0, as detailed in Table 1 below).

Other examples of TfR-binding VHH molecules of the invention are molecules comprising or consisting of an amino acid sequence selected from any one of SEQ ID NOs: 4, 8, 12, 16, 18, 20, 22, 24, 26, 28, 30, 32, 34, 68, 70, 72, 74, 76, 78, 80, 82, 84, 86-92, 114, 116, 118, 120, 122, 124, 126, 127, 129-149, 152-159, 161, 163, 165, 167, 168, 170, 172-174, 178, 181, 183, 185, 189, 193, 197, 204, 208, 393, 411, 414, 417, 420, 422, 424, 427, 429, 432, 435, 438, 440, 442, 444, 446, 448, 450, 453, 456, 616-618, 679-682, and 691-700 (as listed in Table 1 below; in these examples, when x is equal to 1, each VHH molecule comprises the following particular tag sequence of SEQ ID NO: 51: AAAEQKLISEEDLNGAAHHHHHHGS).

In a particular embodiment, the VHH of the invention is humanized. For humanization, one or more of the FR and/or CDR domains may be (further) modified by one or more amino acid substitutions.

In this respect, in a particular embodiment, the VHH are humanized by selected modification (e.g., amino acid substitution) of the FR1 domain. A FR1 domain consists typically in a sequence of 25 amino acid residues. A typical humanized position in FR1 is 19R or 23A, or both (for example by reference to e.g., any one of SEQ ID NOs: 35-39, 331, 332, 400, or to any variants thereof as herein defined). Specific examples of such humanized FR1 thus comprise SEQ ID NOs: 37, 331, 400, wherein K19 and/or V23 are respectively modified into 19R and 23A.

Another humanized position in FR1 is 11L, and a specific example of such humanized FR1 thus comprises SEQ ID NO: 332, wherein V11 is modified into 11L and wherein K19 and V23 are respectively modified into 19R and 23A.

In another particular embodiment, the VHH are humanized by selected modification of the FR2 domain. A typical humanized position in FR2 is selected from 1M, 2S or 2H, 4V, 11G, 12L, 14W, or combinations thereof (by reference to e.g., any one of SEQ ID NOs: 40-44, 342-348 or to any variants thereof as herein defined).

A specific example of such a humanized FR2 thus comprises SEQ ID NO: 41 wherein one or more or all of I1, R2, Y4, Q11, R12, and F14 are respectively modified into 1M, 2S or 2H, 4V, 11G, 12L, and 14W.

Another specific example of the humanized FR2 comprises SEQ ID NO: 342, wherein Y4, Q11, R12 and F14 are respectively modified into 4V, 11G, 12L and 14W.

Another specific example of the humanized FR2 comprises SEQ ID NO: 343, wherein Q11 and R12 are respectively modified into 11G and 12L.

Another specific example of the humanized FR2 comprises SEQ ID NO: 344, wherein Y4, Q11 and R12 are respectively modified into 4V, 11G and 12L.

Another specific example of the humanized FR2 comprises SEQ ID NO: 345, wherein Q11 is modified into 11G.

Another specific example of the humanized FR2 comprises SEQ ID NO: 346, wherein Y4 is modified into 4V.

Another specific example of the humanized FR2 comprises SEQ ID NO: 347, wherein Q11, R12 and F14 are respectively modified into 11G, 12L and 14W.

Another specific example of the humanized FR2 comprises SEQ ID NO: 348, wherein Q11 and R12 are respectively modified into 11G and 12L.

In another particular embodiment, the VHH are humanized by selected modification of the FR3 domain. A typical humanized position in FR3 is selected from 6V, 17A or S, 20T, 21L, 25M, 26N, 29R, 30A, and any combination thereof (by reference to e.g., any one of SEQ ID Nos: 45-49, 366-379 or to any variants thereof as herein defined). A specific example of such a humanized FR3 thus comprises SEQ ID NO: 46 wherein one or more or all of M6, T17, A20, V21, I25, D26, and K29, are respectively modified into 6V, 17A, 20T, 21L, 25M, 26N, and 29R.

Another specific example of the humanized FR3 comprises SEQ ID NO: 366 wherein V21 and K29 are respectively modified into 21L and 29R.

Another specific example of such a humanized FR3 comprises SEQ ID NO: 367 wherein M6, A20, V21 and K29, are respectively modified into 6V, 20T, 21L and 29R.

Another specific example of the humanized FR3 comprises SEQ ID NO: 368 wherein M6, T17, A20, V21, I25, D26, and K29, are respectively modified into 6V, 17A, 20T, 21L, 25M, 26N, and 29R.

Another specific example of the humanized FR3 comprises SEQ ID NO: 369 wherein M6, A20, V21, D26, and K29, are respectively modified into 6V, 20T, 21L, 26N, and 29R.

Another specific example of the humanized FR3 comprises SEQ ID NO: 370 wherein A20, V21, 125, D26, and K29, are respectively modified into 20T, 21L, 25M, 26N, and 29R.

Another specific example of the humanized FR3 comprises SEQ ID NO: 371 wherein M6, T17, A20, V21 and K29, are respectively modified into 6V, 17A, 20T, 21L and 29R.

Another specific example of the humanized FR3 comprises SEQ ID NO: 372 wherein M6, A20, V21, 125, D26, and K29, are respectively modified into 6V, 20T, 21L, 25M, 26N, and 29R.

Another specific example of the humanized FR3 comprises SEQ ID NO: 373 wherein M6, V21, 125, D26, and K29, are respectively modified into 6V, 21L, 25M, 26N, and 29R.

Another specific example of the humanized FR3 comprises SEQ ID NO: 374 wherein M6, A20, V21, 125 and K29, are respectively modified into 6V, 20T, 21L, 25M and 29R.

Another specific example of the humanized FR3 comprises SEQ ID NO: 375 wherein M6, T17, A20, V21, I25, D26, K29 and P30, are respectively modified into 6V, 17S, 20T, 21L, 25M, 26N, 29R and 30A.

Another specific example of the humanized FR3 comprises SEQ ID NO: 376 wherein M6, T17, I25, D26 and K29, are respectively modified into 6V, 17A, 25M, 26N and 29R.

Another specific example of the humanized FR3 comprises SEQ ID NO: 377 wherein M6, T17, A20, V21, I25 and D26, are respectively modified into 6V, 17A, 20T, 21L, 25M and 26N.

Another specific example of the humanized FR3 comprises SEQ ID NO: 378 wherein T17, A20, V21, 125, D26, and K29, are respectively modified into 17A, 20T, 21L, 25M, 26N, and 29R.

Another specific example of the humanized FR3 comprises SEQ ID NO: 379 wherein M6, 125, D26, and K29, are respectively modified into 6V, 25M, 26N, and 29R.

Specific examples of humanized TfR-binding VHH molecules of the invention are molecules comprising or consisting of an amino acid sequence selected from any one of SEQ ID NOs: 236-241 or 252-271, 273-275 (see Table 1).

In a further particular embodiment, the VHH molecules may further comprise one or several tags, suitable for e.g., purification, coupling, detection, etc. Within the context of this invention, the term "tag" includes any peptide sequence which is attached to a polypeptide VHH molecule of the invention to facilitate easy detection or purification of expressed proteins or to identify their binding to TfR or for site-directed enzymatic chemical/enzymatic conjugation purpose. The tag may be an affinity tag, an epitope tag, a site-specific conjugation tag or a fluorescent tag.

Examples of such tags include a Q-tag which is a tag comprising a glutamine residue inserted in a tag sequence, which is specifically recognized by the TGase, preferably comprising or consisting of the sequence LQR, a myc tag (EQKLISEEDL, SEQ ID NO: 394), a poly-His tag (comprising from 2 to 8 histidine residues, preferably 6-8 His residues, e.g, His6 (SEQ ID NO: 395) or His8 (SEQ ID NO: 396)), a poly-Arg tag (comprising from 2 to 8 arginine residues), a poly-Lys tag (comprising from 2 to 8 lysine residues), an HA tag (e.g., YPYDVPDYA, SEQ ID NO: 397), a FLAG tag (e.g., DYKDDDDK, SEQ ID NO: 398) or a GFP tag, a CBP tag, a Strep II tag, a sortase-tag, a SNAP-tag or a combination thereof (as shown in Table 4 below).

Typically, the one or several tags are located at the C-terminal end of the VHH.

In a further particular embodiment, the VHH molecules may further comprise one or several linkers.

Within the context of this invention, the terms "linker" or "spacer" are used interchangeably. The linkers may be peptide linkers or conjugation linkers. The peptide linker includes one or more amino acid residues, typically from 1 to 10 amino acid residues, used for linking the VHH molecule of the invention and a tag, or between various tags as described herein, provided that the linker does not specifically bind to the target protein which is TfR. The linker may be any amino acid residue for example, glycine (Gly or G), alanine (Ala or A), phenylalanine (Phe or F), serine (Ser or S), cysteine (Cys or C), leucine (Leu or L), asparagine (Asn or N), lysine (Lys or K), glutamate (Glu or E), glutamine (Gln or Q), proline (Pro or P), valine (Val or V), arginine (Arg or R), aspartate (Asp or D), etc., or a combination thereof. The peptide linker may be flexible (e.g., any flexible hydrophilic linker) or rigid (e.g., any alpha-helix rigid linker).

Such a peptide linker differs from conjugation linkers which may be introduced between the VHH and the compounds of interest, such as bi- or multifunctional agents containing alkyl, aryl, thiols, azide, alkynes, nucleotidic or peptide groups by esters, aldehydes or alkyl or aryl acids, anhydride, sulfhydryl or carboxyl groups, groups derived from cyanogen bromide or chloride, carbonyldiimidazole, succinimide esters or sulfonic halides (as described herein in the part "Conjugates").

As a further illustration, the VHH of the invention may comprise a linker, preferably located at the C-terminal end of the VHH. The linker may comprise a Gly residue or a Gly repeat of e.g., 2-7 Gly residues (i.e., Gly2, Gly3, Gly4, Gly5, Gly6 or Gly7, respectively). The VHH of the invention may also comprise a combination of Gly and Ser residues. Specific examples of such Gly and Ser combinatorial linkers include: GlySerGlySer (GSGS; SEQ ID NO: 627); SerGlySerGly5 (SGSGGGGG; SEQ ID NO: 628); (Gly4Ser)n, wherein n is from 1 to 6, for example any of SEQ ID NOs: 629 to 634 (as shown in Table 4 below), preferably SEQ ID NOs: 629 to 631; or any linker comprising such (Gly4Ser)n sequence, for example GlyGly(Gly4Ser)3 (GGGGGGSGGGGSGGGGS; SEQ ID NO: 635).

Other specific examples of peptide linkers according to the invention comprise or consist of EAAAK (SEQ ID NO: 636) or comprise a EAAAK repeat in combination with other amino acid residues, for example any of SEQ ID NOs: 637 to 641, as shown in Table 4.

Other specific examples of peptide linkers according to the invention are the following: GG(AP)17 (SEQ ID NO: 642), ASTKGPSVFPLAP (SEQ ID NO: 643), GSAGSAAGSGEF (SEQ ID NO: 644) or KESGSVSSEQLAQFRSLD (SEQ ID NO: 645), as shown in Table 4.

In a particular embodiment, VHH of the invention may comprise a Gly linker and a Q-tag, preferably located C-terminally. More specific examples of such VHH comprise the following structure: VHH - Gly linker - Q-tag, wherein the Gly linker comprises or consists of 2-6 Gly residues; and the Q-tag contains or consists of LQR. As an illustration, the VHH may comprise, at the C-terminal end, the following tag sequence AAAEQKLISEEDLNGAAHHHHHHGS (SEQ ID NO: 51), wherein simple underlined is a myc tag and double underlined is a His6 tag (the remaining residues being linkers such as an Ala linker AAA or residues resulting from cloning).

Specific examples of such tagged TfR-binding VHH molecules of the invention, comprising the tag sequence of SEQ ID NO: 51 at the C-terminal end, are molecules comprising or consisting of an amino acid sequence selected from any one of 4, 8, 12, 16, 18, 20, 22, 24, 26, 28, 30, 32, 34, 68, 70, 72, 74, 76, 78, 80, 82, 84, 86-92, 114, 116, 118, 120, 122, 124, 126, 127, 129-149, 152-159, 161, 163, 165, 167, 168, 170, 172-174, 178, 181, 183, 185, 189, 193, 197, 204, 208, 393, 411, 414, 417, 420, 422, 424, 427, 429, 432, 435, 438, 440, 442, 444, 446, 448, 450, 453, 456, 616-618, 679-682 and 691-700.

Specific examples of humanized TfR-binding VHH molecules of the invention with tag sequences are molecules comprising or consisting of an amino acid sequence selected from any one of SEQ ID NOs: 87-92 or 130-149, 152-154 (see Table 1).

As another illustration, the VHH may comprise, at the C-terminal end, the following tag sequence GGGGSCHHHHHH (SEQ ID NO: 399), wherein simple underline is a spacer, bold C is a free cysteine available for site-directed chemical conjugation and double underline is a His tag (the remaining residues being linkers or resulting from cloning).

As another illustration, the VHH of the invention may comprise a Q-tag preferably comprising or consisting of the sequence LQR, preferably located at the C-terminal end of the VHH.

As a further illustration, the VHH may comprise, at the C-terminal ("C-ter" or "C-terminus") end, the following tag sequence **GGG**LQR (SEQ ID NO: 111) wherein underlined is a Q-tag and bold is a Gly linker. Other examples are GGGGLQR (SEQ ID NO: 401), GGGGGLQR (SEQ ID NO: 402), GGGGGGLQR (SEQ ID NO: 403) and GGGGGGGLQR (SEQ ID NO: 404). In a preferred aspect of the invention, the VHH comprises the tag sequence of SEQ ID NO: 111.

In a further particular embodiment, VHH of the invention may comprise an Ala linker, a His tag, a Gly linker and a Q-tag. Preferably, the linkers and tags are located C-terminally of the VHH. In other embodiments, the Q-tag at least may be located N-terminal end of the VHH. More specific examples of such VHH comprise the following structure: VHH - Ala linker - His tag - Gly linker - Q-tag, wherein the Ala linker comprises 3 residues; the His tag comprises 2-7 His residues, preferably 6 His residues; the Gly linker comprises 2-6 Gly residues, preferably 3 residues; and the Q-tag preferably contains or consists of LQR.

As an illustration, the VHH may comprise, at the C-terminal end, the following tag sequence **AAA**HHHHHH**GGG**LQR (SEQ ID NO: 112) wherein underlined is a Q-tag, bold are an Ala and a Gly linker, double underline is a His tag. Other examples are AAAHHHHHHGGGGLQR (SEQ ID NO: 406), AAAHHHHHHGGGGGLQR (SEQ ID NO: 407), AAAHIIHEMGGGGGGLQR (SEQ ID NO: 408) and AAAHHHHHHGGGGGGGLQR (SEQ ID NO: 409). In a preferred aspect of the invention, the VHH comprises the additional sequence of SEQ ID NO: 112.

As already described herein above, specific examples of such tagged TfR-binding VHH molecules of the invention are molecules comprising or consisting of an amino acid sequence selected from any one of 4, 8, 12, 16, 18, 20, 22, 24, 26, 28, 30, 32, 34, 68, 70, 72, 74, 76, 78, 80, 82, 84, 86-92, 114, 116, 118, 120, 122, 124, 126, 127, 129-149, 152-159, 161, 163, 165, 167, 168, 170, 172-174, 178, 181, 183, 185, 189, 193, 197, 204, 208, 393, 411, 414, 417, 420, 422, 424, 427, 429, 432, 435, 438, 440, 442, 444, 446, 448, 450, 453, 456, 616-618, 679-682, and 691-700.

Further specific examples of TfR-binding VHH molecules of the invention are VHH molecules which competitively inhibit binding of a VHH as defined above to a human and a non-human TfR. The term "competitively inhibits" indicates that the VHH can reduce or inhibit or displace the binding of a said reference VHH to TfR, *in vitro* or *in vivo.* Competition assays can be performed using standard techniques such as, for instance, competitive ELISA or other binding assays. Typically, a competitive binding assay involves a recombinant muscle cell or membrane preparation expressing a TfR, optionally bound to a solid substrate, an unlabeled test VHH (or a phage expressing the same) and a labeled reference VHH (or a phage expressing the same). Competitive inhibition is measured by determining the amount of labeled VHH bound in the presence of the test VHH. Usually, the test VHH is present in excess, such as about 5 to 500 times the amount of reference VHH. Typically, for ELISA, the test VHH is in 100-fold excess. When a test VHH present in excess inhibits or displaces at least 70% of the binding of the reference VHH to TfR, it is considered as competitively inhibiting said reference VHH. Preferred competing VHH bind epitopes that share common amino acid residues.

As shown in the experimental section, VHH molecules bind TfR *in vitro* and *in vivo.* They show adequate affinity, with a Kd (measured for example by SPR or by flow cytometry), from 0.001 nM to 10 µM, preferably from 0.01 nM to 4 µM or from 0.01 nM to 500 nM or from 0.01 nM to 100 nM or from 0.1 nM to 4 µM or from 0.1 nM to 500 nM or from 0.1 nM to 100 nM. Preferably, the VHH molecules of the invention bind human and non-human primate TfR with a Kd below 500 nM, and even more preferably, the VHH molecules of the invention bind human TfR with a Kd from 0.01 nM to 100 nM, rhesus monkey TfR with a Kd from 0.1 nM to 500 nM and mouse TfR with a Kd from 0.1 nM to 4000 nM.

In a particular embodiment of the invention, VHH molecules are C5 or variants thereof, humanized or not, comprising or consisting of any of SEQ ID NOs: 213, 216-271, 274, 275, 675, 676 and 701 (as listed in Table 1).

In another particular embodiment, VHH molecules are C5 or variants thereof which are cross-species VHH molecules binding to human, non-human primate (NHP) and murine TfR. In another particular embodiment, C5 or variants thereof comprise a tag sequence at the N- and/or C-terminal end. Specific examples of such variants comprise or consist of any of SEQ ID NOs: 4, 16, 18, 20, 22, 24, 26, 28, 30, 32, 34, 68, 70, 72, 74, 76, 78, 80, 82, 84, 86-92, 114, 116, 118, 120, 122, 124, 126, 127, 129 or 130-149, 153-154, 393, 679, 680, 692.

In another particular embodiment, VHH molecules are humanized variants of C5 comprising or consisting of any of SEQ ID NOs: 236-241, 252-271, 274-275. In a particular embodiment, the humanized variants of C5 comprise a tag sequence at the N- and/or C-terminal end. Specific examples of such variants comprise or consist of any of SEQ ID NOs: 87-92 or 130-149, 153-154. Examples of humanized variants of C5 are C5h20 variants comprising or consisting of SEQ ID NOs: 265 or 143, or C5V13h20 variants comprising or consisting of SEQ ID NO: 274 or 153.

In another particular embodiment, VHH molecules of the invention are B8 or variants thereof (as listed in Table 1), comprising or consisting of any of SEQ ID NOs: 214, 276-284, 412, 415, 418, 421, 423, 425, 428, 430, 433, 436, 439, 441, 443, 445, 447, 449, 451, 454, 457, 677, 678 and 702-709. In a particular embodiment, B8 or variants thereof comprise a tag sequence at the N- and/or C-terminal end. Specific examples of such variants comprise or consist of any of SEQ ID NOs: 8, 152, 155-159, 161, 163, 165, 167, 411, 414, 417, 420, 422, 424, 427, 429, 432, 435, 438, 440, 442, 444, 446, 448, 450, 453, 456, 681, 682, or 693-700.

In another particular embodiment, VHH molecules of the invention are B8 or variants thereof, which are cross-reactive with human and primate species.

In another particular embodiment, VHH molecule are B8 or variants thereof, humanized or not, comprising an amino acid sequence selected from any one of SEQ ID NOs: 214, 273, 276-284, 412, 415, 418, 421, 423, 425, 428, 430, 433, 436, 439, 441, 443, 445, 447, 449, 451, 454, 457, 677, 678 and 702-709.

In another particular embodiment, VHH molecules of the invention are B8 or variants thereof, comprising or consisting of any of SEQ ID NOs: 677, 678, 681, 682, 702-709 or 693-700.

In another particular embodiment, VHH molecules are humanized variants of B8 comprising or consisting of SEQ ID NOs: 273 or 152 (such as B8h1 variant).

In another particular embodiment, VHH molecules of the invention are B6 or variants thereof (as listed in Table 1), comprising or consisting of any of SEQ ID NOs: 12, 168, 170, 172, 173, 174, 178, 181, 183, 185, 215, 285-293, that bind TfR. B6 and variants thereof according to the invention target the apical domain of the TfR.

In another particular embodiment, VHH molecules of the invention are VHH molecules that bind the apical domain of the TfR, preferably an apical domain of the TfR1, and comprise or consist of SEQ ID NOs: 215, 285-299. In the most preferred embodiment, VHH molecules of SEQ ID NOs: 215, 285-299 bind the human TfR1. In a particular embodiment, such VHH molecules comprise a tag sequence at the N- and/or C-terminal end. Specific examples of such variants comprise or consist of any of SEQ ID NOs: 12, 168, 170, 172-174, 178, 181, 183, 185, 189, 193, 197, 204, 208 or 691.

In another particular embodiment, the VHH molecule is B6 or a variant thereof, comprising an amino acid sequence selected from any one of SEQ ID NOs: 215 and 285-293.

In another particular embodiment, the VHH molecule comprises or consists of an amino acid sequence selected from any one of SEQ ID NOs: 613-618. Interestingly, such VHH molecules comprising or consisting of SEQ ID NOs: 613-618 bind human and non-human primate TfR but they do not bind mouse TfR.

Moreover, binding of said VHH of the invention to a human TfR receptor does not compete with binding of transferrin, the endogenous TfR ligand, and thus does not affect regular functions of said ligand. Conjugates produced with such VHH molecules have further been shown to bind TfR *in vitro* and to accumulate in the muscles and/or in the muscle cells *in vivo,* showing endocytosis. Such VHH thus represent potent agents for drug delivery or targeting to muscles.

The VHH of the invention can be synthesized by any technique known to those skilled in the art (biological or genetic synthesis, chemical, etc.). They can be preserved as-they are, or be formulated in the presence of a substance of interest or any acceptable excipient. For chemical syntheses, commercial apparatuses that can incorporate natural as well as non-natural amino acids, such as D enantiomers and residues with side chains with hydrophobicities and steric obstructions different from those of their natural homologues (socalled exotic, *i.e.,* non-coded, amino acids), or a VHH sequence containing one or more peptidomimetic bonds that can include notably intercalation of a methylene (-CH₂-) or phosphate (-PO₂-) group, a secondary amine (-NH-) or an oxygen (-O-) or an N-alkylpeptide, are used. During synthesis, it is possible to introduce various chemical modifications, such as for example, inserting, linking or conjugating in the N-terminal and/or C-terminal position or on a side chain a lipid (or phospholipid) derivative or a constituent of a liposome or a nanoparticle, in order to incorporate the VHH of the invention within a lipid membrane such as that of a liposome composed of one or more lipid layers or bilayers, or of a nanoparticle. Liposomes and nanoparticles are examples of a "vehicle" which may be conjugated with one or more VHH molecules of the invention. The VHH of the invention can also be obtained from a nucleic acid sequence coding for the same, as described further below (cf. Table 2 and the Sequence listing).

### Conjugates

A further object of the invention relates to conjugates (also interchangeably called herein "chimeric agents") comprising one or more VHH molecules as defined above, conjugated to at least one additional compound, in particular to at least one additional molecule, agent or compound of interest, for example to a scaffold of interest.

This additional compound may be a distinct VHH or a molecule which is not a VHH. The at least one additional molecule, agent or compound of interest may be any molecule, agent or compound such as a half-life extending moiety, a stabilizing group or scaffold, a therapeutic (i.e., active) compound, medicament or drug, a diagnostic agent, an imaging molecule, a tracer, etc., or a vehicle comprising such a therapeutic, diagnostic or imaging compound.

In a particular aspect, the chimeric agent (i.e., conjugate) may comprise both kinds of additional compounds, i.e. i) a half-life extending moiety, a stabilizing group or scaffold and ii) a therapeutic, diagnostic or imaging compound, or a vehicle comprising the same.

The therapeutic compound is for example selected from a peptide, a polypeptide, a protein, an antibody, a nucleic acid and any fragment thereof. In a preferred embodiment, the therapeutic compound is a nucleic acid molecule as described below.

Examples of conjugated molecules, agents or compounds of interest include, without limitation, any chemical entity such as a small chemical molecule (for example a chelating agent, an antibiotic, antiviral, immunomodulator, antineoplastic, anti-inflammatory, or adjuvant, etc.); a peptide, polypeptide or protein (for example an enzyme, hormone, cytokine, apolipoprotein, growth factor, antigen, antibody or part of an antibody, adjuvant, etc.); a nucleic acid (for example a RNA or a DNA of human, viral, animal, eukaryotic, prokaryotic, plant or synthetic origin, etc., including e.g., coding genes, inhibitory nucleic acids such as ribozymes, antisense oligonucleotides (ASOs), interfering nucleic acids(siRNAs), small activating RNAs (saRNAs), mRNAs, full genomes or portions thereof, plasmids, etc.); a lipid (nano)particle, a Cell-Derived Vesicle (CDV) such as an exosome, a virus, a marker, or a tracer, for instance. Generally, the "molecule, agent or compound of interest" can be any drug (active) ingredient, whether a chemical, biochemical, natural or synthetic compound. Generally, the expression "small chemical molecule, agent or compound" designates a molecule of pharmaceutical interest with a maximum molecular weight of 1000 Daltons, typically between 300 Daltons and 700 Daltons.

The vehicle may be selected for example from a virus, a virus-like particle (VLP), a Cell-Derived Vesicle (CDV), an exosome, a lipid vehicle and a polymer vehicle, and is preferably a lipid nanoparticle (LNP), a micelle or a liposome.

The conjugated compound is typically a medicament (such as a small drug, nucleic acid or polypeptide, e.g., an antibody or fragment thereof) or an imaging agent suitable for treating or detecting a muscular or neuromuscular disease, such as a myopathy, a cardiomyopathy, a muscular dystrophy (such as DMD, BMD, FSHD, Pompe disease or familial hypertrophic cardiomyopathy), a peripheral neuropathy, ALS, SMA, MS, CMT or a cancer of the muscle (such as rhabdomyosarcoma or leiomyosarcoma).

The chimeric agent may also contain, in addition to or instead of said compound of interest, a half-life extending moiety or a stabilizing group to increase the plasma half-life of the VHH or conjugate. Particular chimeric agents of the invention thus comprise i) at least one VHH, for example several VHH molecules, ii) a half-life extending moiety or a stabilizing group, iii) a compound of interest, typically a therapeutic, diagnostic or imaging compound, and optionally iv) a vehicle, in any order.

In a particular aspect herein described, the compound of interest is at the same time a group allowing the stabilization and/or increasing the plasma half-life of the VHH molecule(s) of the invention. The half-life extending moiety or stabilizing group may be any group known to have substantial plasma half-life (e.g. at least few hours) and essentially no adverse biological activity. Examples of such half-life extending moieties or stabilizing groups include, for instance, an antibody or a fragment thereof such as a Fc fragment of an immunoglobulin, a VHH molecule or variants thereof, preferably VHH molecules binding to albumin, large human serum proteins such as albumin, HSA, or IgGs or PEGs molecules.

In a particular embodiment, the conjugate according to the invention comprises a half-life extending moiety or stabilizing group which is a small organic albumin-moiety that binds to the albumin with a low micromolar affinity thus improving the pharmacokinetic profile of the compound of interest by a progressive release of the conjugate from the albumin. Such small organic albumin-moieties include e.g. a fragment of Evans blue (EB) dye, fatty acids and derivatives thereof such as the C16 group and the 4-(p-iodophenyl)butytryl (PIB) group.

In another particular embodiment, the conjugate according to the invention comprises a half-life extending moiety or stabilizing group which is an Fc fragment of a human IgG1 or IgG4, preferably IgG1. Such a conjugate is of general formula VHH-hFc-siRNA or VHH-hFc-ASO.

In a further particular embodiment, the conjugate according to the invention comprises a half-life extending moiety or stabilizing group which is an Fc homodimer or heterodimer.

In another embodiment, the conjugate according to the invention comprises a half-life extending moiety or stabilizing group which is a modified Fc fragment homodimer or heterodimer of a IgG1 or IgG4 with attenuated or abolished effector functions and/or extended half-life. In a further particular embodiment, the conjugate according to the invention comprises a modified Fc fragment of an IgG1 which is an aglycosylated Fc fragment of an IgG1, e.g., with N297 mutation.

In a further particular embodiment, the conjugate according to the invention comprises a modified Fc fragment of an IgG1 which is a fragment of an Fc variant having a symmetric or asymmetric amino acid modification (i.e, on only one or two arms of the Fc dimer) selected from deletion, insertion, inversion or substitution, or a combination thereof e.g., an amino acid substitution at L234A and L235A (i.e., LALA mutation). Such Fc modifications allow to modulate Fc receptor interactions, modulate, reduce or eliminate Fc effector functions such as FcgammaR binding, antibody-dependent cell-mediated cytotoxicity (ADCC), or complement-dependent cytotoxicity (CDC) or to modulate glycosylation.

In a further particular embodiment, the conjugate according to the invention comprises a modified Fc fragment which is a fragment of an IgG1 comprising a mutation at residue position N434, E380, M252, I253, S254, T256 or H433, or a combination thereof. Specific examples of such mutations are E380A M252Y, S254T, T256E, H433K, N434A or N434F.

In another particular embodiment, the conjugate according to the invention comprises a modified Fc fragment which is a fragment of an IgG1 comprising a mutation at residue position E233, L234, L235, G236, G237, S239, D265, D270, P329, A327, A330, or a combination thereof. Specific examples of such mutations are E233P, L234A, L234V, L235A, deltaG236, G237A, S239A, D265A, D265N, D270N, D270A, A327G, P329A, P329G, A330S or P331S.

The conjugate according to the invention may also comprise a modified Fc fragment which comprises any combination of the above mutations, optionally in a further combination with the LALA mutation.

In another particular embodiment, the conjugate according to the invention may comprise a modified Fc fragment which is a fragment of an IgG4 comprising a mutation at residue position L248 or L235, or a combination thereof. Specific examples of such mutations are L248E, L235A or L235E.

In a further particular embodiment, the conjugate according to the invention comprises a half-life extending moiety or stabilizing group which is albumin protein or albumin binding moieties.

The VHH may be conjugated in N-terminal or C-terminal end of the half-life extending moiety or stabilizing group, or both. When the half-life extending moiety or stabilizing group is a Fc fragment, conjugation is typically by genetic fusion. The resulting protein may remain as a monomeric agent, or multimerize, depending on the nature of the half-life extending moiety or stabilizing group. In the case of a Fc fragment, the fusion protein Fc-VHH or VHH-Fc may form homodimers or heterodimers.

In this regard, in a particular embodiment, the VHH molecule of the invention is conjugated to at least one oligonucleotide, i.e., one or more oligonucleotide(s) (e.g., any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), preferably ASO or siRNA, by means of the half-life extending moiety or stabilizing group such as a Fc fragment of a human IgG1 or IgG4, wherein Fc-VHH or VHH-Fc are homodimers or heterodimers.

For example, a heterodimeric VHH-Fc fusion may be generated using the "knob-into-hole" or "KiH" technology and may comprise a VHH at the N-terminus (or N-ter) of one arm ("knob" arm) of a human IgG1-derived Fc dimer (hFc) and a tag sequence specifically recognized by transglutaminase (TGase) enzyme (namely a Q-tag) inserted in C-terminus (or C-ter) of the other arm of the Fc dimer ("hole" arm), and may be site-specifically modified on the Q-tag to introduce an azido-linker. The resulting heterodimeric VHH-hFc-Q-tag-azido intermediate may be conjugated to alkyne-siRNA using copper-free click reaction, yielding VHH-hFc-siRNA conjugates with a stable linker (as described in Example 6 and shown in Figure 14). In a particular embodiment, the conjugate according to the invention comprises a VHH-Fc heterodimer, wherein Fc comprises the following mutations: T366W on the "knob" arm of the heterodimer, and/or T366S, L368A and Y407V on the "hole" arm of the heterodimer.

In another particular embodiment, the conjugate according to the invention comprises a VHH-Fc heterodimer, wherein Fc comprises T366W, L234A and L235A mutations on the "knob" arm of the heterodimer and/or T366S, L368A, Y407V, L234A and L235A mutations on the "hole" arm of the heterodimer.

In another particular embodiment, the conjugate according to the invention comprises a VHH-Fc heterodimer, wherein Fc comprises T366W, L234A and L235A mutations on the "knob" arm of the heterodimer and T366S, L368A, Y407V, L234A and L235A mutations on the "hole" arm of the heterodimer. In this regard, the VHH-Fc heterodimer may comprise a modified Fc having a sequence of SEQ ID NO: 664 on the "knob" arm, and a modified Fc having a sequence of SEQ ID NO: 665 on the "hole" arm. Specific examples of such VHH-Fc heterodimers comprising SEQ ID NO: 664 and 665 are described in Example 6 and in Figures 8, 9 and 10.

All the above mutations are defined according to the standard Kabat system for numbering the amino acid residues in an antibody.

In the conjugate compounds of the invention, coupling can be performed by any acceptable means of bonding taking into account the chemical nature steric hindrance and number of conjugated entities. Coupling can thus be carried out by one or more covalent, ionic, hydrogen, hydrophobic or Van der Waals bonds, cleavable or non-cleavable in physiological medium or within cells preferably cleavable in particular when the present invention is used in the context of the delivery of at least one active agent on the muscle site. Furthermore, coupling can be made at various reactive groups, and notably at one or more terminal ends and/or at one or more internal or lateral reactive groups. Coupling can also be carried out using genetic engineering.

A strong interaction is necessary between the VHH and different cargos to prevent their dissociation before the conjugate reaches its site of action (i.e., the muscle site). For this reason, the preferred coupling of the invention is covalent coupling, although non-covalent coupling may also be employed. The compound of interest can be coupled with the VHH either at one of the terminal ends (N-terminal or C-terminal), or at a side chain of one of the constitutive amino acids of the sequence (Majumdar S. and Siahaan TJ., "Peptidemediated targeted drug delivery". Med Res Rev., 2012 May;32(3):637-58). The compound of interest can be coupled directly to a VHH, or indirectly by means of a linker or spacer. Means of covalent chemical coupling, calling upon a linker or not, include classical bioconjugation techniques for instance those selected from bi- or multifunctional agents containing alkyl, aryl, thiols, azide, alkynes, nucleotidic or peptide groups by esters, aldehydes or alkyl or aryl acids, anhydride, sulfhydryl or carboxyl groups, groups derived from cyanogen bromide or chloride, carbonyldiimidazole, succinimide esters or sulfonic halides. It also may additionally include specific enzymatic conjugation for example through the bacterial transglutaminase that catalyzes the transamidation on a glutamine, provided that this glutamine is inserted in a specific tag.

In a particular embodiment, coupling (or conjugation) involves genetic fusion. Such a strategy can be used when the coupled molecule is a peptide or polypeptide. In this case, a nucleic acid molecule encoding the VHH fused to the molecule is prepared and expressed in any suitable expression system, to produce the conjugate. General structures of VHH-Oligo conjugates of the invention are shown in Figure 13, and illustrative strategies for conjugating a VHH of the invention to a molecule or scaffold are disclosed in Figure 14.

In another particular embodiment, coupling (or conjugation) is performed using the thiol/maleimide chemistry technology. For this reaction to occur, the VHHs are fused to their C-terminus with a peptidic sequence containing an additional cysteine. In particular, the additional peptidic tag is typically GGGGSCHHHHHH (SEQ ID NO: 399, wherein simple underline is a Gly linker that is used as a spacer and double underline is a 6His tag that is used for purification purposes.

Since VHHs only contain cysteines engaged in a disulfide bridge, the additional cysteine introduced in the tag is the only one to be chemically reactive towards maleimides. It allows the specific conjugation of the VHH with maleimide-derivatized molecules of interest. The reaction proceeds in two steps. First it is necessary to smoothly reduce the VHH-GGGGSCHHHHHH since the additional cysteine in the tag can be partially engaged in a disulfide bridge during the production process (formation of a disulfide bond with another VHH-GGGGS**C**HHHHHH or with a free cysteine). The first step thus consists in the partial reduction using a mild reducing agent such as 2-MEA (2-mercaptoethanol), TCEP (tris(2-carboxyethyl)phosphine) or DTT (dl-1,4-dithiothreitol). In a second step, the VHH-GGGGSCHHHHHH is allowed to react at a pH in the range of 6.5 - 7.5 with maleimide-functionalized molecules of interest to form VHH-molecule conjugates linked in a covalent and stable manner. The two steps can be performed sequentially or all together *in situ.*

In another particular embodiment, coupling (or conjugation) is performed by enzymatic reaction. In particular, site-specific conjugation onto the VHH can be performed using the transglutaminase enzyme (Tgase). Tgase catalyzes the formation of a stable isopeptidic bond between (i) the side chain of a glutamine residue inserted in a tag sequence specifically recognized by the Tgase (namely a Q-tag) and (ii) an aminofunctionalized donor substrate. In this regard, the inventors have developed a particular tag sequence (named "Q-tag") which is recognized by Tgase and may be used to couple VHH of the invention to any molecule of interest, either chemical drugs or agents or a heterobifunctional linker for further conjugation to chemical drugs or agents. For this purpose, VHHs are prepared by genetic fusion to add in tandem (typically to their C-terminus) the following tags: first an optional trialanine linker, then an optional His-tag, then an optional small triglycine linker, and finally a Q-tag. The triglycine linker allows spacing out the Q-tag to allow a better accessibility of the Tgase to the glutamine while the His-tag aims at facilitating the purification of the VHH and its further functionalized versions.

The general conjugation strategy that was developed is a convergent synthesis that is based on a process comprising:
1) introduction onto the glutamine in the Q-tag fused to the VHH of a reactive moiety for further conjugation to a molecule of interest. In this objective, a heterobifunctional conjugation linker having two different reactive ends is allowed to be processed by the Tgase: one suitable primary amine-group toward the Tgase and one orthogonal reactive moiety. Representative examples of such orthogonal and reactive groups include azides, constraints alkynes such as DBCO (dibenzocyclooctyne) or BCN (bicyclo[6.1.0]nonyne), tetrazines, TCO (trans-cyclooctene), free or protected thiols, maleimides, etc.
2) introduction onto the molecule of interest of a reactive moiety complementary to the one incorporated onto the VHH Q-tag. Representative examples of such orthogonal and reactive groups include azides, constrained alkynes such as DBCO or BCN, tetrazines, TCO, free or protected thiols, maleimides, etc.
3) conjugation of both the functionalized VHH and molecule owing to their complementary reactive groups.

Herein described is also a method for coupling two molecules using a Q-tag as defined above through Tgase coupling reaction. A further object of the invention is a VHH comprising a Q-tag. A further object of the invention is a VHH molecule comprising a linker, such as a Gly linker, and a Q-tag.

Preferred VHH of the invention have the following structure: VHH - Linker - Hisₘ - Linker - LQR, wherein: VHH is any VHH molecule; Linker is any molecular linker such as an Ala or Gly linker (preferably the two linkers are different); and m is an integer from 0 to 8, preferably m is 6 or 8.

In a particular embodiment, the invention relates to a conjugate comprising at least one VHH covalently linked to at least one chemical entity. Preferred variants of such conjugates contain one VHH and one chemical entity.

In another particular embodiment, the invention relates to a conjugate comprising at least one VHH covalently linked to at least one nucleic acid. The nucleic acid may be an antisense oligonucleotide ("ASO"), a ribozyme, an aptamer, a siRNA, etc. Preferred variants of such conjugates contain one VHH and one nucleic acid molecule. In a preferred embodiment, the nucleic acid is an oligonucleotide (i.e., a short DNA or RNA molecule, also called herein "oligo" or "oligomer") selected from any single- or double-stranded oligonucleotide such as a small interfering RNA (siRNA), small activating RNAs (saRNA), gapmer, antisense oligonucleotide (ASO), shRNA, miRNA, aptamer RNA or bridged nucleic acid (BNA), which can specifically bind to a target mRNA thereby modulating gene expression in the cell. Preferably, such an oligonucleotide is an ASO or a siRNA capable of modulating (silencing or inhibiting or activating) the expression of a target gene.

The conjugates according to the invention are complementary to a target gene, and, in particular embodiments, they are capable of silencing or inhibiting the expression of a target gene, preferably by at least 40 %, or at least 50 %, or at least 60 %, or at least 70 %, or at least 80 %, or at least 90 %. More specifically, the conjugates according to the invention are capable of silencing or inhibiting the expression of a target gene by about 40 % to about 80 %.

Accordingly, in a particular embodiment, the invention relates to a conjugate compound comprising at least one VHH binding TfR, preferably at the surface of a muscle cell, and covalently linked to at least one oligonucleotide selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA thereby modulating gene expression in the cell.

In a preferred embodiment, the invention relates to a conjugate compound comprising:
(i) one or more VHH molecule(s) binding TfR at the surface of a muscle cell, comprising:
   - a CDR1 comprising a sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710 and 711, and/or
   - a CDR2 comprising a sequence selected from SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611 and 712, and/or
   - a CDR3 comprising a sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 and 713-715; and
(ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby reducing its level of expression in the cell.

In another preferred embodiment, the invention relates to a conjugate compound comprising:
(i) one or more VHH molecule(s) binding TfR at the surface of a muscle cell, and comprising SEQ ID NOs: 1, 2 and 3; or SEQ ID NOs: 5, 6 and 7; or SEQ ID NOs: 9, 10 and 11; or SEQ ID NOs: 13, 14 and 15; SEQ ID NOs: 17, 2 and 3; or SEQ ID NOs: 19, 2 and 3; or SEQ ID NOs: 1, 21 and 3; or SEQ ID NOs: 1, 23 and 3; or SEQ ID NOs: 1, 2 and 25; or SEQ ID NOs: 1, 2 and 27; or SEQ ID NOs: 1, 2 and 29; or SEQ ID NOs: 1, 2 and 31; or SEQ ID NOs: 1, 2 and 33; or SEQ ID NOs: 67, 2 and 3; or SEQ ID NOs: 69, 2 and 3; or SEQ ID NOs: 1, 71 and 3; or SEQ ID NOs: 1, 73 and 3; or SEQ ID NOs: 1, 75 and 3; or SEQ ID NOs: 1, 2 and 77; or SEQ ID NOs: 1, 2 and 79; or SEQ ID NOs: 1, 2 and 81; or SEQ ID NOs: 1, 2 and 83; or SEQ ID NOs: 1, 2 and 85; or SEQ ID NOs: 392, 2 and 3; or SEQ ID NOs: 1, 113 and 3; or SEQ ID NOs: 1, 115 and 3; or SEQ ID NOs: 1, 2 and 117; or SEQ ID NOs: 1, 2 and 119; or SEQ ID NOs: 1, 2 and 121; or SEQ ID NOs: 1, 2 and 123; or SEQ ID NOs: 125, 2 and 3; or SEQ ID NOs: 17, 73 and 3; or SEQ ID NOs: 17, 128 and 3; or SEQ ID NOs: 5, 160 and 7; or SEQ ID NOs: 5, 162 and 7; or SEQ ID NOs: 5, 164 and 7; or SEQ ID NOs: 5, 166 and 7; or SEQ ID NOs: 9, 169 and 11; or SEQ ID NOs: 9, 171 and 11; or SEQ ID NOs: 175, 176 and 177; or SEQ ID NOs: 179, 176 and 180; or SEQ ID NOs: 182, 176 and 177; or SEQ ID NOs: 184, 176 and 177; or SEQ ID NOs: 186, 187 and 188; or SEQ ID NOs: 190, 191 and 192; or SEQ ID NOs: 194, 195 and 196; or SEQ ID NOs: 198, 199 and 200; or SEQ ID NOs: 201, 202 and 203; or SEQ ID NOs: 205, 206 and 207; or SEQ ID NOs: 410, 6 and 7; or SEQ ID NOs: 413, 6 and 7; or SEQ ID NOs: 5, 416 and 7; or SEQ ID NOs: 5, 419 and 7; or SEQ ID NOs: 426, 6 and 7; or SEQ ID NOs: 5, 431 and 7; or SEQ ID NOs: 434, 6 and 7; or SEQ ID NOs: 437, 6 and 7; or SEQ ID NOs: 5, 6 and 452; or SEQ ID NOs: 5, 6 and 455; or SEQ ID NOs: 607, 608 and 609; or SEQ ID NOs: 610, 611 and 612; or SEQ ID NOs: 671, 2 and 3; or SEQ ID NOs: 672, 2 and 3; or SEQ ID NOs: 673, 6 and 7; or SEQ ID NOs: 674, 6 and 7; or SEQ ID NOs: 1, 2 and 713; or SEQ ID NOs: 5, 6 and 714; or SEQ ID NOs: 674, 164 and 7; or SEQ ID NOs: 710, 6 and 7; or SEQ ID NOs: 5, 6 and 715; or SEQ ID NOs: 674, 712 and 7; or SEQ ID NOs: 711, 6 and 7; and
(ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby modulating gene expression in the cell.

In a preferred embodiment, the conjugate of the invention comprises: (i) at least one VHH molecule binding TfR at the surface of a muscle cell, and comprising or consisting of any of SEQ ID NOs: 4, 8, 12, 16, 18, 20, 22, 24, 26, 28, 30, 32, 34, 68, 70, 72, 74, 76, 78, 80, 82, 84, 86-92, 114, 116, 118, 120, 122, 124, 126, 127, 129-149, 152-159, 161, 163, 165, 167, 168, 170, 172-174, 178, 181, 183, 185, 189, 193, 197, 204, 208, 213-271, 273-299, 393, 411-412, 414-415, 417-418, 420-425, 427-430, 432-433, 435-436, 438-451, 453-454, 456-457 or 691-709; and (ii) at least one oligonucleotide selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby modulating gene expression in the cell.

In another preferred embodiment, the conjugate of the invention comprises: (i) one or more VHH molecule(s) which is C5 or a variant thereof (as listed in Table 1), which is cross-species VHH molecule binding to human, non-human primate (NHP) and murine TfR at the surface of a muscle cell, and which comprises or consists of any of SEQ ID NOs: 4, 16, 18, 20, 22, 24, 26, 28, 30, 32, 34, 68, 70, 72, 74, 76, 78, 80, 82, 84, 86-92, 114, 116, 118, 120, 122, 124, 126, 127, 129 or 130-149, 153-154, 213, 216-271, 274-275, 393, 675, 676, 679, 680, 692 or 701; and (ii) one or more oligonucleotide(s) selected from any singleor double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby modulating gene expression in the cell.

In another preferred embodiment, the conjugate of the invention comprises: (i) at least one VHH molecule which is a humanized variant of C5 (as listed in Table 1), comprising or consisting of any of SEQ ID NOs: 87-92, 130-149, 153-154, 236-241, 252-271 or 274-275; and (ii) at least one oligonucleotide selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable of specifically binding to a target mRNA, thereby modulating gene expression in the cell.

In another preferred embodiment, the conjugate of the invention comprises: (i) one or more VHH molecule(s) which is B6 or a variant thereof (as listed in Table 1), which binds to the apical domain of the TfR, preferably TfR1 at the surface of a muscle cell, and which comprises or consists of any of SEQ ID NOs: 12, 168, 170, 172-174, 178, 181, 183, 185, 215, 285-299; and (ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable specifically binding to a target mRNA, thereby modulating gene expression in the cell.

In another preferred embodiment, the conjugate of the invention comprises: (i) one or more VHH molecule(s) binding to the apical domain of the TfR, preferably TfR1 at the surface of a muscle cell, and comprising or consisting of any of SEQ ID NOs: 12, 168, 170, 172-174, 178, 181, 183, 185, 189, 193, 197, 204, 208, 215, 285-299 or 691; and (ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable specifically binding to a target mRNA, thereby modulating gene expression in the cell.

In another preferred embodiment, the conjugate of the invention comprises: (i) one or more VHH molecule(s) which is B8 or a variant thereof (as listed in Table 1), comprising or consists of any of SEQ ID NOs: 8, 152, 155-159, 161, 163, 165, 167, 214, 273, 276-284, 411, 412, 414, 415, 417, 418, 420-425, 427-430, 432, 433, 435, 436, 438-451, 453, 454, 456, 457, 677, 678, 681, 682, 693-700, 702-709; and (ii) one or more oligonucleotide(s) selected from any single- or double-stranded oligonucleotide such as small interfering RNA (siRNA), small activating RNAs (saRNA), gapmer, antisense oligonucleotide (ASO), shRNA, miRNA, aptamer RNA or bridged nucleic acid (BNA), which is capable specifically binding to a target mRNA, thereby modulating gene expression in the cell.

Specific examples of conjugation methods used to conjugate a VHH molecule to an oligonucleotide are the direct thiol-maleimide chemistry thanks to the introduction of an unpaired cysteine to the C-terminus of the VHH or an indirect SPAAC reaction (Strain Promoted Alkyne Assisted Click) thanks to the preliminary site-specific enzyme-assisted reaction onto the VHH to introduce suitable moieties for click chemistry. Different linkers can be used between the two partners VHH and oligos, that can be stable or cleavable. Example of cleavable linkers are disulfide, enzyme-labile peptide linkers such as valine-citrulline or phenylalanine-lysine dipeptide or pH-labile linkers such as hydrazone.

In a particular embodiment, a VHH molecule and oligonucleotide are linked to each other using preferably a copper-free click reaction yielding VHH-Oligo conjugates with a stable or a cleavable linker.

In another particular embodiment, the invention relates to a conjugate comprising a VHH covalently linked to a peptide. The peptide may be an active molecule, a bait, a tag, a ligand, etc. Preferred variants of such conjugates contain one VHH and one peptide.

In another embodiment, the invention relates to a conjugate comprising a VHH covalently linked to a nanoparticle and/or liposome, for example a lipidic particle or nanoparticle ("LNP"). The nanoparticle and/or liposome may be loaded or functionalized with active agents. Preferred variants of such conjugates contain several VHH molecules coupled to each nanoparticle or liposome.

In a further embodiment, the conjugate comprises, as a half-life extending moiety or a stabilizing group, an antibody or a fragment thereof to which one or several VHH molecules are coupled. Typically, a VHH molecule is coupled to a C- or N-terminal end of a heavy or light chain, or both, or to the C- or N-terminal of an Fc fragment. In a preferred aspect, the VHH molecule is coupled to the N-terminal end of an Fc. In an even more preferred aspect, the VHH molecule is coupled to the C-terminal end of an Fc.

In another particular aspect, the conjugate comprises, or consists of, a single VHH molecule coupled to an antibody fragment which may be a heavy chain or a light chain. In this aspect, the VHH molecule is indifferently coupled to the C-terminal end or to the N-terminal end of the chain. Preferably, it is coupled to the N-terminal end.

The invention also relates to a method for preparing a conjugate compound such as defined above, characterized in that it comprises a step of coupling between a VHH and a molecule or scaffold, preferably by a chemical, biochemical or enzymatic pathway, or by genetic engineering.

In a chimeric agent of the invention, when several VHH are present, they may have a similar or different binding specificity.

### Nucleic acids, vectors and host cells

A further aspect of the invention relates to a nucleic acid encoding a VHH as defined above, or a conjugate thereof (when the conjugated moiety is an amino acid sequence). The nucleic acid may be single- or double-stranded. The nucleic acid can be DNA (for example a cDNA or a gDNA), a RNA (for example a mRNA or a gRNA), or a mixture thereof. It can be in single stranded form or in duplex form, or it can be a mixture of the two. It can comprise modified nucleotides, comprising for example a modified bond, a modified purine or pyrimidine base, or a modified sugar. It can be prepared by any method known to one of ordinary skill in the art, including chemical synthesis, recombination and/or mutagenesis. The nucleic acid according to the invention may be deduced from the amino acid sequence of the VHH molecules according to the invention and codon usage may be adapted according to the host cell in which the nucleic acid shall be transcribed. These steps may be carried out according to methods well known to one of ordinary skill in the art and some of which are described in the reference manual Sambrook et al.

Specific examples of such nucleic acids sequences include the sequences comprising any one of SEQ ID NOs: 301-329, 52-64, 95-110, 469-606, 646-663, 683-690 or 721-738, with no tag coding portion or including the optional tag-coding portion of SEQ ID NO: 330, and the complementary sequence thereto. The corresponding domains encoding CDR1, CDR2 and CDR3 are underlined in Table 2 below. The tag-coding portion of SEQ ID NO: 330 is in bold in Table 2.

The invention also relates to a vector containing such a nucleic acid, optionally under control of regulatory sequences (e.g., promoter, terminator, etc.). The vector may be a plasmid, virus, cosmid, phagemid, artificial chromosome, etc. In particular, the vector may comprise a nucleic acid of the invention operably linked to a regulatory region, i.e. a region comprising one or more control sequences. Optionally, the vector may comprise several nucleic acids of the invention operably linked to several regulatory regions. The term "control sequences" means nucleic acid sequences necessary for expression of a coding region. Control sequences may be endogenous or heterologous. Well-known control sequences and currently used by the person skilled in the art will be preferred. Such control sequences include, but are not limited to, promoter, signal-peptide sequence and transcription terminator. The term "operably linked" means a configuration in which a control sequence is placed at an appropriate position relative to a coding sequence, in such a way that the control sequence directs expression of the coding region.

The present invention further relates to the use of a nucleic acid or vector according to the invention to transform, transfect or transduce a host cell or to produce a composition, including a pharmaceutical composition, to transform, transfect or transduce a host cell. The present invention also provides a host cell comprising one or several nucleic acids of the invention and/or one or several vectors of the invention. The term "host cell" also encompasses any progeny of a parent host cell that is not identical to the parent host cell due to mutations that occur during replication. Suitable host cells may be prokaryotic (e.g., a bacterium) or eukaryotic (e.g., yeast, plant, insect or mammalian cell). Specific illustrative examples of such cells include *E. coli* strains, CHO cells, *Saccharomyces* strains, plant cells, sf9 insect cells etc.

### Uses

VHH molecules of the invention can bind to TfR and thus target/deliver molecules to TfR-expressing muscle cells. Within the context of this invention, binding is preferably specific, so that binding to TfR occurs with higher affinity than binding to any other antigen in the same species. In a particular embodiment, the VHH molecules of the invention specifically bind the human TfR1. In another particular embodiment, the VHH molecules of the invention bind the human and non-human primate TfR1. In another particular embodiment, the VHH molecules of the invention bind the human and rodent TfR1. In another particular embodiment, VHH molecules of the invention bind human TfR1, non-human primate TfR1 and rodent (e.g., a murine or rat) TfR1. In another particular embodiment, the VHH molecules bind the human, non-human primate and murine receptors with a substantially similar affinity.

The invention thus relates to methods of targeting/delivering a compound to/through a TfR-expressing cell or organ, comprising coupling said compound to at least one VHH of the invention.

The invention further relates to the use of a VHH such as defined above, as a vector for the transport of a compound to/through a TfR-expressing muscle cell.

The invention also relates to the use of a VHH such as defined above for preparing a drug or a medicament capable of targeting the muscle site.

The invention also relates to a method for enabling or improving the addressing of a compound of interest to the muscle site, comprising the coupling of the molecule to a VHH of the invention.

As explained herein above, the VHH of the invention may be used to transport or deliver any compound, such as for example chelating agents, small drugs, amino acids, peptides, polypeptides, proteins, lipids, nucleic acids, viruses, liposomes, exosomes etc., preferably nucleic acids, to the muscle.

A vehicle may be used to transport or deliver the conjugate (including the VHH) such as for example a virus, a virus-like particle (VLP), a Cell-Derived Vesicle (CDV), an exosome, a lipid vehicle or a polymer vehicle, and is preferably a lipid nanoparticle (LNP), a micelle or a liposome.

The invention also relates to a pharmaceutical composition, in particular a diagnostic or therapeutic composition, characterized in that it comprises at least one VHH or conjugate compound, associated to, or present in, a vehicle or not, for example, in the context of a therapeutic composition, a VHH-drug conjugate such as defined above and one or more pharmaceutically acceptable supports, carriers or excipients.

The invention also in particular relates to a diagnostic composition characterized in that it comprises a VHH or conjugate compound, associated to, or present in, a vehicle or not, for example a VHH-diagnostic or medical imaging agent conjugate compound such as defined above.

The conjugate can be used in the form of any pharmaceutically acceptable salt. The expression "pharmaceutically acceptable salts" refers to, for example and in a non-restrictive way, pharmaceutically acceptable base or acid addition salts, hydrates, esters, solvates, precursors, metabolites or stereoisomers, said vectors or conjugates loaded with at least one substance of interest. The expression "pharmaceutically acceptable salts" refers to nontoxic salts, which can be generally prepared by reacting a free base with a suitable organic or inorganic acid. These salts preserve the biological effectiveness and the properties of free bases. Representative examples of such salts include water-soluble and water-insoluble salts such as acetates, N-methylglucamine ammonium, amsonates (4,4-diaminostilbene-2,2'-disulphonates), benzenesulphonates, benzonates, bicarbonates, bisulphates, bitartrates, borates, hydrobromides, bromides, buryrates, camsylates, carbonates, hydrochlorates, chlorides, citrates, clavulanates, dichlorhydrates, diphosphates, edetates, calcium edetates, edisylates, estolates, esylates, fumarates, gluceptates, gluconates, glutamates, glycolylarsanylates, hexafluorophosphates, hexylresorcinates, hydrabamines, hydroxynaphthoates, iodides, isothionates, lactates, lactobionates, laurates, malates, maleates, mandelates, mesylates, methylbromides, methylnitrates, methylsulphates, mucates, napsylates, nitrates, 3-hydroxy-2-naphthoates, oleates, oxalates, palmitates, pamoates (1,1-methylene-bis-2-hydroxy-3-naphtoates, or emboates), pantothenates, phosphates, picrates, polygalacturonates, propionates, p-toluenesulphonates, salicylates, stearates, subacetates, succinates, sulphates, sulphosalicylates, suramates, tannates, tartrates, teoclates, tosylates, triethiodides, trifluoroacetates and valerianates.

The compositions of the invention advantageously comprise a pharmaceutically acceptable carrier or excipient. The pharmaceutically acceptable carrier can be selected from the carriers classically used according to each mode of administration. According to the mode of administration envisaged, the compounds can be in solid, semi-solid or liquid form. For solid compositions such as tablets, pills, powders, or granules that are free or are included in gelatin capsules, the active substance can be combined with: a) diluents, for example lactose, dextrose, sucrose, mannitol, sorbitol, cellulose and/or glycine; b) lubricants, for example silica, talc, stearic acid, its magnesium or calcium salt and/or polyethylene glycol; c) binders, for example magnesium and aluminum silicate, starch paste, gelatin, tragacanth, methylcellulose, sodium carboxymethyl cellulose and/or polyvinylpyrrolidone; d) disintegrants, for example starch, agar, alginic acid or its sodium salt, or effervescent mixtures; and/or d) absorbents, dyes, flavoring agents and sweeteners. The excipients can be, for example, mannitol, lactose, starch, magnesium stearate, sodium saccharin, talc, cellulose, glucose, sucrose, magnesium carbonate and analogues of pharmaceutical quality. For semi-solid compositions such as suppositories, the excipient can, for example, be an emulsion or oily suspension, or polyalkylene glycol-based, such as polypropylene glycol. Liquid compositions, in particular injectables or those included in a soft capsule, can be prepared, for example, by dissolution, dispersion, etc., of the active substance in a pharmaceutically pure solvent such as, for example, water, physiological saline solution, aqueous dextrose, glycerol, ethanol, oil and analogues thereof.

The compositions or conjugates of the invention can be administered by any suitable route and, in a non-restrictive way, by parenteral route, such as, for example, in the form of preparations that can be injected by subcutaneous, intravenous or intramuscular route; by oral route (or *per os*), such as, for example, in the form of coated or uncoated tablets, gelatin capsules, powders, pellets, suspensions or oral solutions (one such form for oral administration can be either with immediate release or with extended or delayed release); by rectal route such as, for example, in the form of suppositories; by topical route, in particular by transdermal route, such as, for example, in the form of patches, pomades or gels; by intranasal route such as, for example, in aerosol and spray form; by perlingual route; or by intraocular route. Preferably, the VHH or conjugate of the invention, or composition comprising such a VHH or conjugate, is administered by intravenous or subcutaneous route.

The pharmaceutical compositions typically comprise an effective dose of a VHH or conjugate of the invention. The "therapeutically effective dose" of the conjugate of the invention is for example of about 1 nmole to about 500 nmoles per kilo of body weight of the subject who will be administered with said conjugate (nmoles/kg), preferably of about 10 nmoles/kg to about 500 nmoles/kg. It is understood that the "therapeutically effective dose" for a person in particular will depend on various factors, including the activity/effectiveness of the active substance, its time of administration, its route of administration, its toxicity, its rate of elimination and its metabolism, drug combinations/interactions and the severity of the disease (or disorder) treated on a preventive or curative basis, as well as the age, weight, overall health, sex and/or diet of the patient.

Depending on the substance coupled, the conjugates and compositions of the invention can be used for imaging, diagnosing, preventing and/or treating pathologies or disorders affecting the muscles, such as for example any muscular or neuromuscular disease preferably, e.g., a myopathy, a cardiomyopathy, a muscular dystrophy (such as DMD, BMD, FSHD, Pompe disease or familial hypertrophic cardiomyopathy), a peripheral neuropathy, ALS, SMA, MS, CMT and a cancer of the muscle (such as rhabdomyosarcoma, leiomyosarcoma, etc.). The VHH of the invention have the capacity to target TfR-expressing cells, particularly muscle cells (such as skeletal or cardiac muscle cells or muscle cancer cells, as described herein) and/or to cross muscle cell membranes. The TfR is enriched in muscles compared to distinct organs such as bone marrow, placenta and in the gastrointestinal tract.

In this respect, the invention relates to the use of a pharmaceutical conjugate or pharmaceutical composition (in particular a therapeutic composition) as described herein above for preventing or treating muscle pathologies or disorders such as in a non-restrictive manner, any muscular or neuromuscular disease.

In the context of the present invention, the muscle disease is a muscular or a neuromuscular disease preferably selected from a myopathy, a cardiomyopathy, a muscular dystrophy (such as DMD, BMD, FSHD, Pompe disease or familial hypertrophic cardiomyopathy), a peripheral neuropathy, ALS, SMA, MS, CMT or a cancer of the muscle (such as rhabdomyosarcoma, leiomyosarcoma, etc.).

The invention also relates to a VHH, conjugate, or pharmaceutical composition as described herein above for use for treating a muscle pathology or disorder such as a myopathy, a cardiomyopathy, a muscular dystrophy (such as DMD, BMD, FSHD, Pompe disease or familial hypertrophic cardiomyopathy), a peripheral neuropathy, ALS, SMA, MS, CMT or a cancer of the muscle (such as rhabdomyosarcoma or leiomyosarcoma).

The invention also relates to a VHH, conjugate or pharmaceutical composition such as defined above for use for treating pathologies affecting muscles such as, in a non-restrictive manner, a muscular or a neuromuscular disease such as a myopathy, a cardiomyopathy, a muscular dystrophy (such as DMD, BMD, FSHD, Pompe disease or familial hypertrophic cardiomyopathy), a peripheral neuropathy, ALS, SMA, MS, CMT, or a cancer of the muscle (such as rhabdomyosarcoma or leiomyosarcoma), etc.

The invention also relates to a VHH, conjugate or pharmaceutical composition such as defined herein above for use for imaging, diagnosing, preventing and/or treating a muscular or a neuromuscular disease such as a myopathy, a cardiomyopathy, a muscular dystrophy (such as DMD, BMD, FSHD, Pompe disease or familial hypertrophic cardiomyopathy), a peripheral neuropathy, ALS, SMA, MS, CMT, or a cancer of the muscle (such as rhabdomyosarcoma or leiomyosarcoma), etc.

The invention also relates to a VHH, conjugate or pharmaceutical composition such as defined herein above, wherein the conjugated agent is or comprises a virus or a virus-like particle, such as a recombinant virus. The invention may indeed be used to increase any TfR enriched muscle tissue delivery of recombinant (e.g., replication-defective or attenuated) viruses used in gene therapy, such as adenoviruses, adenoassociated viruses, lentiviruses, retroviruses, etc., or virus-like particles. Coupling to a virus or VLP may be performed e.g., by coupling to the capsid protein of the virus.

The invention also relates to methods for preventing or treating any of the above conditions or diseases by administering to a subject in need thereof a VHH, conjugate or composition of the invention.

The invention also relates to the use of a VHH, conjugate or composition of the invention for the manufacture of a medicament for treating any of the above conditions or diseases.

The following examples are included merely for purposes of illustration of certain aspects and embodiments of the present invention and are not intended to limit the scope of the claims provided herein.

### EXAMPLES

### EXAMPLE 1: Determination of VHH binding properties to hTfR, mTfR and rhTfR.

The binding properties of VHHs with affinity for the TfR from different species (ie: human (h), mouse (m) and rhesus monkey (rh)) were tested using flow cytometry, and apparent affinities (K_{d app}) were determined. All experiments were performed in 96 well plates using 2 × 10⁵ cells/well, at 4 °C with shaking. CHO cell lines expressing the TfR fused to EGFP or CHO WT cells were saturated with PBS/BSA 2% solution during 30 min to avoid nonspecific binding, followed by incubation with purified VHHs at increasing concentrations for 1 hr. After one wash in PBS/BSA 2%, cells were incubated for 1 hr with an anti-6His tag antibody (mouse), washed twice with PBS/BSA 2%, and incubated for 1 hr with an Alexa647-conjugated anti-mouse secondary antibody. After two last washes in PBS/BSA 2%, cells were fixed by incubation for 15 min with PBS/PFA 2%, washed once with PBS and finally resuspended in PBS. Fluorescence levels were assessed using an Attune NxT flow cytometer (Thermo Fisher Scientific). Experimental data were fitted with GraphPad Prism^{®} software using a nonlinear fit to determine the apparent *K*_{d} constants.

There was no nonspecific labelling in the control conditions where cells were incubated with control VHH D 12 or C5neg. All tested VHHs induced a concentration-dependent shift of the signal, confirming binding to the receptor of interest. No labeling of the CHO WT control cells was detected with all the tested VHHs (not shown). Table 5 hereafter summarizes apparent K_{d} obtained for the tested VHHs. Most VHH showed cross-species reactivity and bound all three human, mouse and rhesus monkey TfR with apparent K_{d} ranging from 0.1 nM to 4 µM, highlighting the great interest and diversity of the VHH library generated.

**Table 5:**

| **VHH name** | **Apparent K_{d} on human TfR (nM)** | **Apparent K_{d} on mouse TfR (nM)** | **Apparent K_{d} on rhesus monkey TfR (nM)** |
|---|---|---|---|
| C5 | 2,7 (± 0,4) | 50 (± 13) | 4,9 (± 1,3) |
| B8 | 1,7 (± 0,3) | 7,5 (± 1,1) | 2,2 (± 1,2) |
| B6 | 2,1 (± 0,4) | NB | NB |
| H3 | 1,9 (± 0,3) | 56 (± 4,7) | 1,5 (± 0,3) |
| C5^{v1} | 3,2 (± 0,5) | 259 (± 62) | 13 (± 2,4) |
| C5^{v2} | 3,2 (± 0,6) | 136 (± 41) | 5,7 (± 1,8) |
| C5^{v3} | 3,3 (± 0,6) | 138 (± 42) | 7,0 (± 3,5) |
| C5^{v4} | 3,1 (± 0,5) | 179 (± 51) | 3,4 (± 0,7) |
| C5^{v8} | 23 (± 6,9) | 427 (± 146) | 494 (± 112) |
| C5^{v9} | 3,4 (± 0,5) | 47 (± 17) | 4,6 (± 1,4) |
| C5^{v10} | 3,4 (± 0,9) | 159 (± 70) | 5,1 (± 1,5) |
| C5^{v11} | 3,4 (± 0,8) | 197 (± 78) | 20 (± 12) |
| C5^{v13} | 3,5 (± 1,0) | 187 (± 76) | 28 (± 20) |
| C5^{v14} | 3,3 (± 0,8) | 158 (± 69) | 3,9 (± 0,9) |
| C5^{v15} | 3,7 (± 1,0) | 207 (± 93) | 10 (± 1,6) |
| C5^{v16} | 4,7 (± 1,2) | 131 (± 55) | 425 (± 39) |
| C5^{v18} | 12 (± 3,1) | 416 (± 151) | 481 (± 55) |
| C5^{V20} | 2,5 (± 0,5) | 125 (± 101) | 6,1 (± 2,3) |
| C5^{V21} | 2,4 (± 0,3) | 120 (± 60) | 8,2 (± 3,8) |
| C5^{V22} | 2,7 (± 0,7) | 131 (± 69) | 11 (± 6,2) |
| C5^{V23} | 4,5 (± 1,5) | 254 (± 169) | 353 (± 19) |
| C5^{V25} | 4,5 (± 0,5) | 136 (± 31) | 429 (± 78) |
| C5^{V26} | 7,9 (± 1,2) | 211 (± 131) | 335 (± 53) |
| C5^{V27} | 3,1 (± 0,8) | 197 (± 74) | 178 (± 81) |
| C5^{V28} | 3,3 (± 0,2) | 160 (± 23) | 371 (± 93) |
| C5^{V29} | 2,9 (± 0,8) | 207 (± 95) | 66 (± 19) |
| C5^{V30} | 31 (± 12) | 696 (± 142) | 362 (± 68) |
| C5^{V31} | 11 (± 2,3) | 445 (± 45) | 256 (± 74) |
| C5^{h9} | 6,1 (± 0,5) | 431 (± 59) | 493 (± 128) |
| C5^{h12} | 4,3 (± 0,7) | 331 (± 100) | 361 (± 42) |
| C5^{h20} | 4,1 (± 1,7) | 22 (± 14) | 40 (± 7,9) |
| B8^{V2} | 0,1 (± 0,01) | 0,2 (± 0,02) | 0,1(± 0,02) |
| B8^{V3} | 36 (± 10) | 52 (± 14) | 19 (± 3,9) |
| B8^{V4} | 0,4 (± 0,08) | 0,6 (± 0,02) | 0,3 (± 0,08) |
| B8^{V5} | 2,3 (± 0,5) | 3,8 (± 0,3) | 2,1 (± 0,5) |
| B8^{V6} | 3,8 (± 0,9) | 16 (± 9,4) | 2,7 (± 0,4) |
| B8^{V7} | 5,7 (± 4,9) | 41 (± 19) | 2,6 (± 0,6) |
| B8^{V8} | 2,5 (± 0,8) | 8,1 (± 1,8) | 2,1 (± 0,6) |
| B8^{V9} | 2,8 (± 0,9) | 31 (± 10) | 2,4 (± 0,5) |
| B8^{V11} | 4,6 (± 0,5) | 22 (± 11) | 2,0 (± 0,3) |
| B8^{V12} | 4,7 (± 0,8) | 743 (± 210) | 6,9 (± 4,3) |
| B8^{V14} | 4,8 (± 0,2) | 41 (± 20) | 1,9 (± 0,3) |
| B8^{V15} | 4,7 (± 1,0) | 684 (± 114) | 4,3 (± 1,3) |
| B8^{V16} | 6,2 (± 1,6) | 3,6 (± 0,5) | 307 (± 234) |
| B8^{V17} | 4,8 (± 0,6) | 5,2 (± 0,6) | 2,5 (± 0,7) |
| B8^{V18} | 3,8 (± 0,8) | 43 (± 29) | 1,6 (± 0,2) |
| B8^{V19} | 6,2 (± 1,6) | 7,6 (± 0,9) | 2,3 (± 0,07) |
| B8^{V20} | 4,5 (± 0,4) | 6,4 (± 2,4) | 1,8 (± 0,08) |
| B8^{V21} | 29 (± 6,6) | 3352 (± 1293) | 469 (± 218) |
| B8^{V25} | 14 (± 2,4) | 2353 (± 1099) | 425 (± 226) |
| B8^{V27} | 6,8 (± 0,4) | 760 (± 205) | 16 (± 3,6) |
| B8^{V29} | 9,0 (± 1,6) | 1667 (± 696) | 72 (± 22) |
| B8^{V31} | 14 (± 0,7) | 1689 (± 462) | 20 (± 3,5) |
| B8^{V32} | 7,1 (± 2,7) | 1208 (± 259) | 14 (± 0,08) |
| B8^{V33} | 50 (± 42) | 230 | 170 (± 35) |
| B8^{V34} | 13 (± 4,2) | 1069 | 68 (± 27) |
| B8^{V35} | 7,1 (± 1,6) | 513 | 23 (± 10) |
| B8^{V36} | 27 (± 16) | 891 | 130 (± 35) |
| B8^{V37} | 97 (± 31) | 582 | 334 (± 150) |
| B8^{V38} | 23 (± 13) | 1776 | 123 (± 71) |
| B8^{V39} | 16 (± 8,9) | 1290 | 200 (± 149) |
| B8^{V40} | 42 (± 29) | 1582 | 69 (± 24) |
| B8^{h1} | 4,1 (± 2,3) | 2,8 (± 1,1) | 1,9 (± 0,3) |
| C5^{neg} | NB | NB | NB |
| B6^{V1} | 2,3 (± 0,9) | NB | NB |
| B6^{V3} | 2,6 (± 1,1) | NB | NB |
| B6^{V4} | 5,9 (± 0,8) | NB | NB |
| B7 | 1,9 (± 0,8) | NB | NB |
| H7 | 2,9 (± 0,9) | NB | NB |
| E4 | 3,1 (± 2,0) | NB | NB |
| E2 | 2,5 (± 1,8) | NB | NB |
| F2a | 2,9 (± 1,8) | NB | NB |
| E8 | 3,4 (± 0,4) | NB | 2,1 (± 0,7) |
| C10a | 4,1 (± 0,9) | NB | 3,2 (± 1,0) |
| H1 | 11 (± 2,7) | NB | 3,7 (± 1,2) |
| D12 (neg ctrl) | NB | NB | NB |

| | | | |
|---|---|---|---|
| NB: no binding; ND: not determined | | | |

Binding properties of some VHHs were also evaluated using Surface Plasmon Resonance (SPR) experiments. The human, mouse and rhesus monkey TfR (having respectively GeneBank number NM_003234.2, NM_011638, NC_041755.1) ectodomains were fused to the N-terminal end of a mouse IgG1 Fc fragment and the recombinant proteins were produced and purified in-house. Interaction of VHHs with the receptors was tested using a Biacore T200 (GE Healthcare). Receptors were immobilized on an HC 1500M or a CM5 sensor chips (Xantec) either directly or on an anti-mouse IgG previously immobilized. VHHs were injected over flowcell by using the single-cycle kinetic or the multiple-cycle kinetic methods. Table 6 hereafter summarizes the binding properties of the tested VHHs. These results illustrate once again the cross-species reactivity of the VHHs, and the diversity of binding parameters.

**Table 6:**

| **VHH name** | **On-rate Kₒₙ (M⁻¹ s⁻¹)** | **Off-rate K_{off} (s⁻¹)** | **K_{D} (M)** | **K_{D} at equilibrium (M)** |
|---|---|---|---|---|
| **Human TfR** | | | | |
| C5 | 6,65E+05 | 1,79E-04 | 2,06E-10 | ND |
| B8 | 6,77E+05 | 3,06E-05 | 5,59E-11 | ND |
| B6 | 1,73E+06 | 1,21E-03 | 6,59E-10 | ND |
| H3 | 1,01E+06 | 3,30E-05 | 3,33E-11 | ND |
| C5^{V1} | 5,55E+05 | 4,87E-04 | 8,76E-10 | ND |
| C5^{V5} | 3,32E+05 | 5,09E-02 | 1,54E-07 | 1,80E-07 |
| C5^{V8} | 5,04E+05 | 5,58E-02 | 9,48E-08 | 8,43E-08 |
| C5^{V11} | 7,90E+05 | 3,11E-04 | 3,95E-10 | ND |
| C5^{V13} | 1,14E+06 | 3,92E-04 | 3,57E-10 | ND |
| C5^{V15} | 7,00E+05 | 1,76E-04 | 2,52E-10 | ND |
| C5^{V16} | 4,59E+05 | 5,77E-03 | 1,45E-08 | ND |
| C5^{V18} | 4,87E+05 | 2, 10E-02 | 4,33E-08 | 3,78E-08 |
| C5^{V19} | 4,26E+05 | 9,70E-03 | 2,23E-08 | 7,13E-09 |
| C5^{V29} | 1,17E+06 | 9,82E-04 | 8,46E-10 | ND |
| C5^{V30} | 3,19E+05 | 3,00E-02 | 9,40E-08 | 1,12E-07 |
| C5^{V31} | 3,83E+05 | 1,82E-02 | 4,75E-08 | 6,48E-08 |
| C5^{V32} | 6,92E+05 | 2,47E-02 | 3,58E-08 | 3,86E-08 |
| C5^{h9} | 5,49E+05 | 5,82E-03 | 1,06E-08 | ND |
| C5^{h18} | 4,62E+05 | 1,30E-02 | 3,09E-08 | 2,73E-08 |
| C5^{h19} | 6,24E+05 | 9,04E-03 | 1,63E-08 | 1,32E-08 |
| C5^{h20} | 8,66E+05 | 1,05E-03 | 9,85E-10 | ND |
| C5^{h22} | 1,50E+06 | 9,06E-03 | 8,96E-09 | ND |
| B8^{V1} | 1,83E+05 | 1,10E-03 | 6,04E-09 | ND |
| B8^{V3} | 6,69E+04 | 3,38E-05 | 5,05E-10 | ND |
| B8^{V12} | 1,04E+06 | 1,69E-03 | 9,84E-10 | ND |
| B8^{V16} | 1,03E+06 | 1,33E-02 | 1,28E-08 | 7,63E-08 |
| B8^{V27} | 1,01E+06 | 7,22E-03 | 7,29E-09 | 3,22E-09 |
| B8^{V29} | 6,82E+05 | 1,69E-02 | 4,40E-08 | 2,18E-08 |
| B8^{V31} | 3,64E+05 | 2,21E-02 | 6,08E-08 | 6,38E-08 |
| B8^{V32} | 4,20E+05 | 1,26E-02 | 3,01E-08 | 3,65E-08 |
| B8^{V33} | 7,10E+05 | 3,28E-02 | 4,62E-08 | 4,65E-08 |
| B8^{V34} | 7,33E+05 | 2,44E-02 | 3,33E-08 | 3,24E-08 |
| B8^{V35} | 6,98E+05 | 1,43E-02 | 2,06E-08 | 2,39E-08 |
| B8^{V36} | 5,71E+05 | 1,90E-02 | 3,33E-08 | 3,92E-08 |
| B8^{V37} | 6,99E+05 | 4,93E-02 | 7,05E-08 | 7,55E-08 |
| B8^{V38} | 5,96E+05 | 1,92E-02 | 3,22E-08 | 3,58E-08 |
| B8^{V39} | 4,48E+05 | 1,75E-02 | 3,91E-08 | 4,03E-08 |
| B8^{V40} | 6,72E+05 | 2,83E-02 | 4,21E-08 | 4,25E-08 |
| C5^{V13h20} | 7,86E+05 | 3,57E-03 | 4,55E-09 | ND |
| C5^{V16h20} | 3,60E+05 | 3,99E-02 | 1,12E-07 | 1,15E-07 |
| B8^{h1} | 4,44E+05 | 9,49E-05 | 2,12E-10 | ND |
| B7 | 1,16E+06 | 7,58E-04 | 6,52E-10 | ND |
| H7 | 1,13E+06 | 1,47E-03 | 1,30E-09 | ND |
| E4 | 1,51E+06 | 1,89E-03 | 1,25E-09 | ND |
| C3 | 5,90E+06 | 2,49E-03 | 5,59E-10 | ND |
| F2a | 1,39E+06 | 7,52E-04 | 5,45E-10 | ND |
| C10a | 1,88E+05 | 7,26E-03 | 4,46E-08 | 8,68E-08 |
| H1 | 1,08E+05 | 5,54E-03 | 5,77E-08 | 2,24E-07 |

| **Mouse TfR** | | | | |
|---|---|---|---|---|
| C5 | 9,15E+05 | 4,47E-02 | 8,85E-08 | 8,82E-08 |
| B8 | 1,23E+06 | 2,43E-02 | 2,70E-08 | 3,75E-08 |
| H3 | 5,81E+06 | 9,09E-03 | 1,56E-08 | ND |
| C5^{V1} | 5,46E+05 | 1,20E-01 | 2,44E-07 | 2,28E-07 |
| C5^{V2} | 5,83E+05 | 5,79E-02 | 1,09E-07 | 1,08E-07 |
| C5^{V3} | 6,47E+05 | 4,67E-02 | 8,35E-08 | 9,88E-08 |
| C5^{V4} | 6,52E+05 | 5,32E-02 | 8,97E-08 | 9,18E-08 |
| C5^{V9} | 5,24E+05 | 1,97E-02 | 4,08E-08 | 4,05E-08 |
| C5^{V11} | 3,55E+05 | 2,18E-02 | 6,13E-08 | 7,47E-08 |
| C5^{V13} | 2,31E+05 | 1,82E-02 | 7,89E-08 | 1,13E-07 |
| C5^{V15} | 7,97E+05 | 5,63E-02 | 7,06E-08 | 6,11 E-08 |
| C5^{V27} | 4,93E+05 | 1,02E-01 | 2,07E-07 | 3,79E-07 |
| C5^{V29} | 4,72E+05 | 6,75E-02 | 1,43E-07 | 2,40E-07 |
| C5^{h18} | 8,16E+05 | 2,35E-01 | 3,36E-07 | 3,00E-07 |
| C5^{h19} | 1,28E+04 | 8,25E-03 | 6,43E-07 | 2,75E-06 |
| B8^{V1} | 6,51E+03 | 1,25E-02 | 1,96E-06 | ND |
| B8^{V3} | 1,78E+05 | 3,43E-02 | 1,93E-07 | 2,06E-07 |
| C5^{V16h20} | 2,51E+05 | 7,50E-02 | 3,01E-07 | ND |

| **Rhesus monkey TfR** | | | | |
|---|---|---|---|---|
| C5 | 1,55E+06 | 7,74E-03 | 5,35E-09 | 1,31E-08 |
| B8 | 1,00E+06 | 9,54E-05 | 9,51E-11 | ND |
| C5^{V27} | 3,32E+05 | 2,78E-02 | 8,39E-08 | ND |
| C5^{V30} | 5,15E+05 | 1,92E-01 | 3,73E-07 | 3,39E-07 |
| C5^{V31} | 5,24E+05 | 1,23E-01 | 2,34E-07 | 2,42E-07 |
| C5^{V32} | 7,45E+05 | 2,66E-01 | 3,58E-07 | 2,65E-07 |
| B8^{V12} | 9,97E+05 | 1,19E-02 | 1,21E-08 | 2,51E-08 |
| B8^{V16} | 3,25E+05 | 4,38E-02 | 1,35E-07 | 2,22E-07 |
| B8^{V27} | 4,55E+05 | 2,06E-02 | 5,38E-08 | 3,68E-08 |
| B8^{V29} | 1,16E+06 | 9,42E-02 | 2,02E-07 | 1,00E-07 |
| B8^{V31} | 1,59E+06 | 5,09E-02 | 3,21E-08 | 3,54E-08 |
| B8^{V32} | 1,28E+06 | 4,18E-02 | 3,27E-08 | 3,51E-08 |
| B8^{V33} | 6,93E+05 | 5,09E-02 | 7,34E-08 | 8,60E-08 |
| B8^{V34} | 1,97E+06 | 1,02E-01 | 5,15E-08 | 4,94E-08 |
| B8^{V35} | 8,98E+05 | 3,03E-02 | 3,37E-08 | 3,44E-08 |
| B8^{V36} | ND | ND | ND | 2,06E-07 |
| B8^{V37} | 7,00E+05 | 1,03E-01 | 1,48E-07 | 1,75E-07 |
| B8^{V38} | 5,49E+05 | 2,55E-02 | 4,63E-08 | 5,54E-08 |
| B8^{V39} | 3,62E+05 | 3,27E-02 | 9,05E-08 | 9,33E-08 |
| B8^{V40} | 5,75E+05 | 2,00E-02 | 3,48E-08 | 3,98E-08 |
| C10a | 9,03E+05 | 1,78E-02 | 2,46E-08 | 3,81E-08 |
| H1 | 6,62E+05 | 1,09E-02 | 2,22E-08 | 2,78E-08 |

| | | | | |
|---|---|---|---|---|
| ND: not determined | | | | |

### EXAMPLE 2: Oligonucleotide sequences and their conjugation to TfR-binding VHH.

The *in vitro* and *in vivo* experiments described in the examples provided herein were performed using chemically modified siRNA or gapmer ASO sequences listed in Table 7 below. The RNAi-competent siRNA sequences used in the following examples target the ubiquitous superoxide dismutase 1 (SOD1) mRNA of either the mouse and rat (siSOD1m), described in WO2019217459, or the human and non-human primate (NHP, including *Papio anubis, Macaca mulatto, Macaca fascicularis*), which was adapted from siSOD1m to match *SOD1* mRNA of human and NHP origin (siSOD1h). The single-stranded gapmer, rNase H-competent antisense oligonucleotide (ASO) used in the following examples targets the ubiquitously expressed long non-coding RNA (lncRNA) MALAT-1, which is preferentially enriched in nuclear speckles where it regulates post-transcriptional RNA processing, adapted from Tripathi et al. by introduction, in a sequence of SEQ ID NO: 405 of Table 7 below, of a modification 'm*c*'( i.e., 2'-O-methoxyethyl-5-methyl-cytidine ; 2'MOE meC)' instead of 2'-MOE-C (i.e., 2'-O-methoxyethyl-cytidine).

**Table 7:**

| **SEQ ID NO** | **Oligo ID** | **Strand (5'-3')** | **Sequence** |
|---|---|---|---|
| SEQ465 | siSODlm (mouse/rat) | S | c•a•uuuu*A*a*UCC*ucacucuaaaL |
| SEQ466 | | AS | u•*U•*uagAg*UG*agga*U*u*A*aaaug•a•g |
| SEQ465 | siSOD1m-5'VP (mouse/rat) | S | c•a•uuuuAaUCCucacucuaaaL |
| (VP)-SEQ466 | | AS | (VP)-u•*U•*uagAg*UG*agga*U*u*A*aaaug•a•g |
| SEQ467 | siSOD1h (hum/NHP) | S | c•a•cuuuAa*UCC*ucuauccagaL |
| SEQ468 | | AS | u•C•ugg*A*u*AG*agga*U*u*A*aagug•a•g |
| SEQ405 | *hMALAT1*-ASO | AS | L*g*•*g•*g*•a*•*g*•dT•dT•dA•dC•dT•dT•dG•dC•dC•dA•*a*•m*c*•*t*•*t*•*g* |

siSOD1m: siRNA duplex targeting the mouse and rat superoxide dismutase 1 (SOD1) mRNA; siSOD1h: siRNA duplex targeting the human and non-human primate (NHP, including *Papio anubis, Macaca mulatta, Macaca fascicularis*) superoxide dismutase 1 mRNA; *hMALAT1-*ASO: gapmer antisense oligonucleotide (ASO) targeting the human MALAT-1 long non-coding RNA; lower case indicates 2'-O-methyl (2'-OMe) sugar modifications, respectively, to adenosine, cytidine, guanosine or uridine nucleotides; italicized upper case indicates 2'-deoxy-2'-fluoro (2'F) sugar modifications, respectively, to adenosine, cytidine, guanosine or uridine nucleotides; • indicates phosphorothioate (PS) internucleosidic linkage; VP indicates vinyl-phosphonate; L indicates linker (conjugation handle); dN indicates deoxynucleotides; italicized lower case indicates 2'-O-methoxyethyl (2'-MOE) sugar modifications to adenosine, guanosine or thymidine nucleotides; mc indicates 2'-O-methoxyethyl-5-methyl-cytidine (2'MOE meC).

Oligonucleotides were purchased from Horizon Discovery Biosciences Ltd. or GeneLink Inc. with conjugation handles, such as hexylamino-linker, introduced on the 3'-end of the sense strand of siRNA duplexes or on the 5'-end of gapmer ASOs. The general conjugation strategy involved a convergent synthesis such as the strategy described in WO 2020/144233, consisting of the parallel modification of: i) the VHH or the VHH-hFc heterodimer to site-specifically introduce for example an azido-linker; and ii) the oligonucleotide to introduce for example a constrained alkyne group complementary to the azido functional group. In a final step, both functionalized azido-VHH, or VHH-hFc-azido, and alkyne-Oligo precursors are linked to each other using preferably a copper-free click reaction, yielding VHH-Oligo or heterodimeric VHH-hFc-Oligo conjugates with a stable linker (cf. Figure 14). Alternative methods were employed by inventors to generate VHH-Oligo conjugates with either other types of linkers such as cleavable disulfide linkers or longer PEG linkers or another conjugation chemistry such as thiol-maleimide chemistry (cf. Figure 13).

### EXAMPLE 3: Evaluation of the binding of VHH-Oligo conjugates to mTfR, rhTfR and hTfR in living cells.

The binding of VHH-Oligo conjugates to mTfR, rhTfR and hTfR was evaluated using a competition assay on living cells expressing the receptor of interest, namely murine neuroblastoma Neuro-2a cells (N2a), CHO cells genetically engineered by the inventors to stably express the macaque rhesus TfR fused to the eGFP protein in C-terminal (CHO-rhTfR-GFP cells), or human breast ductal carcinoma MCF-7 cells, respectively. MCF-7 and N2a cells were grown in Dulbecco's Modified Eagle Medium (DMEM) GlutaMAX supplemented with 10% v/v FBS in 5% CO₂ at 37°C. CHO-rhTfR-GFP cells were grown in Ham F12 GlutaMAX supplemented with 10% v/v FBS in 5% CO₂ at 37°C. Cells were seeded two days before experiments in 96-well plates at a density of 50.000 cells/well. On the day of the experiment, free VHHs or VHH-Oligo conjugates diluted at various concentrations in DMEM supplemented with 1% bovine serum albumin (BSA) (MCF-7 and N2a cells) or in Ham F12 supplemented with 1% BSA (CHO-rhTfR-GFP cells) were co-incubated at various concentrations on each cell line during 3 hrs at 37°C with a subsaturating concentration of the fluorescent reference compound C5-Alexa680 (100 nM on N2a and CHO-rhTfR-GFP cell lines, 10 nM on MCF-7 cells). After treatment, cells were washed with D-PBS and dissociated by incubating with Trypsin/EDTA for 5 mins at 37°C, before the addition of cold culture media (4°C) to inhibit Trypsin activity. Resuspended cells were transferred in a 96-deepwell plate (V-bottom) containing 1% fetal bovine serum (FBS) / 0.02% sodium azide in phosphate-buffered saline (PBS) and then centrifuged for 5 min at 2000 rpm at 4°C. After removing the supernatant, 5 mM EDTA in D-PBS and 4% paraformaldehyde (PFA) (v/v 1:1) were added into wells to fix cells for 15 min at room temperature. PFA was removed by centrifugation (5 min, 3000 rpm, 4°C) and cells were resuspended with 5 mM EDTA in D-PBS. Then the cellular A680-associated fluorescence signal was quantified using an Attune^{™} NxT flow cytometer (Thermo Fisher Scientific) equipped with Attune^{™} NxT v3.1.2. Experimental data (experimental triplicates) were fitted with GraphPad Prism^{®} software using a nonlinear fit to determine the apparent *K*ᵢ inhibition constants. Graphs of Figure 1 show representative inhibition curves and tables show means ± SD of at least 2 independent experiments.

All tested VHH-Oligo conjugates (e.g., B8-siSOD1m, C5-siSOD1m, B8-siSOD1h, C5-siSOD1h, B8-MALAT1-ASO) induced a concentration-dependent inhibition of the binding and uptake of the reference compound in cells expressing the human, macaque rhesus and murine TfR, with similar or lower *K*ᵢ values to that of free VHHs, demonstrating efficient and cross-species binding to the TfR. (Figure 1).

### EXAMPLE 4: In vitro functional delivery and gene silencing potential of TfR-binding VHH-Oligo conjugates after free uptake in human and murine cell lines.

Human cell lines MCF-7 (breast ductal carcinoma cells), Igrov-1 (ovarian cancer cells) and MIA PaCa-2 cells (pancreatic cancer cells) and the murine neuroblastoma cell line Neuro-2a were grown in DMEM GlutaMAX supplemented with 10% v/v FBS in 5% CO₂ at 37°C. For free uptake experiments, cells were seeded in 96-well plates at a density of 2.000 cells/well. Twenty-four hours later, the medium was removed, and cells were further incubated during 3 days in DMEM supplemented with 1% v/v FBS containing various concentrations of free or conjugated oligos. Cells were then washed with D-PBS and harvested in lysis buffer from either the SuperScript^{™} IV CellsDirect^{™} cDNA Synthesis Kit (Invitrogen), for human cell lines, or the Nucleospin RNA XS Kit (Macherey-Nagel), for N2a cells, according to the manufacturer's recommendations. For experiments performed with the Nucleospin RNA XS Kit, cell lysates were stored at -20°C before RNA extraction, and reverse-transcription was performed using the High-Capacity RNA-to-cDNA^{™} Kit (Applied Biosystems). Relative RNA expression levels were quantified by RT-qPCR using the TaqMan^{™} Fast Universal PCR Master Mix (2X) no AmpErase^{™} UNG kit (Applied Biosystems) and commercially available TaqMan^{™} probes for murine SOD1, human SOD1 or human MALAT-1 genes (Applied Biosystems). Expression data were analyzed using the DDCq method normalized to the expression of reference RpL 13 or GAPDH genes, based on raw quantification cycle (Cq) values (Bustin et al.) and are presented as mRNA levels relative to untreated cells. Experimental data (experimental triplicates) are presented as means ± standard deviation (SD) and were analysed with GraphPad Prism^{®} software using a three-parameter log(inhibitor) vs. response nonlinear regression to determine the IC50. Figure 2 (human cell lines) and Figure 3 (mouse N2a cells) show representative inhibition curves and estimated IC50 values.

Whereas the free siSOD1h or the siSOD1h conjugated to the non-binding C5neg VHH showed no effect on *hSOD1* mRNA levels, TfR-binding VHH-siSOD1h conjugates (such as C5-siSOD1h, C5V8-siSOD1h or B8-siSOD1h, respectively comprising the following VHH molecules: C5, C5V8 or B8) all showed potent and concentration-dependent hSOD1 mRNA downregulation on various human cell lines (Figure 2A and Table 1). A similar potent knock-down effect was observed after free uptake of the VHH-MALATI-ASO conjugate on MCF-7 cells (Figure 2B). A similar specific and potent knock-down effect was observed with various VHH-siSOD1m conjugates (i.e., conjugates comprising the following VHH molecules which are B8 or C5 or their variants such as B8h1, C5, C5V1, C5V7, C5V8, C5V13, C5h18, C5h19, as listed in Table 1) as well as the VHH-MALAT1-ASO conjugate (comprising a VHH molecule such as B8 as described in Table 1) after free uptake on the murine N2a cell line (Figures 3A and 3B respectively).

These results illustrate that the VHH-Oligo conjugates of the invention efficiently bind to cell surface human or murine TfR followed by TfR-mediated endocytosis, endosomal escape and cytosol or nuclear delivery of active oligonucleotides, thereby allowing effective modulation of target gene expression using different oligonucleotide modalities.

### EXAMPLE 5: In vivo functional delivery and mRNA knock-down in muscle tissues of TfR-binding VHH-siRNA conjugates in wild-type mice.

VHH-siSOD 1m conjugates were assessed for their potential to mediate murine *SOD1* mRNA knock-down in muscle tissues following systemic administration in wild-type C57Bl/6 mice. Mice (4-8 per group) were dosed via either intravenous (IV bolus) or subcutaneous (SC) injection of PBS vehicle (control), unconjugated siSODlm or the indicated VHH-siSOD1m conjugates at the doses indicated in Figures 4-7. At indicated times post-injection, tissue samples were withdrawn, snap-frozen in 10 vol. of NucleoProtect RNA stabilization reagent (Macherey-Nagel) and stored at -20°C. Frozen tissue samples were homogenized in QUIAzol lysis reagent using a Precellys Evolution tissue homogenizer equipped with a Cryolys Evolution cooling system (Bertin Instruments). Total RNA was extracted from tissue homogenates using the rNeasy 96 QIAcube HT Kit (QIAGEN) in a QIAcube HT system. RNA samples were analyzed and dosed using a Fragment Analyzer RNA kit in a Fragment Analyzer system (Agilent). Relative RNA expression levels were quantified by RT-qPCR using the TaqMan^{™} Fast Universal PCR Master Mix (2X) no AmpErase^{™} UNG kit (Applied Biosystems) and commercially available TaqMan^{™} probes for murine SOD1 and RpL13 genes (Applied Biosystems). Expression data were analyzed using the DDCq method normalized to the expression of the reference RpL13 gene, based on raw quantification cycle (Cq) values (Bustin et al.). The results are expressed as means ± standard error of the mean (SEM) and are presented as mRNA levels relative to the control animals injected with PBS. When administered by either IV or SC route of administration in wild-type mice at the low dose of 3 mg/kg (siRNA molar equivalent), TfR-binding VHH-siSOD1m conjugates showed from about 70 % to about 80% downregulation of the target *SOD1* mRNA levels in the gastrocnemius muscle and about 60% in cardiac muscle (Figure 4). No or very little effect was observed in the liver or lung (Figure 4) and no effect was observed in any tissue with the non-binding C5neg-siSOD1m conjugate (Figure 5). The results presented in this example demonstrate tissue selectivity and confirm TfR-dependent functional delivery of these TfR-binding conjugates *in vivo* at low therapeutic dose. A dose-response study with one of these conjugates showed sub-maximal 80% knock-down effect at the dose of 3 mg/kg in skeletal muscle, and at the dose of 15 mg/kg in cardiac muscle, with no effect of unconjugated siRNA up to 15 mg/kg (Figure 6). The ED50 of this knock-down effect was about 0.6 mg/kg (siRNA molar equivalent) in the gastrocnemius muscle and about 3 mg/kg (siRNA molar equivalent) in the cardiac muscle. Time-course evaluation of these conjugates showed that a single administration of TfR-binding VHH-siRNA conjugates at the low dose of 3 mg/kg is sufficient to trigger potent and durable knock-down effect, for more than one month (Figure 7). This demonstrates that early TfR-mediated uptake in muscle tissues allows efficient intracellular delivery of VHH-siRNA conjugates, with sustained release of active siRNA from endo-lysosomal compartments to the cytosol, RNA-induced silencing complex (RISC)-loading and continuous degradation of target mRNA molecules.

### EXAMPLE 6: Generation and evaluation of the functional delivery and mRNA knock-down potential in vitro and in vivo of TfR-binding VHH-hFc-siRNA conjugates.

To introduce a half-life extending moiety or stabilizing moiety with improved pharmacokinetics profile, TfR-binding VHHs can be conjugated to oligos via an antibody or antibody fragment scaffold. This strategy is exemplified here using VHH-hFc-siRNA conjugates that were generated using the same general conjugation strategy as described for direct VHH-siRNA conjugates. In addition, the VHH-hFc-siRNA conjugates further comprise a human IgG1-derived Fc dimer (hFc) with two arms called "knob" and "hole". In this example, various TfR-binding VHH-hFc-siRNA heterodimeric conjugates were generated using the "knob-into-hole" technology. Each of these VHH-hFc-siRNA comprise a selected VHH at the N-terminus of "knob" arm of the Fc dimer and a tag sequence specifically recognized by transglutaminase (TGase) enzyme (namely a Q-tag) inserted in C-terminus of the "hole" arm of the Fc dimer. The VHH-hFc-siRNA heterodimers were site-specifically modified on the Q-tag to introduce an azido-linker. The resulting heterodimeric VHH-hFc-Q-tag-azido intermediates were conjugated to alkyne-siRNA using copper-free click reaction, yielding VHH-hFc-siRNA conjugates with a stable linker (Figure 14). More particularly, VHH-hFc-siSOD1m and VHH-hFc-siSOD1h conjugates were generated using the same siSOD1m and siSOD1h sequences, respectively, as described in previous examples and assessed for their biological properties in different systems. The VHH used for the production of the VHH-hFc-siRNA conjugates were C5 and B8 variants (shown in Figures 8, 9 and 10). These conjugates were produced using a modified IgG1-derived Fc dimer having a sequence of SEQ ID NO: 664 on the "knob" arm, and a sequence of SEQ ID NO: 665 on the "hole" arm. The linker used in the "knob" arm between the VHH and hFc was GGGGSGGGGS (SEQ ID NO: 630). For example, for the production of the C5-Fc conjugates, the whole amino acid sequence used for the "knob" arm is shown in SEQ ID NO: 666, and the whole amino acid sequence used for the "hole" arm is shown in SEQ ID NO: 667. These sequences of the "knob" and "hole" arms are encoded respectively by SEQ ID NOs: 668 or 669.

First, VHH-hFc-siSOD1 conjugates demonstrated cross-species and similar or slightly higher binding to mTfR and hTfR as compared to free VHHs (Figure 8). Second, using the same *in vitro* free uptake assay as described in previous examples on either human breast cancer MCF-7 cells (Figure 9A) or murine neuroblastoma Neuro-2a cells (Figure 9B), VHH-hFc-siSOD1 conjugates showed specific, potent and concentration-dependent *SOD1* mRNA downregulation. Third, the ability of VHH-hFc-siSOD1m conjugates to mediate murine *SOD1* mRNA knock-down in muscle tissues following intravenous (IV bolus) administration in wild-type C57Bl/6 mice was shown. Mice (4-8 per group) were dosed via single intravenous (IV bolus) injection of PBS vehicle (control), non-binding (C5neg) or TfR-binding (C5) VHH-hFc-siSOD1m conjugates at 0.5 or 1.5 mg/kg (siRNA molar equivalent). Fourteen days following administration, tissue samples were withdrawn, snap-frozen in 10 vol. of NucleoProtect RNA stabilization reagent (Macherey-Nagel) and stored at -20°C. Frozen tissue samples were homogenized in QUIAzol lysis reagent using a Precellys Evolution tissue homogenizer equipped with a Cryolys Evolution cooling system (Bertin Instruments). Total RNA was extracted from tissue homogenates using the RNeasy 96 QIAcube HT Kit (QIAGEN) in a QIAcube HT system. RNA samples were analyzed and dosed using a Fragment Analyzer RNA kit in a Fragment Analyzer system (Agilent). Relative RNA expression levels were quantified by RT-qPCR using the TaqMan^{™} Fast Universal PCR Master Mix (2X) no AmpErase^{™} UNG kit (Applied Biosystems) and commercially available TaqMan^{™} probes for murine SOD1 and RpL13 genes (Applied Biosystems). Expression data were analyzed using the DDCq method normalized to the expression of the reference RpL13 gene, based on raw quantification cycle (Cq) values (Bustin et al.). The results are expressed as means ± standard error of the mean (SEM) and are presented as mRNA levels relative to the control animals injected with PBS.

While no or little downregulation of murine *SOD1* mRNA levels was observed in any tissue analysed after treatment with the non-binding conjugate (C5neg-hFc-siSOD1m), treatment with the TfR-binding conjugate (C5-hFc-siSOD1m) induced potent, muscle tissue-selective and specific effect, with more than 70% knock-down in gastrocnemius, diaphragm or cardiac muscles at the dose of 1.5 mg/kg and similar or slightly lower effect at 0.5 mg/kg, while no effect in the liver or lungs (Figure 10A). Notably, the ED50 of the C5-hFc-siSOD1m conjugate administered as a single IV bolus was about 0.16 mg/kg (siRNA molar equivalent) in the gastrocnemius muscle and about 0.4 mg/kg (siRNA molar equivalent) in the cardiac muscle, namely 4-fold and 8-fold lower than ED50 values obtained after single SC administration of the direct C5-siSOD1m conjugate (Figure 6), showing an improvement with the introduction of the half-life extending Fc backbone. Similarly, another conjugate comprising an additional 5'-VP on the siSOD1m antisense strand (C5-hFc-siSOD1m-5'VP) was administered as a single SC at various doses in wild-type mice. Potent (more than 80%) and muscle-selective KD was observed, with ED50 between 0.1 and 0.6 mg/kg (siRNA molar equivalent). The results presented in this example demonstrate tissue selectivity and TfR-dependent functional delivery of these TfR-binding VHH-hFc-siRNA conjugate designs when administered as a single dose by either IV or SC to wild-type mice at low therapeutic dose.

### EXAMPLE 7: In vivo functional delivery and mRNA knock-down in muscle tissues of TfR-binding VHH-siRNA conjugates in hTfR1^{+/+} mice.

VHH-siSOD1m conjugates were assessed for their ability to mediate murine *SOD1* mRNA knock-down in muscle tissues of transgenic mice constitutively expressing the human TfR1 (B-hTfR1 mice, Biocytogen). PBS vehicle (control), unconjugated siSOD1m or VHH-siSOD1m conjugates comprising either a cross-species rodent/NHP/human TfR1-binding VHH (C5) or a VHH binding to only the human TfR1 (B6) were administered as a single subcutaneous (SC) injection in *hTfR1*^{+/+} mice (3-4 per group) at the dose of 3 mg/kg (siRNA molar equivalent). Seven days following administration, tissue samples were withdrawn, snap-frozen in 10 vol. of NucleoProtect RNA stabilization reagent (Macherey-Nagel) and stored at -20°C. Frozen tissue samples were homogenized in QUIAzol lysis reagent using a Precellys Evolution tissue homogenizer equipped with a Cryolys Evolution cooling system (Bertin Instruments). Total RNA was extracted from tissue homogenates using the RNeasy 96 QIAcube HT Kit (QIAGEN) in a QIAcube HT system. RNA samples were analyzed and dosed using a Fragment Analyzer RNA kit in a Fragment Analyzer system (Agilent). Relative RNA expression levels were quantified by RT-qPCR using the TaqMan^{™} Fast Universal PCR Master Mix (2X) no AmpErase^{™} UNG kit (Applied Biosystems) and commercially available TaqMan^{™} probes for murine SOD1 and RpL13 genes (Applied Biosystems). Expression data were analyzed using the DDCq method normalized to the expression of the reference RpL13 gene, based on raw quantification cycle (Cq) values (Bustin et al.). The results are expressed as means ± standard error of the mean (SEM) and are presented as mRNA levels relative to the control animals injected with PBS.

While no downregulation of murine *SOD1* mRNA levels was observed in any of the tested samples analysed after treatment with the unconjugated siSOD1m, both hTfR-binding conjugates induced similar potent and muscle tissue-selective effect, with about 70-80% knock-down in gastrocnemius and diaphragm, and about 40-50% knock-down in cardiac muscle, while no effect in the liver or lungs, after a single subcutaneous injection in *hTfR1*^{+/+} mice at the dose of 3 mg/kg (siRNA molar equivalent). (Figure 11). The results presented in this example demonstrate cross-species rodent/human ability of the TfR-binding VHH-siRNA conjugates to undergo TfR-dependent binding, functional uptake and target mRNA downregulation *in vivo* at low therapeutic dose in muscle tissues of mice expressing the human TfR.

### EXAMPLE 8: In vivo functional delivery and mRNA knock-down in muscle tissues of TfR-binding VHH-siRNA conjugates in non-human primates.

VHH-siSOD1h conjugates targeting the human and non-human primate (NHP) *SOD1* gene were assessed for their ability to mediate *SOD1* mRNA knock-down in muscle tissues of olive baboons (*Papio Anubis*). PBS vehicle (control) or a TfR-binding VHH-siSOD 1h conjugate was administered as a single intravenous (IV infusion, 30 min) in olive baboons (males, 10-17-month-old, 2.6-4.5kg) at the dose of 5 mg/kg (siRNA molar equivalent). At indicated times following administration, approximately 100 mg samples of gastrocnemius, quadriceps and tibialis anterior muscle were withdrawn under general anesthesia, snap-frozen in 10 vol. ofNucleoProtect RNA stabilization reagent (Macherey-Nagel) and stored at -20°C. Frozen tissue samples were homogenized in QUIAzol lysis reagent using a Precellys Evolution tissue homogenizer equipped with a Cryolys Evolution cooling system (Bertin Instruments). Total RNA was extracted from tissue homogenates using the RNeasy 96 QIAcube HT Kit (QIAGEN) in a QIAcube HT system. RNA samples were analyzed and dosed using a Fragment Analyzer RNA kit in a Fragment Analyzer system (Agilent). Relative RNA expression levels were quantified by RT-qPCR using the TaqMan^{™} Fast Universal PCR Master Mix (2X) no AmpErase^{™} UNG kit (Applied Biosystems) and commercially available TaqMan^{™} probes for murine SOD1 and RpL13 genes (Applied Biosystems). Expression data were analyzed using the DDCq method normalized to the expression of the reference RpL13 gene, based on raw quantification cycle (Cq) values (Bustin et al.). The results are expressed as means ± standard error of the mean (SEM) and are presented as mRNA levels relative to the control animals injected with PBS. When compared to the control group injected with PBS, a single IV administration of TfR-binding VHH-siSOD1h conjugate induced a potent and durable downregulation of the target *SOD1* mRNA levels in all muscle tissues tested, with 60% knock-down observed for more than three months (Figure 12). The results presented in this example confirm cross-species rodent/NHP/human ability of the TfR-binding VHH-siRNA conjugates to undergo TfR-dependent binding, functional uptake and target mRNA downregulation *in vivo* at low therapeutic dose in muscle tissues of non-human primates.

### LIST OF SEQUENCES

**Table 1 : List of amino acid sequences of the VHH molecules of the invention as well as of negative controls C5^{neg} and D12, the corresponding CDR1-3, and their identifiers, i.e. SEQ ID NO or SEQ.**

| ***VHH*** | **Amino acid sequence** | **CDR1** | **CDR2** | **CDR3** |
|---|---|---|---|---|
| C5 | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ4 (x=1) or SEQ213 (x=0) | GTDFSI NF SEQ 1 | FTAT GNT SEQ 2 | YMLDK SEQ 3 |
| B8 | EVQLVESGGGVVQPGGSLRLSCAASGEIFSINFMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ8 (x=1) or SEQ214 (x=0) | GEIFSIN F SEQ5 | FTRD GST SEQ6 | YMLDT SEQ7 |
| B6 | EVQLVESGGGLVQPGGSLRLSCTASGGPIEQYPMGWFRRAPGKERELVAS ISRSGDGTYYAISSVKGRFTISRDNAENTVFLQMNSLKPDDTAVYYCGAGI NPTKIWGQGTQVTVSS(AAAEQKLISEEDLNGAAHHHHHHGS)x, with x=1 or 0 SEQ12 (x=1) or SEQ215 (x=0) | GGPIEQ YP SEQ9 | ISRSG DGTY SEQ10 | GAGINP TKI SEQ11 |
| H3 | EVQLVESGGGEVQPGGSLKLSCVASGTDFSINFVRWYRQRPGKQREWVA GFTANGDTNYPDSMKGRFTISRDNAKNTVYLQINSLKSEDTAVYYCYML DNWGQGTQVTVSS(AAAEQKLISEEDLNGAAHHHHHHGS)x SEQ16 (x=1) or SEQ216 (x=0) | GTDFSI NF SEQ13 | FTAN GDT SEQ14 | YMLDN SEQ 15 |
| C5^{v1} | EVQLVESGGGVVQPGGSLKLSCVASGADFSINFIRWYRQAPGKQREFVAG FTATGNTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ18 (x=1) or SEQ217 (x=0) | GADFSI NF SEQ17 | FTAT GNT SEQ2 | YMLDK SEQ3 |
| C5^{v2} | EVQLVESGGGVVQPGGSLKLSCVASGTAFSINFIRWYRQAPGKQREFVAG FTATGNTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ20 (x=1) or SEQ218 (x=0) | GTAFSI NF SEQ19 | FTAT GNT SEQ2 | YMLDK SEQ3 |
| C5^{v3} | EVQLVESGGGVVQPGGSLKLSCVASGTDFSINFIRWYRQAPGKQREFVAG FTAAGNTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ22 (x=1) or SEQ219 (x=0) | GTDFSI NF SEQ1 | FTAA GNT SEQ21 | YMLDK SEQ3 |
| C5^{v4} | EVQLVESGGGVVQPGGSLKLSCVASGTDFSINFIRWYRQAPGKQREFVAG FTATGATNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ24 (x=1) or SEQ220 (x=0) | GTDFSI NF SEQ1 | FTAT GAT SEQ23 | YMLDK SEQ3 |
| C5^{v5} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCAMLD KWGQGTQVTVSS(AAAEQKLISEEDLNGAAHHHHHHGS)x, with x=1 or 0 SEQ26 (x=1) or SEQ221 (x=0) | GTDFSI NF SEQ1 | FTAT GNT SEQ2 | AMLDK SEQ25 |
| C5^{v6} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYALD KWGQGTQVTVSS(AAAEQKLISEEDLNGAAHHHHHHGS)x, with x=1 or 0 SEQ28 (x=1) or SEQ222 (x=0) | GTDFSI NF SEQ1 | FTAT GNT SEQ2 | YALDK SEQ27 |
| C5^{v7} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYMAD KWGQGTQVTVSS(AAAEQKLISEEDLNGAAHHHHHHGS)x, with x=1 or 0 SEQ30 (x=1) or SEQ223 (x=0) | GTDFSI NF SEQ1 | FTAT GNT SEQ2 | YMADK SEQ29 |
| C5^{v8} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYMLA KWGQGTQVTVSS(AAAEQKLISEEDLNGAAHHHHHHGS)x, with x=1 or 0 SEQ32 (x=1) or SEQ224 (x=0) | GTDFSI NF SEQ1 | FTAT GNT SEQ2 | YMLAK SEQ31 |
| C5^{v9} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYMLD AWGQGTQVTVSS(AAAEQKLISEEDLNGAAHHHHHHGS)x, with x=1 or 0 SEQ34 (x=1) or SEQ225 (x=0) | GTDFSI NF SEQ1 | FTAT GNT SEQ2 | YMLDA SEQ33 |
| C5^{v10} | EVQLVESGGGVVQPGGSLKLSCVASGTDFSLNFIRWYRQAPGKQREFVA GFTATGNTNYADSMKGRFTISRDNTKNAVYLQIDSLKPEDTAVYYCYML DKWGQGTQVTVSS(AAAEQKLISEEDLNGAAHHHHHHGS)x, with x=1 or 0 SEQ68 (x=1) or SEQ226 (x=0) | GTDFSL NF SEQ67 | FTAT GNT SEQ2 | YMLDK SEQ3 |
| C5^{v11} | EVQLVESGGGVVQPGGSLKLSCVASGTDFSINYIRWYRQAPGKQREFVA GFTATGNTNYADSMKGRFTISRDNTKNAVYLQIDSLKPEDTAVYYCYML DKWGQGTQVTVSS(AAAEQKLISEEDLNGAAHHHHHHGS)x, with x=1 or 0 SEQ70 (x=1) or SEQ227 (x=0) | GTDFSI NY SEQ69 | FTAT GNT SEQ2 | YMLDK SEQ3 |
| C5^{v12} | EVQLVESGGGVVQPGGSLKLSCVASGTDFSINFIRWYRQAPGKQREFVAG ITATGNTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ72 (x=1) or SEQ228 (x=0) | GTDFSI NF SEQ1 | ITATG NT SEQ71 | YMLDK SEQ3 |
| C5^{v13} | EVQLVESGGGVVQPGGSLKLSCVASGTDFSINFIRWYRQAPGKQREFVAG FSATGNTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ74 (x=1) or SEQ229 (x=0) | GTDFSI NF SEQ1 | FSAT GNT SEQ73 | YMLDK SEQ3 |
| C5^{v14} | EVQLVESGGGVVQPGGSLKLSCVASGTDFSINFIRWYRQAPGKQREFVAG FTATGNSNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ76 (x=1) or SEQ230 (x=0) | GTDFSI NF SEQ1 | FTAT GNS SEQ75 | YMLDK SEQ3 |
| C5^{v15} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCFMLD KWGQGTQVTVSS(AAAEQKLISEEDLNGAAHHHHHHGS)x, with x=1 or 0 SEQ78 (x=1) or SEQ231 (x=0) | GTDFSI NF SEQ1 | FTAT GNT SEQ2 | FMLDK SEQ77 |
| C5^{v16} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYILDK WGQGTQVTVSS(AAAEQKLISEEDLNGAAHHHHHHGS)x, with x=1 or 0 SEQ80 (x=1) or SEQ232 (x=0) | GTDFSI NF SEQ1 | FTAT GNT SEQ2 | YILDK SEQ79 |
| C5^{v17} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYMID KWGQGTQVTVSS(AAAEQKLISEEDLNGAAHHHHHHGS)x, with x=1 or 0 SEQ82 (x=1) or SEQ233 (x=0) | GTDFSI NF SEQ1 | FTAT GNT SEQ2 | YMIDK SEQ81 |
| C5^{v18} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYMVD KWGQGTQVTVSS(AAAEQKLISEEDLNGAAHHHHHHGS)x, with x=1 or 0 SEQ84 (x=1) or SEQ234 (x=0) | GTDFSI NF SEQ1 | FTAT GNT SEQ2 | YMVDK SEQ83 |
| C5^{v19} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYMLE KWGQGTQVTVSS(AAAEQKLISEEDLNGAAHHHHHHGS)x, with x=1 or 0 SEQ86 (x=1) or SEQ235 (x=0) | GTDFSI NF SEQ1 | FTAT GNT SEQ2 | YMI,EK SEQ85 |
| C5^{h1} | EVQLVESGGGVVQPGGSLRLSCAASGTDFSINFMSWVRQAPGKGLEWVA GFTATGNTNYADSVKGRFTISRDNAKNTLYLQMNSLRPEDTAVYYCYML DKWGQGTQVTVSS(AAAEQKLISEEDLNGAAHHHHHHGS)x, with x=1 or 0 SEQ87 (x=1) or SEQ236 (x=0) | GTDFSI NF SEQ1 | FTAT GNT SEQ2 | YMLDK SEQ3 |
| C5^{h2} | EVQLVESGGGVVQPGGSLRLSCAASGTDFSINFIRWVRQAPGKQREFVAG FTATGNTNYADSVKGRFTISRDNAKNTLYLQMNSLRPEDTAVYYCYMLD KWGQGTQVTVSS(AAAEQKLISEEDLNGAAHHHHHHGS)x, with x=1 or 0 SEQ88 (x=1) or SEQ237 (x=0) | GTDFSI NF SEQ1 | FTAT GNT SEQ2 | YMLDK SEQ3 |
| C5^{h3} | EVQLVESGGGVVQPGGSLRLSCAASGTDFSINFMHWVRQAPGKGLEWV AGFTATGNTNYADSVKGRFTISRDNAKNTLYLQMNSLRPEDTAVYYCYM LDKWGQGTQVTVSS(AAAEQKLISEEDLNGAAHHHHHHGS)x, with x=1 or 0 SEQ89 (x=1) or SEQ238 (x=0) | GTDFSI NF SEQ1 | FTAT GNT SEQ2 | YMLDK SEQ3 |
| C5^{h4} | EVQLVESGGGVVQPGGSLRLSCAASGTDFSINFMSWVRQAPGKQREFVA GFTATGNTNYADSVKGRFTISRDNAKNTLYLQMNSLRPEDTAVYYCYML DKWGQGTQVTVSS(AAAEQKLISEEDLNGAAHHHHHHGS)x, with x=1 or 0 SEQ90 (x=1) or SEQ239 (x=0) | GTDFSI NF SEQ1 | FTAT GNT SEQ2 | YMLDK SEQ3 |
| C5^{h5} | EVQLVESGGGVVQPGGSLRLSCAASGTDFSINFIRWVRQAPGKGLEWVA GFTATGNTNYADSVKGRFTISRDNAKNTLYLQMNSLRPEDTAVYYCYML DKWGQGTQVTVSS(AAAEQKLISEEDLNGAAHHHHHHGS)x, with x=1 or 0 SEQ91 (x=1) or SEQ240 (x=0) | GTDFSI NF SEQ1 | FTAT GNT SEQ2 | YMLDK SEQ3 |
| C5^{h6} | EVQLVESGGGVVQPGGSLRLSCAASGTDFSINFIHWVRQAPGKGLEWVA GFTATGNTNYADSVKGRFTISRDNAKNTLYLQMNSLRPEDTAVYYCYML DKWGQGTQVTVSS(AAAEQKLISEEDLNGAAHHHHHHGS)x, with x=1 or 0 SEQ92 (x=1) or SEQ241 (x=0) | GTDFSI NF SEQ1 | FTAT GNT SEQ2 | YMLDK SEQ3 |
| C5^{v20} | EVQLVESGGGVVQPGGSLKLSCVASGHDFSINFIRWYRQAPGKQREFVAG FTATGNTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ393 (x=1) or SEQ242 (x=0) | GHDFSI NF SEQ392 | FTAT GNT SEQ2 | YMLDK SEQ3 |
| C5^{v21} | EVQLVESGGGVVQPGGSLKLSCVASGTDFSINFIRWYRQAPGKQREFVAG WTATGNTNYADSMKGRFTISRDNTKNAVYLQIDSLKPEDTAVYYCYML DKWGQGTQVTVSS(AAAEQKLISEEDLNGAAHHHHHHGS)x, with x=1 or 0 SEQ114 (x=1) or SEQ243 (x=0) | GTDFSI NF SEQ1 | WTAT GNT SEQ11 3 | YMLDK SEQ3 |
| C5^{v22} | EVQLVESGGGVVQPGGSLKLSCVASGTDFSINFIRWYRQAPGKQREFVAG YTATGNTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ116 (x=1) or SEQ244 (x=0) | GTDFSI NF SEQ1 | YTAT GNT SEQ11 5 | YMLDK SEQ3 |
| C5^{v23} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCHMLD KWGQGTQVTVSS(AAAEQKLISEEDLNGAAHHHHHHGS)x, with x=1 or 0 SEQ118 (x=1) or SEQ245 (x=0) | GTDFSI NF SEQ1 | FTAT GNT SEQ2 | HMLDK SEQ117 |
| C5^{v24} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCWMLD KWGQGTQVTVSS(AAAEQKLISEEDLNGAAHHHHHHGS)x, with x=1 or 0 SEQ120 (x=1) or SEQ246 (x=0) | GTDFSI NF SEQ1 | FTAT GNT SEQ2 | WMLDK SEQ119 |
| C5^{v25} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYLLD KWGQGTQVTVSS(AAAEQKLISEEDLNGAAHHHHHHGS)x, with x=1 or 0 SEQ122 (x=1) or SEQ247 (x=0) | GTDFSI NF SEQ1 | FTAT GNT SEQ2 | YLLDK SEQ121 |
| C5^{v26} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCYVLD KWGQGTQVTVSS(AAAEQKLISEEDLNGAAHHHHHHGS)x, with x=1 or 0 SEQ124 (x=1) or SEQ248 (x=0) | GTDFSI NF SEQ1 | FTAT GNT SEQ2 | YVLDK SEQ123 |
| C5^{v27} | EVQLVESGGGVVQPGGSLKLSCVASGADFSINYIRWYRQAPGKQREFVA GFTATGNTNYADSMKGRFTISRDNTKNAVYLQIDSLKPEDTAVYYCYML DKWGQGTQVTVSS(AAAEQKLISEEDLNGAAHHHHHHGS)x, with x=1 or 0 SEQ126 (x=1) or SEQ249 (x=0) | GADFSI NY SEQ125 | FTAT GNT SEQ2 | YMLDK SEQ3 |
| C5^{v28} | EVQLVESGGGVVQPGGSLKLSCVASGADFSINFIRWYRQAPGKQREFVAG FSATGNTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ127 (x=1) or SEQ250 (x=0) | GADFSI NF SEQ17 | FSAT GNT SEQ73 | YMLDK SEQ3 |
| C5^{v29} | EVQLVESGGGVVQPGGSLKLSCVASGADFSINFIRWYRQAPGKQREFVAG FTATGHTNYADSMKGRFTISRDNTKNAVYLQIDSLKPEDTAVYYCYMLD KWGQGTQVTVSS(AAAEQKLISEEDLNGAAHHHHHHGS)x, with x=1 or 0 SEQ129 (x=1) or SEQ251 (x=0) | GADFSI NF SEQ17 | FTAT GHT SEQ12 8 | YMLDK SEQ3 |
| C5^{v30} | EVQLVESGGGVVQPGGSLKLSCVASGTDKSINFIRWYRQAPGKQREFVA GFTATGNTNYADSMKGRFTISRDNTKNAVYLQIDSLKPEDTAVYYCYML DKWGQGTQVTVSS(AAAEQKLISEEDLNGAAHHHHHHGS)x, with x=1 or 0 | GTDKSI NF SEQ671 | FTAT GNT SEQ2 | YMLDK SEQ3 |
| | SEQ679 (x=1) or SEQ675 (x=0) | | | |
| C5^{v31} | EVQLVESGGGVVQPGGSLKLSCVASGTDRSINFIRWYRQAPGKQREFVA GFTATGNTNYADSMKGRFTISRDNTKNAVYLQIDSLKPEDTAVYYCYML DKWGQGTQVTVSS(AAAEQKLISEEDLNGAAHHHHHHGS)x, with x=1 or 0 SEQ680 (x=1) or SEQ676 (x=0) | GTDRSI NF SEQ672 | FTAT GNT SEQ2 | YMLDK SEQ3 |
| C5^{v32} | EVQLVESGGGVVQPGGSLKLSCVASGTDFSINFIRWYSQAPGKQREFVAG FTATGNTNYADSMKGRLTISRDNTKNAVYLQIDSLKPEDTAVYYCYMLG KWGQGTQVTVSS (AAAEQKLISEEDLNGAAHHHHHHGS)x, with x=1 or 0 SEQ692 (x=1) or SEQ701 (x=0) | GTDFSI NF SEQ1 | FTAT GNT SEQ2 | YMLGK SEQ713 |
| C5^{h7} | EVQLVESGGGVVQPGGSLKLSCVASGTDFSINFIRWVRQAPGKGLEWVA GFTATGNTNYADSMKGRFTISRDNTKNALYLQIDSLRPEDTAVYYCYML DKWGQGTQVTVSS(AAAEQKLISEEDLNGAAHHHHHHGS)x, with x=1 or 0 SEQ130 (x=1) or SEQ252 (x=0) | GTDFSI NF SEQ1 | FTAT GNT SEQ2 | YMLDK SEQ3 |
| C5^{h8} | EVQLVESGGGVVQPGGSLRLSCAASGTDFSINFIRWVRQAPGKGLEWVA GFTATGNTNYADSVKGRFTISRDNTKNTLYLQIDSLRPEDTAVYYCYMLD KWGQGTQVTVSS(AAAEQKLISEEDLNGAAHHHHHHGS)x, with x=1 or 0 SEQ131 (x=1) or SEQ253 (x=0) | GTDFSI NF SEQ1 | FTAT GNT SEQ2 | YMLDK SEQ3 |
| C5^{h9} | EVQLVESGGGVVQPGGSLRLSCAASGTDFSINFIRWYRQAPGKGLEFVAG FTATGNTNYADSVKGRFTISRDNAKNTLYLQMNSLRPEDTAVYYCYMLD KWGQGTQVTVSS(AAAEQKLISEEDLNGAAHHHHHHGS)x, with x=1 or 0 SEQ132 (x=1) or SEQ254 (x=0) | GTDFSI NF SEQ1 | FTAT GNT SEQ2 | YMLDK SEQ3 |
| C5^{h10} | EVQLVESGGGVVQPGGSLKLSCAASGTDFSINFIRWVRQAPGKGLEWVA GFTATGNTNYADSVKGRFTISRDNTKNTLYLQINSLRPEDTAVYYCYMLD KWGQGTQVTVSS(AAAEQKLISEEDLNGAAHHHHHHGS)x, with x=1 or 0 SEQ133 (x=1) or SEQ255 (x=0) | GTDFSI NF SEQ1 | FTAT GNT SEQ2 | YMLDK SEQ3 |
| C5^{h11} | EVQLVESGGGVVQPGGSLRLSCAASGTDFSINFIRWVRQAPGKGLEWVA GFTATGNTNYADSMKGRFTISRDNTKNTLYLQMNSLRPEDTAVYYCYML DKWGQGTQVTVSS(AAAEQKLISEEDLNGAAHHHHHHGS)x, with x=1 or 0 SEQ134 (x=1) or SEQ256 (x=0) | GTDFSI NF SEQ1 | FTAT GNT SEQ2 | YMLDK SEQ3 |
| C5^{h12} | EVQLVESGGGVVQPGGSLRLSCAASGTDFSINFIRWYRQAPGKGLEFVAG FTATGNTNYADSVKGRFTISRDNAKNTLYLQIDSLRPEDTAVYYCYMLD KWGQGTQVTVSS(AAAEQKLISEEDLNGAAHHHHHHGS)x, with x=1 or 0 SEQ135 (x=1) or SEQ257 (x=0) | GTDFSI NF SEQ1 | FTAT GNT SEQ2 | YMLDK SEQ3 |
| C5^{h13} | EVQLVESGGGVVQPGGSLRLSCAASGTDFSINFIRWYRQAPGKGLEFVAG FTATGNTNYADSVKGRFTISRDNTKNTLYLQMNSLRPEDTAVYYCYMLD KWGQGTQVTVSS(AAAEQKLISEEDLNGAAHHHHHHGS)x, with x=1 or 0 SEQ136 (x=1) or SEQ258 (x=0) | GTDFSI NF SEQ1 | FTAT GNT SEQ2 | YMLDK SEQ3 |
| C5^{h14} | EVQLVESGGGVVQPGGSLRLSCAASGTDFSINFIRWVRQAPGKGLEWVA GFTATGNTNYADSVKGRFTISRDNTKNALYLQMNSLRPEDTAVYYCYML DKWGQGTQVTVSS(AAAEQKLISEEDLNGAAHHHHHHGS)x, with x=1 or 0 SEQ137 (x=1) or SEQ259 (x=0) | GTDFSI NF SEQ1 | FTAT GNT SEQ2 | YMLDK SEQ3 |
| C5^{h15} | EVQLVESGGGVVQPGGSLRLSCVASGTDFSINFIRWVRQAPGKGLEWVA GFTATGNTNYADSVKGRFTISRDNTKNTLYLQMDSLRPEDTAVYYCYML DKWGQGTQVTVSS(AAAEQKLISEEDLNGAAHHHHHHGS)x, with x=1 or 0 SEQ138 (x=1) or SEQ260 (x=0) | GTDFSI NF SEQ1 | FTAT GNT SEQ2 | YMLDK SEQ3 |
| C5^{h16} | EVQLVESGGGLVQPGGSLRLSCAASGTDFSINFIRWYRQAPGKGLEFVAG FTATGNTNYADSVKGRFTISRDNSKNTLYLQMNSLRAEDTAVYYCYMLD KWGQGTLVTVSS(AAAEQKLISEEDLNGAAHHHHHHGS)x, with x=1 or 0 SEQ139 (x=1) or SEQ261 (x=0) | GTDFSI NF SEQ1 | FTAT GNT SEQ2 | YMLDK SEQ3 |
| C5^{h17} | EVQLVESGGGVVQPGGSLKLSCVASGTDFSINFIRWVRQAPGKGLEFVAG FTATGNTNYADSMKGRFTISRDNTKNALYLQIDSLRPEDTAVYYCYMLD KWGQGTQVTVSS(AAAEQKLISEEDLNGAAHHHHHHGS)x, with x=1 or 0 SEQ140 (x=1) or SEQ262 (x=0) | GTDFSI NF SEQ1 | FTAT GNT SEQ2 | YMLDK SEQ3 |
| C5^{h18} | EVQLVESGGGVVQPGGSLKLSCVASGTDFSINFIRWYRQAPGKGLEFVAG FTATGNTNYADSVKGRFTISRDNAKNTLYLQMNSLRPEDTAVYYCYMLD KWGQGTQVTVSS(AAAEQKLISEEDLNGAAHHHHHHGS)x, with x=1 or 0 | GTDFSI NF SEQ1 | FTAT GNT SEQ2 | YMLDK SEQ3 |
| | SEQ141 (x=1) or SEQ263 (x=0) | | | |
| C5^{h19} | EVQLVESGGGVVQPGGSLRLSCAASGTDFSINFIRWYRQAPGKQREFVAG FTATGNTNYADSVKGRFTISRDNAKNTLYLQMNSLRPEDTAVYYCYMLD KWGQGTQVTVSS(AAAEQKLISEEDLNGAAHHHHHHGS)x, with x=1 or 0 SEQ142 (x=1) or SEQ264 (x=0) | GTDFSI NF SEQ1 | FTAT GNT SEQ2 | YMLDK SEQ3 |
| C5^{h20} | EVQLVESGGGVVQPGGSLRLSCAASGTDFSINFIRWYRQAPGKGLEFVAG FTATGNTNYADSVKGRFTISRDNAKNAVYLQMNSLRPEDTAVYYCYML DKWGQGTQVTVSS(AAAEQKLISEEDLNGAAHHHHHHGS)x, with x=1 or 0 SEQ143 (x=1) or SEQ265 (x=0) | GTDFSI NF SEQ1 | FTAT GNT SEQ2 | YMLDK SEQ3 |
| C5^{h21} | EVQLVESGGGVVQPGGSLRLSCAASGTDFSINFIRWYRQAPGKGLEFVAG FTATGNTNYADSVKGRFTISRDNAKNTLYLQMNSLKPEDTAVYYCYMLD KWGQGTQVTVSS(AAAEQKLISEEDLNGAAHHHHHHGS)x, with x=1 or 0 SEQ144 (x=1) or SEQ266 (x=0) | GTDFSI NF SEQ1 | FTAT GNT SEQ2 | YMLDK SEQ3 |
| C5^{h22} | EVQLVESGGGVVQPGGSLRLSCAASGTDFSINFIRWYRQAPGKGREFVAG FTATGNTNYADSVKGRFTISRDNAKNTLYLQMNSLRPEDTAVYYCYMLD KWGQGTQVTVSS(AAAEQKLISEEDLNGAAHHHHHHGS)x, with x=1 or 0 SEQ145 (x=1) or SEQ267 (x=0) | GTDFSI NF SEQ1 | FTAT GNT SEQ2 | YMLDK SEQ3 |
| C5^{h23} | EVQLVESGGGVVQPGGSLRLSCAASGTDFSINFIRWVRQAPGKQREFVAG FTATGNTNYADSVKGRFTISRDNAKNTLYLQMNSLRPEDTAVYYCYMLD KWGQGTQVTVSS(AAAEQKLISEEDLNGAAHHHHHHGS)x, with x=1 or 0 SEQ146 (x=1) or SEQ268 (x=0) | GTDFSI NF SEQ1 | FTAT GNT SEQ2 | YMLDK SEQ3 |
| C5^{h24} | EVQLVESGGGVVQPGGSLRLSCAASGTDFSINFIRWYRQAPGKGLEFVAG FTATGNTNYADSMKGRFTISRDNAKNTLYLQMNSLRPEDTAVYYCYML DKWGQGTQVTVSS(AAAEQKLISEEDLNGAAHHHHHHGS)x, with x=1 or 0 SEQ147 (x=1) or SEQ269 (x=0) | GTDFSI NF SEQ1 | FTAT GNT SEQ2 | YMLDK SEQ3 |
| C5^{h25} | EVQLVESGGGVVQPGGSLRLSCAASGTDFSINFIRWVRQAPGKGLEFVAG FTATGNTNYADSVKGRFTISRDNAKNTLYLQIDSLRPEDTAVYYCYMLD KWGQGTQVTVSS(AAAEQKLISEEDLNGAAHHHHHHGS)x, with x=1 or 0 SEQ148 (x=1) or SEQ270 (x=0) | GTDFSI NF SEQ1 | FTAT GNT SEQ2 | YMLDK SEQ3 |
| C5^{h26} | EVQLVESGGGVVQPGGSLRLSCAASGTDFSINFIRWYRQAPGKGLEWVA GFTATGNTNYADSVKGRFTISRDNAKNTLYLQIDSLRPEDTAVYYCYML DKWGQGTQVTVSS(AAAEQKLISEEDLNGAAHHHHHHGS)x, with x=1 or 0 SEQ149 (x=1) or SEQ271 (x=0) | GTDFSI NF SEQ1 | FTAT GNT SEQ2 | YMLDK SEQ3 |
| C5^{neg (control )} | EVQLVESGGGVVQPGGSLKLSCVASGTDFSINFIRWYRQAPGKQREFVAG FTATGNTNYADSMKGRFTISRDNTKNAVYLQIDSLKPEDTAVYYCAMAD KWGQGTQVTVSS(AAAEQKLISEEDLNGAAHHHHHHGS)x, with x=1 or 0 SEQ151 (x=1) or SEQ272 (x=0) | GTDFSI NF SEQ1 | FTAT GNT SEQ2 | AMADK SEQ150 |
| B8^{h1} | EVQLVESGGGVVQPGGSLRLSCAASGEIFSINFMRWYRQAPGKGLEWVA GFTRDGSTNYPDSVKGRFTISRDNAKNTVYLQMNSLRPEDTAVYYCYML DTWGQGTQVTVSS(AAAEQKLISEEDLNGAAHHHHHHGS)x, with x=1 or 0 SEQ152 (x=1) or SEQ273 (x=0) | GEIFSIN F SEQ5 | FTRD GST SEQ6 | YMLDT SEQ7 |
| C5^{V1 3h20} | EVQLVESGGGVVQPGGSLRLSCAASGTDFSINFIRWYRQAPGKGLEFVAG FSATGNTNYADSVKGRFTISRDNAKNAVYLQMNSLRPEDTAVYYCYML DKWGQGTQVTVSS(AAAEQKLISEEDLNGAAHHHHHHGS)x, with x=1 or 0 SEQ153 (x=1) or SEQ274 (x=0) | GTDFSI NF SEQ1 | FSAT GNT SEQ73 | YMLDK SEQ3 |
| C5^{V1 6h20} | EVQLVESGGGVVQPGGSLRLSCAASGTDFSINFIRWYRQAPGKGLEFVAG FTATGNTNYADSVKGRFTISRDNAKNAVYLQMNSLRPEDTAVYYCYILD KWGQGTQVTVSS(AAAEQKLISEEDLNGAAHHHHHHGS)x, with x=1 or 0 SEQ154 (x=1) or SEQ275 (x=0) | GTDFSI NF SEQ1 | FTAT GNT SEQ2 | YILDK SEQ79 |
| B8^{V1} | EVQLVESGGGVVQPGGSLRLSCAADGEIFSINFMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ155 (x=1) or SEQ276 (x=0) | GEIFSIN F SEQ5 | FTRD GST SEQ6 | YMLDT SEQ7 |
| B8^{V2} | EVQLVESGGGVVQPGGSLRLSCAASGEIFSINFMRWYGQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x with x=1 or 0 | GEIFSIN F | FTRD GST | YMLDT SEQ7 |
| | SEQ156 (x=1) or SEQ277 (x=0) | SEQ5 | SEQ6 | |
| B8^{V3} | EVQLVESGGGVVQPGGSLRLSCAASGEIFSINFMRWYREAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ157 (x=1) or SEQ278 (x=0) | GEIFSIN F SEQ5 | FTRD GST SEQ6 | YMLDT SEQ7 |
| B8^{V4} | EVQLVESGGGVVQPGGSLRLSCAASGEIFSINFMRWYRQAPIKQREWVAG FTRDGSTNYPDSAKGRFTISRDNAKNTVYLQIDSLKPEDTAVYYCYMLDT WGQGTQVTVSS(AAAEQKLISEEDLNGAAHHHHHHGS)x, with x=1 or 0 SEQ158 (x=1) or SEQ279 (x=0) | GEIFSIN F SEQ5 | FTRD GST SEQ6 | YMLDT SEQ7 |
| B8^{V5} | EVQLVESGGGVVQPGGSLRLSCAASGEIFSINFMRWYRQAPGKIREWVAG FTRDGSTNYPDSAKGRFTISRDNAKNTVYLQIDSLKPEDTAVYYCYMLDT WGQGTQVTVSS(AAAEQKLISEEDLNGAAHHHHHHGS)x, with x=1 or 0 SEQ159 (x=1) or SEQ280 (x=0) | GEIFSIN F SEQ5 | FTRD GST SEQ6 | YMLDT SEQ7 |
| B8^{V6} | EVQLVESGGGVVQPGGSLRLSCAASGEIFSINFMRWYRQAPGKQREWVA GFTRDGFTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ161 (x=1) or SEQ281 (x=0) | GEIFSIN F SEQ5 | FTRD GFT SEQ16 0 | YMLDT SEQ7 |
| B8^{V7} | EVQLVESGGGVVQPGGSLRLSCAASGEIFSINFMRWYRQAPGKQREWVA GFTRDGYTNYPDSAKGRFTISRDNAKNTVYLQIDSLKPEDTAVYYCYML DTWGQGTQVTVSS(AAAEQKLISEEDLNGAAHHHHHHGS)x, with x=1 or 0 SEQ163 (x=1) or SEQ282 (x=0) | GEIFSIN F SEQ5 | FTRD GYT SEQ16 2 | YMLDT SEQ7 |
| B8^{V8} | EVQLVESGGGVVQPGGSLRLSCAASGEIFSINFMRWYRQAPGKQREWVA GFTRDGSLNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ165 (x=1) or SEQ283 (x=0) | GEIFSIN F SEQ5 | FTRD GSL SEQ16 4 | YMLDT SEQ7 |
| B8^{V9} | EVQLVESGGGVVQPGGSLRLSCAASGEIFSINFMRWYRQAPGKQREWVA GFTRDGSKNYPDSAKGRFTISRDNAKNTVYLQIDSLKPEDTAVYYCYML DTWGQGTQVTVSS(AAAEQKLISEEDLNGAAHHHHHHGS)x, with x=1 or 0 SEQ167 (x=1) or SEQ284 (x=0) | GEIFSIN F SEQ5 | FTRD GSK SEQ16 6 | YMLDT SEQ7 |
| B6^{V1} | EVQLVESGGGLVQPGGSLRLSCTASGGPIEQYPMGWFRRAPGKERNLVA SISRSGDGTYYAISSVKGRFTISRDNAENTVFLQMNSLKPDDTAVYYCGA GINPTKIWGQGTQVTVSS(AAAEQKLISEEDLNGAAHHHHHHGS)x, with x=1 or 0 SEQ168 (x=1) or SEQ285 (x=0) | GGPIEQ YP SEQ9 | ISRSG DGTY SEQ10 | GAGINP TKI SEQ11 |
| B6^{V2} | EVQLVESGGGLVQPGGSLRLSCTASGGPIEQYPMGWFRRAPGKERELVAS FSRSGDGTYYAISSVKGRFTISRDNAENTVFLQMNSLKPDDTAVYYCGAG INPTKIWGQGTQVTVSS(AAAEQKLISEEDLNGAAHHHHHHGS)x, with x=1 or 0 SEQ170 (x=1) or SEQ286 (x=0) | GGPIEQ YP SEQ9 | FSRSG DGTY SEQ16 9 | GAGINP TKI SEQ11 |
| B6^{V3} | EVQLVESGGGLVQPGGSLRLSCTASGGPIEQYPMGWFRRAPGKERELVAS SSRSGDGTYYAISSVKGRFTISRDNAENTVFLQMNSLKPDDTAVYYCGAG INPTKIWGQGTQVTVSS(AAAEQKLISEEDLNGAAHHHHHHGS)x, with x=1 or 0 SEQ172 (x=1) or SEQ287 (x=0) | GGPIEQ YP SEQ9 | SSRSG DGTY SEQ17 1 | GAGINP TKI SEQ11 |
| B6^{V4} | EVQLVESGGGLVQPGGSLRLSCTASGGPIEQYPMGWFRRAPGKERESVAS ISRSGDGTYYAISSVKGRFTISRDNAENTVFLQMNSLKPDDTAVYYCGAGI NPTKIWGQGTQVTVSS(AAAEQKLISEEDLNGAAHHHHHHGS)x, with x=1 or 0 SEQ173 (x=1) or SEQ288 (x=0) | GGPIEQ YP SEQ9 | ISRSG DGTY SEQ10 | GAGINP TKI SEQ11 |
| B6^{V5} | EVQLVESGGGLVQPGGSLRLSCTASGGPIEQYPMGWFRRAPGKERELQAS ISRSGDGTYYAISSVKGRFTISRDNAENTVFLQMNSLKPDDTAVYYCGAGI NPTKIWGQGTQVTVSS(AAAEQKLISEEDLNGAAHHHHHHGS)x, with x=1 or 0 SEQ174 (x=1) or SEQ289 (x=0) | GGPIEQ YP SEQ9 | ISRSG DGTY SEQ10 | GAGINP TKI SEQ11 |
| B7 | QVQLVQSGGGLVQAGGSLTLSCTASGRPLSDYPMGWFRRAPEKERELVA SISRTGDGTYYADGMRGRFTISRDNSENTVSLQMNNLKPEDTAVYYCGA GINPTKYWGQGTQVTVSS(AAAEQKLISEEDLNGAAHHHHHHGS)x, with x=1 or 0 SEQ178 (x=1) or SEQ290 (x=0) | GRPLSD YP SEQ175 | ISRTG DGT SEQ17 6 | GAGINP TKY SEQ177 |
| H7 | EVQLVESGGGLVQAGGSLRLSCTASGRPISDYPMGWFRRAPGKDRELVA SISRTGDGTYYANSMKERFTISRDNAQNTVSLQISSLKPEDTAVYYCGAGI NPTRYWGQGTQVTVSS(AAAEQKLISEEDLNGAAHHHHHHGS)X, with x=1 or 0 SEQ181 (x=1) or SEQ291 (x=0) | GRPISD YP SEQ179 | ISRTG DGT SEQ17 6 | GAGINP TRY SEQ180 |
| C12b | QVQLVQSGGGLVQPGGSLRLSCAASGFTFSAYAMSWVRQAPGKGRELV ASISRTGDGTYYADGMKGRFTISRDNAENTVSLQINSLKPEDTAIYYCGA GINPTKYWGQGTQVTVSS(AAAEQKLISEEDLNGAAHHHHHHGS)X, with x=1 or 0 SEQ183 (x=1) or SEQ292 (x=0) | GFTFSA YA SEQ182 | ISRTG DGT SEQ17 6 | GAGINP TKY SEQ177 |
| E4 | EVQLVESGGGLVQAGDSLRLSCTASGLPLSNYPMGWFRRAPGKERELIAS ISRTGDGTYYADSSVKGRFTISRDNAENTVSLQMNSLKPEDTAVYYCGAG INPTKYWGQGTQVTVSS(AAAEQKLISEEDLNGAAHHHHHHGS)X, with x=1 or 0 SEQ185 (x=1) or SEQ293 (x=0) | GLPLSN YP SEQ184 | ISRTG DGT SEQ17 6 | GAGINP TKY SEQ177 |
| E2 | QVQLVQSGGGLVQAGGSLRLSCAASGSIFSDITLAWHRQIPGKEREWVAG ISRSGPSYRDSVKGRFTISRDNAKNTVFLQMNSLEPEDTGVYYCYSRGY WGQGTQVTVSS(AAAEQKLISEEDLNGAAHHHHHHGS)x, with x=1 or 0 SEQ189 (x=1) or SEQ294 (x=0) | GSIFSDI T SEQ186 | ISRSG SP SEQ18 7 | YSRGY SEQ188 |
| C3 | EVQLVQSGGGLVQAGGSLRLSCAASGRDFSDTSMAWHRQAPGKERLWV AGIRSGSTSTSYADSVKGRFTISRDDAKNTVYLQMDNLTPEDTAVYFCYS KGYWGQGTQVTVSS(AAAEQKLISEEDLNGAAHHHHHHGS)x, with x=1 or 0 SEQ193 (x=1) or SEQ295 (x=0) | GRDFSD TS SEQ190 | IRSGS TST SEQ19 1 | YSKGY SEQ192 |
| F2a | EVQLVESGGGLVQPGESLRLSCTASESISIYGVGWYRQAPGEQRVLVAHL KRNGLTEYKDSVKGRFTISRDNARNTIYLEMKNLKPEDTAIYYCNTALGS VLPWGRGTQVTVSS(AAAEQKLISEEDLNGAAHHHHHHGS)x, with x=1 or 0 SEQ197 (x=1) or SEQ296 (x=0) | ESISIYG SEQ194 | LKRN GLT SEQ19 5 | NTALG SVLP SEQ196 |
| E9 | EVQLVESGGGLVQPGGSLRLSCVSSATFGGYSMGWFRQAPGKEREFVAA ITWNGGIIKDYADGVMGRFTISRNSALNTVYLQMDSLKSTDTGVYVCAG DRTYYAGGYVPTSEREYAFWGQGTQVTVSS(AAAEQKLISEEDLNGAAH HHHHHGS)x, with x=1 or 0 SEQ691 (x=1) or SEQ297 (x=0) | ATFGG YS SEQ198 | ITWN GGIIK SEQ19 9 | AGDRT YYAGG YVPTSE REYAF SEQ200 |
| A9a | QVQLVQSGGGLVQAGGSLRLSCAASGSIFLINDMGWYRQAPGKQRELVA VRGGSTKYGDSVKGRFTISRDDAKNTVYLQMNSLKPEDTAVYYCHRESG LGSSEYWGQGTQVTVSS(AAAEQKLISEEDLNGAAHHHHHHGS)x, with x=1 or 0 SEQ204 (x=1) or SEQ298 (x=0) | GSIFLIN D SEQ201 | RGGS T SEQ20 2 | HRESGL GSSEY SEQ203 |
| B4a | EVQLVESGGGLVQAGGSLRLSCTASGRTVSNYRMGWFRQTPGKEREFVA AIAWAGGSLTYADSVKGRFTISRDNAKNTVYLQMNSLEPTDTAVYYCKG SRIFPTRDYWGKGTQVTVSS(AAAEQKLISEEDLNGAAHHHHHHGS)x, with x=1 or 0 SEQ208 (x=1) or SEQ299 (x=0) | GRTVS NYR SEQ205 | IAWA GGSL SEQ20 6 | KGSRIF PTRDY SEQ207 |
| D12 ^{(control)} | EVQLVESGGGLVQPGGSLRLSCAASGFTFGSYVMSWVRQAPGKGPEWVS TVSSGGGTTYYADSVKGRFTISRDNAQNTLYLQMNSLKPEDTAVYYCAT GRTGLGEGDHRGQGTQVTSS(AAAEQKLISEEDLNGAAHHHHHHGS)x, with x=1 or 0 SEQ212 (x=1) or SEQ300 (x=0) | GFTFGS YV SEQ209 | VSSG GGTT SEQ21 0 | ATGRT GLGEG DH SEQ211 |
| B8^{V11} | EVQLVESGGGVVQPGGSLRLSCAASDEIFSINFMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ411 (x=1) or SEQ412 (x=0) | DEIFSIN F SEQ410 | FTRD GST SEQ6 | YMLDT SEQ7 |
| B8^{V12} | EVQLVESGGGVVQPGGSLRLSCAASGEIFYINFMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ414 (x=1) or SEQ415 (x=0) | GEIFYI NF SEQ413 | FTRD GST SEQ6 | YMLDT SEQ7 |
| B8^{V14} | EVQLVESGGGVVQPGGSLRLSCAASGEIFSINFMRWYRQAPGKQREWVA GFTRLGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ417 (x=1) or SEQ418 (x=0) | GEIFSIN F SEQ5 | FTRL GST SEQ41 6 | YMLDT SEQ7 |
| B8^{V15} | EVQLVESGGGVVQPGGSLRLSCAASGEIFSINFMRWYRQAPGKQREWVA GFTRDDSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ420 (x=1) or SEQ421 (x=0) | GEIFSIN F SEQ5 | FTRD DST SEQ41 9 | YMLDT SEQ7 |
| B8^{V16} | EVQLVESGGGVVQPGGSLRLSCAASGEIFSINFMRWYRQAPGKQREQVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ422 (x=1) or SEQ423 (x=0) | GEIFSIN F SEQ5 | FTRD GST SEQ6 | YMLDT SEQ7 |
| B8^{V17} | EVQLVESGGGVVQPGGSLRLSCAASGEIFSINFMRWYRQAPGKQREFVAG FTRDGSTNYPDSAKGRFTISRDNAKNTVYLQIDSLKPEDTAVYYCYMLDT WGQGTQVTVSS(AAAEQKLISEEDLNGAAHHHHHHGS)X, with x=1 or 0 SEQ424 (x=1) or SEQ425 (x=0) | GEIFSIN F SEQ5 | FTRD GST SEQ6 | YMLDT SEQ7 |
| B8^{V18} | EVQLVESGGGVVQPGGSLRLSCAASGEIHSINFMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ427 (x=1) or SEQ428 (x=0) | GEIHSI NF SEQ426 | FTRD GST SEQ6 | YMLDT SEQ7 |
| B8^{V19} | EVQLVESGGGVVQPGGSLRLSCAASGEIFSINFMRWYRQAPGKQRHWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ429 (x=1) or SEQ430 (x=0) | GEIFSIN F SEQ5 | FTRD GST SEQ6 | YMLDT SEQ7 |
| B8^{V20} | EVQLVESGGGVVQPGGSLRLSCAASGEIFSINFMRWYRQAPGKQREWVA GFTRDGSHNYPDSAKGRFTISRDNAKNTVYLQIDSLKPEDTAVYYCYML DTWGQGTQVTVSS(AAAEQKLISEEDLNGAAHHHHHHGS)x, with x=1 or 0 SEQ432 (x=1) or SEQ433 (x=0) | GEIFSIN F SEQ5 | FTRD GSH SEQ43 1 | YMLDT SEQ7 |
| B8^{V21} | EVQLVESGGGVVQPGGSLRLSCAASGEIFSINNMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ435 (x=1) or SEQ436 (x=0) | GEIFSIN N SEQ434 | FTRD GST SEQ6 | YMLDT SEQ7 |
| B8^{V22} | EVQLVESGGGVVQPGGSLRLSCAASGEIFSINQMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ438 (x=1) or SEQ439 (x=0) | GEIFSIN Q SEQ437 | FTRD GST SEQ6 | YMLDT SEQ7 |
| B8^{V23} | EVQLVESGGGVVQPGGSLRLSCAASGEIFSINFMIWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ440 (x=1) or SEQ441 (x=0) | GEIFSIN F SEQ5 | FTRD GST SEQ6 | YMLDT SEQ7 |
| B8^{V24} | EVQLVESGGGVVQPGGSLRLSCAASGEIFSINFMEWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ442 (x=1) or SEQ443(x=0) | GEIFSIN F SEQ5 | FTRD GST SEQ6 | YMLDT SEQ7 |
| B8^{V25} | EVQLVESGGGVVQPGGSLRLSCAASGEIFSINFMRWYRQAPGKQREWVA AFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ444 (x=1) or SEQ445 (x=0) | GEIFSIN F SEQ5 | FTRD GST SEQ6 | YMLDT SEQ7 |
| B8^{V26} | EVQLVESGGGVVQPGGSLRLSCAASGEIFSINFMRWYRQAPGKQREWVA KFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ446 (x=1) or SEQ447 (x=0) | GEIFSIN F SEQ5 | FTRD GST SEQ6 | YMLDT SEQ7 |
| B8^{V27} | EVQLVESGGGVVQPGGSLRLSCAASGEIFSINFMRWYRQAPGKQREWVA GFTRDGSTVYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ448 (x=1) or SEQ449 (x=0) | GEIFSIN F SEQ5 | FTRD GST SEQ6 | YMLDT SEQ7 |
| B8^{V28} | EVQLVESGGGVVQPGGSLRLSCAASGEIFSINFMRWYRQAPGKQREWVA GFTRDGSTFYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ450 (x=1) or SEQ451(x=0) | GEIFSIN F SEQ5 | FTRD GST SEQ6 | YMLDT SEQ7 |
| B8^{V29} | EVQLVESGGGVVQPGGSLRLSCAASGEIFSINFMRWYRQAPGKQREWVA GFTRDGSTNYPDSAKGRFTISRDNAKNTVYLQIDSLKPEDTAVYYCYMA DTWGQGTQVTVSS(AAAEQKLISEEDLNGAAHHHHHHGS)x, with x=1 or 0 SEQ453 (x=1) or SEQ454(x=0) | GEIFSIN F SEQ5 | FTRD GST SEQ6 | YMADT SEQ452 |
| B8^{V30} | EVQLVESGGGVVQPGGSLRLSCAASGEIFSINFMRWYRQAPGKQREWVA GFTRDGSTNYPDSAKGRFTISRDNAKNTVYLQIDSLKPEDTAVYYCYMQ DTWGQGTQVTVSS(AAAEQKLISEEDLNGAAHHHHHHGS)x, with x=1 or 0 SEQ456 (x=1) or SEQ457(x=0) | GEIFSIN F SEQ5 | FTRD GST SEQ6 | YMQDT SEQ455 |
| B8^{V31} | EVQLVESGGGVVQPGGSLRLSCAASGEIKSINFMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ681 (x=1) or SEQ677 (x=0) | GEIKSI NF SEQ673 | FTRD GST SEQ6 | YMLDT SEQ7 |
| B8^{V32} | EVQLVESGGGVVQPGGSLRLSCAASGEIRSINFMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ682 (x=1) or SEQ678 (x=0) | GEIRSI NF SEQ674 | FTRD GST SEQ6 | YMLDT SEQ7 |
| B8^{V33} | EVQLVESGGGVVQPGGSLRLSCAASGEIFSINFMRWYRQAPGKQREWVA GFTRDGSTNYPDSAKGRFTISRDNAKNTVYLQIDSLKPEDTAVYYCYMLK TWGQGTQVTVSS(AAAEQKLISEEDLNGAAHHHHHHGS)x, with x=1 or 0 SEQ693 (x=1) or SEQ702 (x=0) | GEIFSIN F SEQ5 | FTRD GST SEQ6 | YMLKT SEQ714 |
| B8^{V34} | EVQLVESGGGVVQPGGSLRLSCAASGEIRSINFMRWYRQAPGKQREWVA GFTRDGSTNYPDIAKGRFTISRDNAKNTVYLQIDSLKPEDTAVYYCYMLD TWGQGTQVTVSS(AAAEQKLISEEDLNGAAHHHHHHGS)x, with x=1 or 0 SEQ694 (x=1) or SEQ703 (x=0) | GEIRSI NF SEQ674 | FTRD GST SEQ6 | YMLDT SEQ7 |
| B8^{V35} | EVQLVESGGGVVQPGGSLRLSCAASGEIRSINFMRWYRQAPGKQREWVA GFTRDGSLNYPDSAKGRFTISRDNAKNTVYLQIDSLKPEDTAVYYCYMLD TWGKGTQVTVSS(AAAEQKLISEEDLNGAAHHHHHHGS)x, with x=1 or 0 SEQ695 (x=1) or SEQ704 (x=0) | GEIRSI NF SEQ674 | FTRD GSL SEQ16 4 | YMLDT SEQ7 |
| B8^{V36} | EVQLVESGGGVVQPGGSLRLSCAASGEIFSINVMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ696 (x=1) or SEQ705 (x=0) | GEIFSIN V SEQ710 | FTRD GST SEQ6 | YMLDT SEQ7 |
| B8^{V37} | EVQLVESGGGVVQPGGSLRLSCAASGEIFSINFMRWYRQAPGKQREWVA GFTRDGSTNYPDSAKGRFTISEDNAKNTVYLQIDSLKPEDTAVYYCYMLR TWGQGTQVTVSS(AAAEQKLISEEDLNGAAHHHHHHGS)x, with x=1 or 0 SEQ697 (x=1) or SEQ706 (x=0) | GEIFSIN F SEQ5 | FTRD GST SEQ6 | YMLRT SEQ715 |
| B8^{V38} | EVQLVESGGGVVQPGGSLRLSCAASGEIRSINFMRWYRQAPGKQREWVA GFTRTGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ698 (x=1) or SEQ707 (x=0) | GEIRSI NF SEQ674 | FTRT GST SEQ71 2 | YMLDT SEQ7 |
| B8^{V39} | EVQLVESGGGVVQPGGSLRLSCAASGEIFKINFMRWYRQAPGKQREWVA GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ699 (x=1) or SEQ708 (x=0) | GEIFKI NF SEQ711 | FTRD GST SEQ6 | YMLDT SEQ7 |
| B8^{V40} | EVQLVESGGGVVQPGGSLRLSCAASGEIKSINFMRWYRQAPGKQREWVS GFTRDGSTNYPDSAKGRFTISRDNAKNTVYLQIDSLKPEDTAVYYCYMLD TWGQGTQVTVSS(AAAEQKLISEEDLNGAAHHHHHHGS)x, with x=1 or 0 SEQ700 (x=1) or SEQ709 (x=0) | GEIKSI NF SEQ673 | FTRD GST SEQ6 | YMLDT SEQ7 |
| C10a | EVQLVESGGGLVQAGDSLRLSCAASGFASHLYAIGWFRQAPGKEREKVS CISSNDGSTYYADSVKGRFTISRDTVKDMVYLQMNSLKPEDTAVYYCAT DLTVHCFMAEYDWWGQGTQVTVSS(AAAEQKLISEEDLNGAAHHHHHH GS)x, with x=1 or 0 SEQ616 (x=1) or SEQ613 (x=0) | GFASHL YA SEQ607 | ISSND GST SEQ60 8 | ATDLT VHCFM AEYDW SEQ609 |
| H1 | EVQLVESGGGLVQPGGSLRLSCAASGFASHLYAIGWFRQAPGKEREKVSC ISSNDGSTYYADSVKGRFTISRDTVKDMVYLQMNSLKPEDTAVYYCATD LTVHCFMAEYDWWGQGTQVTVSS(AAAEQKLISEEDLNGAAHHHHHHG S)x, with x=1 or 0 SEQ617 (x=1) or SEQ614 (x=0) | GFASHL YA SEQ607 | ISSND GST SEQ60 8 | ATDLT VHCFM AEYDW SEQ609 |
| E8 | VQLVESGGRLVQAGGSLRLSCTASGLAFSTHTMHWFRQAPGKEREFVGA IRWSDEDTEYADSVKGRFTISRDNAKSTVYLQMNNLKPEDTAVYYCAAA RGVGTSGHMKYWGQGTQVTVSS(AAAEQKLISEEDLNGAAHHHHHHGS) x, with x=1 or 0 SEQ618 (x=1) or SEQ615 (x=0) | GLAFST HT SEQ610 | IRWS DEDT SEQ61 1 | AAARG VGTSG HMKY SEQ612 |

**Table 2: List of nucleotide sequences coding for the VHH molecules**

| **VHH** | **SEQ ID** | **nucleotide sequences comprising or not an optional tag sequence of SEQ ID NO: 330 (in bold)** |
|---|---|---|
| C5 | 52 (x=1)or 301 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{V1} | 53 (x=1)or 302 (x=0) | GAGGTGcAGCTGGTGGAGTCTGGGGGAGGCGTGGTGCAGCCTGGGGGGTCTCTAAAACTCTCCTG CGTAGCCTCGGGAGCGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{V2} | 54 (x=1)or 303 (x=0) | GAGGTGcAGCTGGTGGAGTCTGGGGGAGGCGTGGTGCAGCCTGGGGGGTCTCTAAAACTCTCCTG CGTAGCCTCGGGAACGGCGTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{V3} | 55 (x=1)or | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGGCGGGTAACACAAACTATGCAGACTCCATGAAGGGG |
| | 304 (x=0) | CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{V4} | 56 (x=1)or 305 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTGCGACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x,with x=1 or 0 |
| C5^{V5} | 57 (x=1)or 306 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCGCGATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x,with x=1 or 0 |
| C5^{V6} | 58 (x=1)or 307 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATGCGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{V7} | 59 (x=1)or 308 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGGCGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{V8} | 60 (x=1)or 309 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGCGAAGTGGGGCCAGGGGACCCAGGTCACCG TCTCCTCAGCGGCCGCAGAACAAAAACTCATCTCAGAAGAGGATCTGAATGGGGCCGCACAT CACCACCATCACCATGGGAGCTAG)x, with x=1 or 0 |
| C5^{V9} | 61 (x=1)or 310 (x=0) | GAGGTGcAGCTGGTGGAGTCTGGGGGAGGCGTGGTGCAGCCTGGGGGGTCTCTAAAACTCTCCTG CGTAGCCTCGGGAACGGACTTCAGTATCAATTTTATACGCTGGTACCGCCAGGCTCCAGGGAAGC AGCGCGAGTTCGTCGCAGGATTTACTGCGACTGGTAACACAAACTATGCAGACTCCATGAAGGGG CGATTCACCATCTCCAGAGACAACACCAAGAACGCGGTGTATCTGCAAATAGACAGCCTGAAACC TGAGGACACGGCCGTGTATTACTGCTATATGTTGGACGCGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| B8 | 62 (x=1)or 311 (x=0) | GAgGTGCAGCTGGTGGAGTCTGGGGGagGCGTGGTGCAGCCTGGGGGGTCTCTGAGACTCTCCTGT GCAGCCTCTGGAGAGATCTTCAGTATCAATTTTATGCGCTGGTACCGCCAGGCTCCAGGGAAGCA GCGCGAGTGGGTCGCAGGTTTTACTAGGGATGGAAGCACAAACTATCCAGACTCCGCGAAGGGCC GATTCACCATCTCTAGAGACAACGCCAAGAACACGGTGTATCTGCAAATAGACAGCCTGAAACCT GAGGACACGGCCGTCTATTATTGTTATATGTTGGACACCTGGGGCCAGGGGACCCAGGTCACTGT CTCCTCA(GCGGCCGCAGAACAAAAACTCATCTCAGAAGAGGATCTGAATGGGGCCGCACAT CACCACCATCACCATGGGAGCTAG)x, with x=1 or 0 |
| B6 | 63 (x=1)or 312 (x=0) | GAGGTGCAGCTGGTGGAGTCTGGGGGAGGCTTGGTGCAGCCTGGGGGTTCTCTGAGACTCTCCTG TACAGCCTCTGGAGGCCCCATCGAGCAGTATCCCATGGGCTGGTTCCGCCGGGCCCCAGGAAAGG AGCGTGAATTGGTAGCAAGTATTAGCCGAAGTGGAGATGGCACATACTATGCAATCTCTTCCGTG AAGGGCCGATTCACCATCTCTAGAGACAACGCCGAGAACACGGTATTTCTGCAAATGAACAGCCT |
| | | GAAACCTGACGACACGGCCGTTTATTACTGTGGGGCTGGTATAAACCCAACCAAGATCTGGGGCC AGGGGACCCAGGTCACCGTCTCCTCA(GCGGCCGCAGAACAAAAACTCATCTCAGAAGAGGAT CTGAATGGGGCCGCACATCACCACCATCACCATGGGAGCTAG)x, with x=1 or 0 |
| H3 | 64 (x=1)or 313 (x=0) | GAGGTGCAGCTGGTGGaGTCTGGGGGAggCGAGGTGCAGCCtgGgGGGTCTCTGAAACTCTCCTGTG TAGCCTCTGGAACCGACTTCAGTATCAATTTTGTGCGCTGGTACCGTCAGCGTCCAGGGAAGCAG CGCGAGTGGGTCGCAGGATTTACTGCGAATGGTGATACAAACTATCCAGACTCCATGAAGGGGCG ATTCACCATTTCCAGAGACAACGCCaAGAATACGGTGTATCTACAGATAAACAGCCTGAAATCTG AGGACACGGCCGTCTATTATTGCTATATGTTAgATAATTGGGGCCAGGGGACCCAGGTCACCGTCT CCTCA(GCGGCCGCAGAACAAAAACTCATCTCAGAAGAGGATCTGAATGGGGCCGCACATCA CCACCATCACCATGGGAGCTAG)x, with x=1 or 0 |
| C5^{V10} | 95 (x=1)or 314 (x=0) | GagGTGCAGCTGGTGGagtCTGGGGgaGgCGTGGTGCAGCctGGGGGGTCTCtaAAACTCTCCTGCgtAG CCTCGGGAACGGACTTCagtctgAATTTTATACGCTGGTACCGCCAGGCTCCAGGGAAGCAGCGCGA GTTCGTCGCAGGATTTACTGCGACTGgTAACACAAACTATGCAGACTCCAtgAAGGGGCGATTCAC CATCTCCAGAGACAACACCAAGAACGCGGTGTATCTGCAAATAGACAGCCTGAAACCTGAGGAC ACGGCCGTGTATTACTGCTATATGTTGGACAAGTGGGGCCAGGGGACCCAGGTCACCGTCTCCTC A(GCGGCCGCAGAACAAAAACTCATCTCAGAAGAGGATCTGAATGGGGCCGCACATCACCAC CATCACCATGGGAGCTAG)x, with x=1 or 0 |
| C5^{V11} | 96 (x=1)or 315 (x=0) | GagGTGCAGCTGGTGGagtCTGGGGgaGgCGTGGTGCAGCctGGGGGGTCTCtaAAACTCTCCTGCgtAG CCTCGGGAACGGACTTCagtATCAATtacATACGCTGGTACCGCCAGGCTCCAGGGAAGCAGCGCGA GTTCGTCGCAGGATTTACTGCGACTGgTAACACAAACTATGCAGACTCCAtgAAGGGGCGATTCAC CATCTCCAGAGACAACACCAAGAACGCGGTGTATCTGCAAATAGACAGCCTGAAACCTGAGGAC ACGGCCGTGTATTACTGCTATATGTTGGACAAGTGGGGCCAGGGGACCCAGGTCACCGTCTCCTC A(GCGGCCGCAGAACAAAAACTCATCTCAGAAGAGGATCTGAATGGGGCCGCACATCACCAC CATCACCATGGGAGCTAG)x, with x=1 or 0 |
| C5^{V12} | 97 (x=1)or 316 (x=0) | GagGTGCAGCTGGTGGagtCTGGGGgaGgCGTGGTGCAGCctGGGGGGTCTCtaAAACTCTCCTGCgtAG CCTCGGGAACGGACTTCagtATCAATTTTATACGCTGGTACCGCCAGGCTCCAGGGAAGCAGCGCG AGTTCGTCGCAGGAATTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGTTGGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| C5^{V13} | 98 (x=1)or 317 (x=0) | GagGTGCAGCTGGTGGagtCTGGGGgaGgCGTGGTGCAGCctGGGGGGTCTCtaAAACTCTCCTGCgtAG CCTCGGGAACGGACTTCagtATCAATTTTATACGCTGGTACCGCCAGGCTCCAGGGAAGCAGCGCG AGTTCGTCGCAGGATTTTCAGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGTTGGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| C5^{V14} | 99 (x=1)or 318 (x=0) | GagGTGCAGCTGGTGGagtCTGGGGgaGgCGTGGTGCAGCctGGGGGGTCTCtaAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACTCAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGTTGGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| C5^{V15} | 100 (x=1)or 319 (x=0) | GagGTGCAGCTGGTGGagtCTGGGGgaGgCGTGGTGCAGCctGGGGGGTCTCtaAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTTTATGTTGGACAAGTGGGGCCAGGGGACCCAGGTCACCGTCTCCTC A(GCGGCCGCAGAACAAAAACTCATCTCAGAAGAGGATCTGAATGGGGCCGCACATCACCAC CATCACCATGGGAGCTAG)x, with x=1 or 0 |
| C5^{v16} | 101 (x=1)or 320 (x=0) | GagGTGCAGCTGGTGGagtCTGGGGGaGgCGTGGTGCAGCctGGGGGGTCTCtaAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATTTTGGACAAGTGGGGCCAGGGGACCCAGGTCACCGTCTCCTC |
| | | A(GCGGCCGCAGAACAAAAACTCATCTCAGAAGAGGATCTGAATGGGGCCGCACATCACCAC CATCACCATGGGAGCTAG)x, with x=1 or 0 |
| C5^{V17} | 102 (x=1)or 321 (x=0) | GagGTGCAGCTGGTGGagtCTGGGGgaGgCGTGGTGCAGCctGGGGGGTCTCtaAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGATTGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| C5^{V18} | 103 (x=1)or 322 (x=0) | GagGTGCAGCTGGTGGagtCTGGGGgaGgCGTGGTGCAGCctGGGGGGTCTCtaAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGGTGGACAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| C5^{V19} | 104 (x=1)or 323 (x=0) | GagGTGCAGCTGGTGGagtCTGGGGgaGgCGTGGTGCAGCctGGGGGGTCTCtaAAACTCTCCTGCgtAG CCTCGGGAACGGACTTCagtATCAATTTTATACGCTGGTACCGCCAGGCTCCAGGGAAGCAGCGCG AGTTCGTCGCAGGATTTACTGCGACTGgTAACACAAACTATGCAGACTCCAtgAAGGGGCGATTCA CCATCTCCAGAGACAACACCAAGAACGCGGTGTATCTGCAAATAGACAGCCTGAAACCTGAGGA CACGGCCGTGTATTACTGCTATATGTTGGAAAAGTGGGGCCAGGGGACCCAGGTCACCGTCTCCT CA(GCGGCCGCAGAACAAAAACTCATCTCAGAAGAGGATCTGAATGGGGCCGCACATCACCA CCATCACCATGGGAGCTAG)x, with x=1 or 0 |
| C5^{h1} | 105 (x=1)or 324 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATGAGCTGGGTTCGCCAGGCTCCAGGGAAGG GTCTGGAGTGGGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{h2} | 106 (x=1)or 325 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATACGCTGGGTTCGCCAGGCTCCAGGGAAGC AGCGCGAGTTCGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{h2} | 107 (x=1)or 326 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATGCATTGGGTTCGCCAGGCTCCAGGGAAGG GTCTGGAGTGGGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{h4} | 108 (x=1)or 327 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATGAGCTGGGTTCGCCAGGCTCCAGGGAAGC AGCGCGAGTTCGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{h5} | 109(x=1) or 328 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATACGCTGGGTTCGCCAGGCTCCAGGGAAGG GTCTGGAGTGGGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG |
| | | TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| C5^{h6} | 110 (x=1)or 329 (x=0) | GAGGTGcAGCTGGTGGAGTCTGGGGGAGGCGTGGTGCAGCCTGGGGGGTCTCTACGTCTCTCCTG CGCAGCCTCGGGAACGGACTTCAGTATCAATTTTATACATTGGGTTCGCCAGGCTCCAGGGAAGG GTCTGGAGTGGGTCGCAGGATTTACTGCGACTGGTAACACAAACTATGCAGACTCCGTTAAGGGG CGATTCACCATCTCCAGAGACAACGCAAAGAACACCCTGTATCTGCAAATGAATAGCCTGCGTCC TGAGGACACGGCCGTGTATTACTGCTATATGTTGGACAAGTGGGGCCAGGGGACCCAGGTCACCG TCTCCTCA(GCGGCCGCAGAACAAAAACTCATCTCAGAAGAGGATCTGAATGGGGCCGCACA TCACCACCATCACCATGGGAGCTAG)x, with x=1 or 0 |
| DNA sequences of C5^{neg} (negative control), C5^{V20-V32,} C5^{h8-h26,} B8^{h1}, C5^{V13h20}, C5^{V16h20}, B8^{V1-V9, V11, V12, V14-V40,} B6^{V1-V4,} B7, H7, C12b, E4, E2, C3, F2a, E9, A9a, B4a, C10a, H1, E8 are listed in the enclosed Sequence listing ST26 as SEQ ID NO: 469 to 606, 646-663, 683-690 and 721-738. | | |

**Table 3: List of FR (Framework Regions) sequences of the VHH molecules**

| **VHH ref.** | **Amino acid FR sequences** | | | |
|---|---|---|---|---|
| | FR1 | FR2 | FR3 | FR4 |
| C5^{v20} to C5²⁹ | EVQLVESGGGVV QPGGSLKLSCVAS SEQ 36 | IRWYRQAPGKQREFVAG SEQ41 | NYADSMKGRFTISRDNTKNAVYLQI DSLKPEDTAVYYC SEQ46 | WGQGTQVT VSS SEQ50 |
| C5^{h7} | | IRWVRQAPGKGLEWVAG SEQ342 | NYADSMKGRFTISRDNTKNALYLQI DSLRPEDTAVYYC SEQ366 | |
| C5^{h8} | EVQLVESGGGVV QPGGSLRLSCAAS SEQ37 | | NYADSVKGRFTISRDNTKNTLYLQI DSLRPEDTAVYYC SEQ367 | |
| C5^{h9} | | IRWYRQAPGKGLEFVAG SEQ343 | NYADSVKGRFTISRDNAKNTLYLQ MNSLRPEDTAVYYC SEQ368 | |
| C5^{h10} | EVQLVESGGGVV QPGGSLKLSCAAS SEQ331 | IRWVRQAPGKGLEWVAG SEQ342 | NYADSVKGRFTISRDNTKNTLYLQI NSLRPEDTAVYYC SEQ369 | |
| C5^{h11} | EVQLVESGGGVV QPGGSLRLSCAAS SEQ37 | | NYADSMKGRFTISRDNTKNTLYLQ MNSLRPEDTAVYYC SEQ370 | |
| C5^{h12} | | IRWYRQAPGKGLEFVAG SEQ343 | NYADSVKGRFTISRDNAKNTLYLQI DSLRPEDTAVYYC SEQ371 | |
| C5^{h13} | | | NYADSVKGRFTISRDNTKNTLYLQ MNSLRPEDTAVYYC SEQ372 | |
| C5^{h14} | EVQLVESGGGVV QPGGSLRLSCVAS SEQ400 | IRWVRQAPGKGLEWVAG SEQ342 | NYADSVKGRFTISRDNTKNALYLQ MNSLRPEDTAVYYC SEQ373 | |
| C5^{h15} | | | NYADSVKGRFTISRDNTKNTLYLQ MDSLRPEDTAVYYC SEQ374 | |
| C5^{h16} |  SEQ332 | IRWYRQAPGKGLEFVAG SEQ343 | NYADSVKGRFTISRDNSKNTLYLQ MNSLRAEDTAVYYC SEQ375 | WGQGTLVT VSS SEQ39 |
| C5^{h17} | EVQLVESGGGVV QPGGSLKLSCVAS | IRWVRQAPGKGLEFVAG SEQ344 | NYADSMKGRFTISRDNTKNALYLQI DSLRPEDTAVYYC | WGQGTQVT VSS |
| | SEQ36 | | SEQ366 | SEQ50 |
| C5^{h18} | EVQLVESGGGVV QPGGSLKLSCVAS SEQ36 | IRWYRQAPGKGLEFVAG SEQ343 | NYADSVKGRFTISRDNAKNTLYLQ MNSLRPEDTAVYYC SEQ368 | |
| C5^{h19} | EVQLVESGGG VV QPGGSLRLSCAAS SEQ37 | IRWYRQAPGKQREFVAG SEQ41 | | |
| C5^{h20} | | IRWYRQAPGKGLEFVAG SEQ343 | NYADSVKGRFTISRDNAKNAVYLQ MNSLRPEDTAVYYC SEQ376 | |
| C5^{h21} | | | NYADSVKGRFTISRDNAKNTLYLQ MNSLKPEDTAVYYC SEQ377 | |
| C5^{h22} | | IRWYRQAPGKGREFVAG SEQ345 | NYADSVKGRFTISRDNAKNTLYLQ MNSLRPEDTAVYYC SEQ368 | |
| C5^{h23} | | IRW VRQAPGKQREF VAG SEQ346 | | |
| C5^{h24} | | IRWYRQAPGKGLEFVAG SEQ343 | NYADSMKGRFTISRDNAKNTLYLQ MNSLRPEDTAVYYC SEQ378 | |
| C5^{h25} | | IRWYRQAPGKGLEFVAG SEQ344 | NYADSVKGRFTISRDNAKNTLYLQI DSLRPEDTAVYYC SEQ371 | |
| C5^{h26} | | IRW YRQAPGKGLEW VAG SEQ347 | | |
| C5^{v32} | SEQ36 | IRWYSQAPGKQREFVAG SEQ716 | NYADSMKGRLTISRDNTKNAVYLQ IDSLKPEDTAVYYC SEQ717 | |
| C5^{neg} | EVQLVESGGGVV QPGGSLKLSCVAS SEQ36 | IRWYRQAPGKQREFVAG SEQ41 | NYADSMKGRFTISRDNTKNAVYLQI DSLKPEDTAVYYC SEQ46 | |
| B8^{h1} | EVQLVESGGGVV QPGGSLRLSCAAS SEQ37 | MRWYRQAPGKGLEWVAG SEQ348 | NYPDSVKGRFTISRDNAKNTVYLQ MNSLRPEDTAVYYC SEQ379 | |
| C5^{V13} h20 C5^{V16} h20 | | IRWYRQAPGKGLEFVAG SEQ343 | NYADSVKGRFTISRDNAKNAVYLQ MNSLRPEDTAVYYC SEQ376 | |
| B8^{V1} | EVQLVESGGGVV QPGGSLRLSCAAD SEQ333 | MRWYRQAPGKQREWVAG SEQ42 | NYPDSAKGRFTISRDNAKNTVYLQI DSLKPEDTAVYYC SEQ47 | |
| B8^{V2} | EVQLVESGGGVV QPGGSLRLSCAAS SEQ37 | MRWYGQAPGKQREWVAG SEQ349 | | |
| B8^{V3} | | MRW YREAPGKQREW VAG SEQ350 | | |
| B8^{V4} | | MRWYRQAPIKQREWVAG SEQ351 | | |
| B8^{V5} | | MRWYRQAPGKIREWVAG SEQ352 | | |
| B8^{V6- V9} | | MRWYRQAPGKQREWVAG SEQ42 | | |
| B6^{V1} | | MGWFRRAPGKERNLVAS | | |
| | EVQLVESGGGLV QPGGSLRLSCTAS SEQ38 | SEQ353 | YAISSVKGRFTISRDNAENTVFLQM NSLKPDDTAVYYC SEQ48 | |
| B6^{V2- V3} | | MGWFRRAPGKERELVAS SEQ42 | | |
| B6^{V4} | | MGWFRRAPGKERE**S**VAS SEQ354 | | |
| B6^{V5} | | MGWFRRAPGKEREL**Q**AS SEQ355 | | |
| B7 | QVQLVQSGGGLV QAGGSLTLSCTAS SEQ334 | MGWFRRAPEKERELVAS SEQ356 | YYADGMRGRFTISRDNSENTVSLQ MNNLKPEDTAVYYC SEQ380 | |
| H7 | EVQLVESGGGLV QAGGSLRLSCTAS SEQ335 | MGWFRRAPGKDRELVAS SEQ357 | YYANSMKERFTISRDNAQNTVSLQI SSLKPEDTAVYYC SEQ381 | |
| C12b | QVQLVQSGGGLV QPGGSLRLSCAAS SEQ336 | MSWVRQAPGKGRELVAS SEQ358 | YYADGMKGRFTISRDNAENTVSLQI NSLKPEDTAIYYC SEQ382 | |
| E4 | EVQLVESGGGLV QAGDSLRLSCTAS SEQ337 | MGWFRRAPGKERELIAS SEQ359 | YYADSSVKGRFTISRDNAENTVSLQ MNSLKPEDTAVYYC SEQ383 | |
| E2 | QVQLVQSGGGLV QAGGSLRLSCAAS SEQ338 | LAWHRQIPGKEREWVAG SEQ360 | SYRDSVKGRFTISRDNAKNTVFLQM NSLEPEDTGVYYC SEQ384 | |
| C3 | EVQLVQSGGGLV QAGGSLRLSCAAS SEQ339 | MAWHRQAPGKERLWVAG SEQ361 | SYADSVKGRFTISRDDAKNTVYLQ MDNLTPEDTAVYFC SEQ385 | |
| F2a | EVQLVESGGGLV QPGESLRLSCTAS SEQ340 | VGWYRQAPGEQRVLVAH SEQ362 | EYKDSVKGRFTISRDNARNTIYLEM KNLKPEDTAIYYC SEQ386 | WGRGTQVT VSS SEQ670 |
| E9 | EVQLVESGGGLV QPGGSLRLSCVSS SEQ341 | MGWFRQAPGKEREFVAA SEQ363 | DYADGVMGRFTISRNSALNTVYLQ MDSLKSTDTGVYVC SEQ387 | |
| A9a | QVQLVQSGGGLV QAGGSLRLSCAAS SEQ338 | MGWYRQAPGKQRELVAV SEQ364 | KYGDSVKGRFTISRDDAKNTVYLQ MNSLKPEDTAVYYC SEQ388 | |
| B4a | EVQLVESGGGLV QAGGSLRLSCTAS SEQ335 | MGWFRQTPGKEREFVAA SEQ365 | TYADSVKGRFTISRDNAKNTVYLQ MNSLEPTDTAVYYC SEQ389 | WGKGTQVT VSS SEQ391 |
| B8^{V16} | SEQ37 | MRWYRQAPGKQRE**Q**VAG SEQ458 | SEQ47 | SEQ50 |
| B8^{V17} | | MRWYRQAPGKQRE**F**VAG SEQ459 | | |
| B8^{V19} | | MRWYRQAPGKQR**H**WVAG SEQ460 | | |
| B8^{V23} | | M**I**WYRQAPGKQREWVAG SEQ461 | | |
| B8^{V24} | | M**E**WYRQAPGKQREWVAG SEQ462 | | |
| B8^{V25} | | MRWYRQAPGKQREWVA**A** SEQ463 | | |
| B8^{V26} | | MRW YRQAPGKQREW VA**K** SEQ464 | | |
| B8^{V27} | | SEQ42 | VYPDSAKGRFTISRDNAKNTVYLQI DSLKPEDTAVYYC SEQ625 | |
| B8^{V28} | | | FYPDSAKGRFTISRDNAKNTVYLQI DSLKPEDTAVYYC SEQ626 | |
| B8^{V34} | SEQ37 | SEQ42 | NYPDIAKGRFTISRDNAKNTVYLQI DSLKPEDTAVYYC SEQ718 | |
| B8^{V37} | | | NYPDSAKGRFTISEDNAKNTVYLQI DSLKPEDTAVYYC SEQ719 | |
| B8^{V40} | | MRWYRQAPGKQREWVSG SEQ720 | SEQ47 | |
| C10a | EVQLVESGGGLV QAGDSLRLSCAAS SEQ619 | IGWFRQAPGKEREKVSC SEQ621 | YYADSVKGRFTISRDTVKDMVYLQ MNSLKPEDTAVYYC SEQ623 | |
| H1 |  SEQ332 | IGWFRQAPGKEREKVSC SEQ621 | YYADSVKGRFTISRDTVKDMVYLQ MNSLKPEDTAVYYC SEQ623 | |
| E8 | VQLVESGGRLVQ AGGSLRLSCTAS SEQ620 | MHWFRQAPGKEREFVGA SEQ622 | EYADSVKGRFTISRDNAKSTVYLQ MNNLKPEDTAVYYC SEQ624 | |

**Table 4: Examples of tag sequences and linkers**

| **SEQ ID NO** | **nucleotide or amino acid tag sequence** |
|---|---|
| 51 | AAAEQKLISEEDLNGAAHHHHHHGS |
| 330 | GCGGCCGCAGAACAAAAACTCATCTCAGAAGAGGATCTGAATGGGGCCGCACATCACCACCATCAC CATGGGAGCTAG |
| 394 | EQKLISEEDL |
| 395 | HHHHHH |
| 396 | HHHHHHHH |
| 397 | YPYDVPDYA |
| 398 | DYKDDDDK |
| 399 | GGGGSCHHHHHH |
| 111 | GGGLQR |
| 401 | GGGGLQR |
| 402 | GGGGGLQR |
| 403 | GGGGGGLQR |
| 404 | GGGGGGGLQR |
| 112 | AAAHHHHHHGGGLQR |
| 406 | AAAHHHHHHCGGGLQR |
| 407 | AAAHHHHHHGGGGGLQR |
| 408 | AAAHHHHHHGGGGGGLQR |
| 409 | AAAHHHHHHGGGGGGGLQR |
| 627 | GlySerGlySer or GSGS |
| 628 | SerGlySerGly5 or SGSGGGGG |
| 629 | Glv4Ser or GGGGS |
| 630 | (Gly4Ser)2 or GGGGSGGGGS |
| 631 | (Gly4Ser)3 or GGGGSGGGGSGGGGS |
| 632 | (Gly4Ser)4 or GGGGSGGGGSGGGGSGGGGS |
| 633 | (Glv4Ser)5 or GGGGSGGGGSGGGGSGGGGSGGGGS |
| 634 | (Glv4Ser)6 or GGGGSGGGGSGGGGSGGGGSGGGGSGGGGS |
| 635 | GlyGly(Gly4Ser)3 or GGGGGGSGGGGSGGGGS |
| 636 | EAAAK |
| 637 | A(EAAAK)2A or AEAAAKEAAAKA |
| 638 | A(EAAAK)3A or AEAAAKEAAAKEAAAKA |
| 639 | GG(EAAAK)2EA or GGEAAAKEAAAKEA |
| 640 | GG(EAAAK)4EA or GGEAAAKEAAAKEAAAKEAAAKEA |
| 641 | GGA(EAAAK)4ALEA(EAAAK)4A or GGAEAAAKEAAAKEAAAKEAAAKALEAEAAAKEAAAKEAAAKEAAAKA |
| 642 | GG(AP)17 or GGAPAPAPAPAPAPAPAPAPAPAPAPAPAPAPAPAP |
| 643 | ASTKGPSVFPLAP |
| 644 | GSAGSAAGSGEF |
| 645 | KESGSVSSEQLAQFRSLD |

### REFERENCES

Barrientos T. et al. (2015). "Metabolic Catastrophe in Mice Lacking Transferrin Receptor in Muscle." EBioMedicine 2(11): 1705-1717.
Bustin et al., Clin Chem. 2009 Apr;55(4):611-22.
Chan and Gerhardt, Transferrin receptor gene is hyperexpressed and transcriptionally regulated in differentiating erythroid cells. J Biol Chem 1992 Apr 25;267(12):8254-9.
Jefferies et al., Transferrin receptor on endothelium of brain capillaries. Nature 1984 Nov 8-14;312(5990): 162-3.
Debacker, A. J., et al. (2020). "Delivery of Oligonucleotides to the Liver with GalNAc: From Research to Registered Therapeutic Drug." Mol Ther 28(8): 1759-1771.
Johnsen K.B. et al, Targeting the transferrin receptor for brain drug delivery. Prog Neurobiol. 2019 Oct;181: 101665.
Majumdar S. and Siahaan TJ., "Peptide-mediated targeted drug delivery". Med Res Rev. 2012 May;32(3):637-58.
Nair JK et al., Multivalent N-acetylgalactosamine-conjugated siRNA localizes in hepatocytes and elicits robust RNAi-mediated gene silencing, J. Am. Chem. Soc. 136 (2014) 16958-16961.
Needleman, S.B. and Wunsch, C.D., (1970), Journal of Molecular Biology, 48, 443-453.
Pardridge et al., Human blood-brain barrier transferrin receptor. Metabolism1987 Sep;36(9):892-5.
Sambrook J, Russell D (2001) Molecular cloning: a laboratory manual, Third Edition Cold Spring Harbor.
Tai, W. "Current Aspects of siRNA Bioconjugate for In Vitro and In Vivo Delivery." Molecules. 2019 Jun 13;24(12).
Tripathi et al., "The nuclear-retained noncoding RNA MALAT1 regulates alternative splicing by modulating SR splicing factor phosphorylation". Mol Cell. 2010 Sep 24;39(6):925-38.
Xu W., et al. (2015). "Lethal Cardiomyopathy in Mice Lacking Transferrin Receptor in the Heart." Cell Rep 13(3): 533-545.
Ying Li et al., Transferrin receptor 1 plays an important role in muscle development and denervation-induced muscular atrophy. Neural Regen Res 2021 Jul;16(7):1308-1316.

## Claims

1. A conjugate compound comprising:
(i) one or more VHH molecule(s) of formula FR1-CDR1-FR2-CDR2-FR3-CDR3-FR4, and
(ii) one or more oligonucleotide(s),
wherein said VHH molecule binds TfR at the surface of a muscle cell.

2. The conjugate compound of claim 1, wherein said VHH molecule comprises:
. a CDR1 comprising a sequence selected from SEQ ID NOs: 1, 5, 9, 13, 17, 19, 67, 69, 125, 175, 179, 182, 184, 186, 190, 194, 198, 201, 205, 392, 410, 413, 426, 434, 437, 607, 610, 671-674, 710 and 711, and/or
. a CDR2 comprising a sequence selected from SEQ ID NOs: 2, 6, 10, 14, 21, 23, 71, 73, 75, 113, 115, 128, 160, 162, 164, 166, 169, 171, 176, 187, 191, 195, 199, 202, 206, 416, 419, 431, 608, 611 and 712, and/or
. a CDR3 comprising a sequence selected from SEQ ID NOs: 3, 7, 11, 15, 25, 27, 29, 31, 33, 77, 79, 81, 83, 85, 117, 119, 121, 123, 177, 180, 188, 192, 196, 200, 203, 207, 452, 455, 609, 612 and 713-715.

3. The conjugate compound of claim 1 or 2, wherein said VHH molecule comprises SEQ ID NOs: 1, 2 and 3; or SEQ ID NOs: 5, 6 and 7; or SEQ ID NOs: 9, 10 and 11; or SEQ ID NOs: 13, 14 and 15; SEQ ID NOs: 17, 2 and 3; or SEQ ID NOs: 19, 2 and 3; or SEQ ID NOs: 1, 21 and 3; or SEQ ID NOs: 1, 23 and 3; or SEQ ID NOs: 1, 2 and 25; or SEQ ID NOs: 1, 2 and 27; or SEQ ID NOs: 1, 2 and 29; or SEQ ID NOs: 1, 2 and 31; or SEQ ID NOs: 1, 2 and 33; or SEQ ID NOs: 67, 2 and 3; or SEQ ID NOs: 69, 2 and 3; or SEQ ID NOs: 1, 71 and 3; or SEQ ID NOs: 1, 73 and 3; or SEQ ID NOs: 1, 75 and 3; or SEQ ID NOs: 1, 2 and 77; or SEQ ID NOs: 1, 2 and 79; or SEQ ID NOs: 1, 2 and 81; or SEQ ID NOs: 1, 2 and 83; or SEQ ID NOs: 1, 2 and 85; or SEQ ID NOs: 392, 2 and 3; or SEQ ID NOs: 1, 113 and 3; or SEQ ID NOs: 1, 115 and 3; or SEQ ID NOs: 1, 2 and 117; or SEQ ID NOs: 1, 2 and 119; or SEQ ID NOs: 1, 2 and 121; or SEQ ID NOs: 1, 2 and 123; or SEQ ID NOs: 125, 2 and 3; or SEQ ID NOs: 17, 73 and 3; or SEQ ID NOs: 17, 128 and 3; or SEQ ID NOs: 5, 160 and 7; or SEQ ID NOs: 5, 162 and 7; or SEQ ID NOs: 5, 164 and 7; or SEQ ID NOs: 5, 166 and 7; or SEQ ID NOs: 9, 169 and 11; or SEQ ID NOs: 9, 171 and 11; or SEQ ID NOs: 175, 176 and 177; or SEQ ID NOs: 179, 176 and 180; or SEQ ID NOs: 182, 176 and 177; or SEQ ID NOs: 184, 176 and 177; or SEQ ID NOs: 186, 187 and 188; or SEQ ID NOs: 190, 191 and 192; or SEQ ID NOs: 194, 195 and 196; or SEQ ID NOs: 198, 199 and 200; or SEQ ID NOs: 201, 202 and 203; or SEQ ID NOs: 205, 206 and 207; or SEQ ID NOs: 410, 6 and 7; or SEQ ID NOs: 413, 6 and 7; or SEQ ID NOs: 5, 416 and 7; or SEQ ID NOs: 5, 419 and 7; or SEQ ID NOs: 426, 6 and 7; or SEQ ID NOs: 5, 431 and 7; or SEQ ID NOs: 434, 6 and 7; or SEQ ID NOs: 437, 6 and 7; or SEQ ID NOs: 5, 6 and 452; or SEQ ID NOs: 5, 6 and 455; or SEQ ID NOs: 607, 608 and 609; or SEQ ID NOs: 610, 611 and 612; or SEQ ID NOs: 671, 2 and 3; or SEQ ID NOs: 672, 2 and 3; or SEQ ID NOs: 673, 6 and 7; or SEQ ID NOs: 674, 6 and 7; or SEQ ID NOs: 1, 2 and 713; or SEQ ID NOs: 5, 6 and 714; or SEQ ID NOs: 674, 164 and 7; or SEQ ID NOs: 710, 6 and 7; or SEQ ID NOs: 5, 6 and 715; or SEQ ID NOs: 674, 712 and 7; or SEQ ID NOs: 711, 6 and 7.

4. The conjugate compound of any one of claims 1 to 3, wherein said VHH molecule comprises an amino acid sequence selected from any one of SEQ ID NOs: 214, 273, 276-284, 412, 415, 418, 421, 423, 425, 428, 430, 433, 436, 439, 441, 443, 445, 447, 449, 451, 454, 457, 677, 678 and 702-709.

5. The conjugate compound of any one of claims 1 to 3, wherein said VHH molecule comprises an amino acid sequence selected from any one of SEQ ID NOs: 213, 216-271, 274, 275, 675, 676 and 701.

6. The conjugate compound of any one of claims 1 to 3, wherein said VHH molecule comprises an amino acid sequence selected from any one of SEQ ID NOs: 215 and 285-299.

7. The conjugate compound of any one of claims 1 to 3, wherein said VHH molecule comprises an amino acid sequence selected from any one of SEQ ID NOs: 613-615.

8. The conjugate compound of any one of the preceding claims, wherein said VHH molecule further comprises a tag and/or a linker.

9. The conjugate compound of any one of the preceding claims, wherein said VHH molecule is humanized, preferably selected from SEQ ID NO: 87-92, 130-149, 152-154, 236-241, 252-271 and 273-275.

10. The conjugate compound of any one of the preceding claims, wherein said VHH molecule binds the human, non-human primate and/or rodent TfR1.

11. The conjugate compound of any one of the preceding claims, wherein the oligonucleotide is selected from any single- or double-stranded oligonucleotide such as small interfering RNA (siRNA), small activating RNAs (saRNA), gapmer, antisense oligonucleotide (ASO), shRNA, miRNA, aptamer RNA and bridged nucleic acid (BNA).

12. The conjugate compound of any one of the preceding claims, wherein said muscle cell is a skeletal or a cardiac muscle cell, or a muscle cancer cell.

13. The conjugate compound of any one of the preceding claims, further comprising at least one additional compound, preferably a half-life extending moiety or stabilizing group or scaffold such as an antibody or a fragment thereof (such as a Fc fragment), a VHH molecule, PEG, a serum albumin protein, a serum albumin binding moiety, more preferably a Fc fragment, wherein the Fc fragment is even more preferably a Fc heterodimer comprising a modified Fc having a sequence of SEQ ID NO: 664 on the knob arm, and a modified Fc having a sequence of SEQ ID NO: 665 on the hole arm.

14. A pharmaceutical composition comprising a conjugate compound of any one of claims 1 to 13 and a pharmaceutically acceptable support, carrier or excipient.

15. The conjugate compound of any one of claims 1 to 13, or composition according to claim 14, for use in the treatment of a muscular or a neuromuscular disease preferably selected from the group consisting of a myopathy, a cardiomyopathy, a muscular dystrophy (such as DMD, BMD, FSHD, Pompe disease or familial hypertrophic cardiomyopathy), a peripheral neuropathy, ALS, SMA, MS, CMT and a cancer of the muscle (such as rhabdomyosarcoma or leiomyosarcoma).

16. The conjugate compound of any one of claims 1 to 13, or composition according to claim 14, or conjugate compound or composition for use according to claim 15, wherein the conjugate compound or composition is administered parenterally, preferably systemically, intravenously, intramuscularly or subcutaneously.
